(19) Europäisches Patentamt
European Patent Office
Office européen des brevets

(11) Veröffentlichungsnummer: **0 201 575 B1**

(12) **EUROPÄISCHE PATENTSCHRIFT**

(45) Veröffentlichungstag der Patentschrift: **12.08.92**

(51) Int. Cl.$^5$: **C07D 401/12**, C07D 491/04, A61K 31/415, A61K 31/44

(21) Anmeldenummer: **85905796.0**

(22) Anmeldetag: **29.10.85**

(86) Internationale Anmeldenummer: **PCT/EP85/00575**

(87) Internationale Veröffentlichungsnummer: **WO 86/02646 (09.05.86 86/10)**

Die Akte enthält technische Angaben, die nach dem Eingang der Anmeldung eingereicht wurden und die nicht in dieser Patentschrift enthalten sind.

# (54) NEUE PICOLINDERIVATE, VERFAHREN ZU IHRER HERSTELLUNG, IHRE ANWENDUNG UND SIE ENTHALTENDE ARZNEIMITTEL.

(30) Priorität: **31.10.84 CH 5235/84**

(43) Veröffentlichungstag der Anmeldung: **20.11.86 Patentblatt 86/47**

(45) Bekanntmachung des Hinweises auf die Patenterteilung: **12.08.92 Patentblatt 92/33**

(84) Benannte Vertragsstaaten: **AT BE CH DE FR GB IT LI LU NL SE**

(56) Entgegenhaltungen:
**EP-A- 80 602**
**EP-A- 127 763**
**EP-A- 134 400**
**GB-A- 2 082 580**
**GB-A- 2 134 523**

(73) Patentinhaber: **Byk Gulden Lomberg Chemische Fabrik GmbH**
**Byk-Gulden-Strasse 2**
**W-7750 Konstanz(DE)**

(72) Erfinder: **RAINER, Georg**
**Josef Anton Feuchtmayer-Str. 7**
**W-7750 Konstanz(DE)**
Erfinder: **RIEDEL, Richard**
**Salmannsweilerg. 36**
**W-7750 Konstanz(DE)**
Erfinder: **FIGALA, Volker**
**Am Hochfirst 2**
**W-7753 Allensbach 4(DE)**
Erfinder: **KOHL, Bernhard**
**Heinrich v. Tettingen-Str. 35a**
**W-7750 Konstanz 19(DE)**
Erfinder: **KLEMM, Kurt**
**Im Weinberg 2**
**W-7753 Allensbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Erfinder: **SCHAEFER, Hartmann**
**Zum Purren 27**
**W-7750 Konstanz 16(DE)**
Erfinder: **SENN-BILFINGER, Jörg**
**Säntisstr. 7**
**W-7750 Konstanz(DE)**

## Beschreibung

Anwendungsgebiet der Erfindung

Die Erfindung betrifft neue Picolinderivate, Verfahren zu ihrer Herstellung, ihre Anwendung und sie enthaltende Arzneimittel. Die erfindungsgemäßen Verbindungen werden in der pharmazeutischen Industrie als Zwischenprodukte und zur Herstellung von Medikamenten verwendet.

Stand der Technik

In der europäischen Patentanmeldung 0 005 129 werden substituierte Pyridylsulfinylbenzimidazole beschrieben, die magensäuresekretionshemmende Eigenschaften besitzen sollen. - In der europäischen Patentanmeldung 0 074 341 wird die Verwendung einer Reihe von Benzimidazolderivaten zur Magensäuresekretionshemmung beschrieben. - In der deutschen Offenlegungsschrift 34 04 610 werden weitere Benzimidazolderivate als zellschützende Mittel für den Magen-Darmkanal und als Hemmittel für die Magensäuresekretion bei Säugetieren beschrieben. - In den europäischen Patentanmeldungen 127 763 und 134 400 werden fluorsubstituierte Pyridylmethylsulfinyl-benzimidazole mit ausgeprägten magensäuresekretionshemmenden und ulcusprotectiven Eigenschaften beschrieben. - In der deutschen Offenlegungsschrift 31 32 613 werden weitere, die Magensäuresekretion hemmende Pyridylmethylsulfinyl-benzimidazole beschrieben.

Es wurde nun überraschenderweise gefunden, daß die unten näher beschriebenen Picolinderivate interessante und unerwartete Eigenschaften aufweisen, durch die sie sich in vorteilhafter Weise von den bekannten Verbindungen unterscheiden.

Beschreibung der Erfindung

Gegenstand der Erfindung sind neue Picolinderivate der Formel I

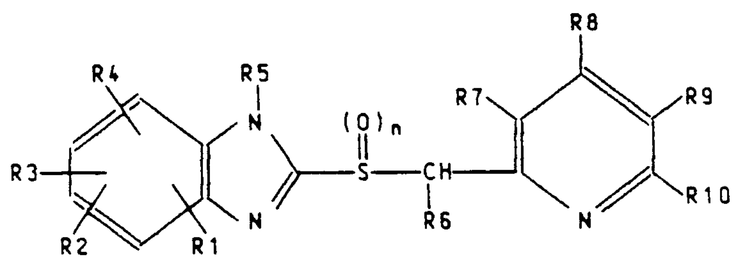

(I),

worin

R1, R2, R3 und R4 an beliebigen Positionen im Benzoteil des Benzimidazols stehen können und worin

- R1 Wasserstoff oder $C_1$-$C_6$-Alkyl bedeutet,
- R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,
- R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,
- R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,
- R5 Wasserstoff oder eine unter physiologischen Bedingungen leicht abspaltbare Gruppe bedeutet,
- R6 Wasserstoff oder $C_1$-$C_4$-Alkyl bedeutet,
- R7 Wasserstoff, $C_1$-$C_6$-Alkyl oder $C_1$-$C_6$-Alkoxy bedeutet,
- R8 Wasserstoff, $C_1$-$C_6$-Alkyl, $C_1$-$C_6$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,
- R9 Wasserstoff, $C_1$-$C_6$-Alkyl oder $C_1$-$C_6$-Alkoxy bedeutet,
- R10 Wasserstoff oder $C_1$-$C_6$-Alkyl bedeutet und
- n die Zahlen 0 oder 1 darstellt,

und die Salze dieser Verbindungen, wobei

ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy

haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und

R5 Wasserstoff und

R6 Wasserstoff und

R7 Wasserstoff oder $C_1$-$C_6$-Alkyl oder $C_1$-$C_6$-Alkoxy und

R8 Wasserstoff oder $C_1$-$C_6$-Alkyl oder $C_1$-$C_6$-Alkoxy und

R9 Wasserstoff oder $C_1$-$C_6$-Alkyl oder $C_1$-$C_6$-Alkoxy und

R10 Wasserstoff

bedeutet und in 4- und 7-Position des Benzimidazols Wasserstoffatome stehen.

Halogen im Sinne der vorliegenden Erfindung ist Brom, Chlor und insbesondere Fluor.

$C_1$-$C_6$-Alkyl steht für geradkettige und verzweigte Alkylreste. Geradkettige Alkylreste sind der Hexyl-, Pentyl-, Butyl-, Propyl-, Ethyl-, und insbesondere der Methylrest. Verzweigte Alkylreste sind beispielsweise der Neopentyl-, i-Butyl-, sec.-Butyl-, t-Butyl- und der Isopropylrest.

$C_1$-$C_6$-Alkoxy steht für geradkettige oder verzweigte Alkoxyreste. Beispielsweise seien genannt der Hexyloxy-, Neopentyloxy-, Butoxy-, i-Butoxy-, sec.-Butoxy-, t-Butoxy-, Propoxy-, Isopropoxy-, Ethoxy- und insbesondere der Methoxyrest.

$C_1$-$C_4$-Alkoxy steht für geradkettige oder verzweigte Alkoxyreste; beispielsweise seien genannt der Butoxy-, i-Butoxy-, sec.-Butoxy-, t-Butoxy-, Propoxy-, Isopropoxy-, Ethoxy- und insbesondere der Methoxyrest.

$C_1$-$C_4$-Alkyl steht für geradkettige oder verzweigte Alkylreste; beispielsweise seien der Butyl-, i-Butyl-, sec.-Butyl-, t-Butyl-, Propyl-, Isopropyl-, Ethyl- und insbesondere der Methylrest genannt.

$C_1$-$C_4$-Alkoxy-$C_1$-$C_4$-alkyl steht für $C_1$-$C_4$-Alkyl, das durch $C_1$-$C_4$-Alkoxy substituiert ist. Beispielsweise seien der Ethoxymethyl-, Propoxybutyl-, Methoxymethyl- und insbesondere der Methoxyethyl- und der Ethoxyethylrest genannt.

$C_1$-$C_4$-Alkoxy-$C_1$-$C_4$-alkoxy steht für $C_1$-$C_4$-Alkoxy, das durch $C_1$-$C_4$-Alkoxy substituiert ist. Beispielsweise seien der Methoxypropoxy-, Ethoxymethoxy- und insbesondere der Ethoxyethoxy- und der Methoxyethoxyrest genannt.

Phenoxy-$C_1$-$C_4$-alkyl steht für $C_1$-$C_4$-Alkyl, das durch einen Phenoxyrest substituiert ist. Beispielsweise seien der Phenoxypropyl- und der Phenoxyethylrest genannt.

Phenoxy-$C_1$-$C_4$-alkoxy steht für $C_1$-$C_4$-Alkoxy, das durch einen Phenoxyrest substituiert ist. Beispielsweise seien der Phenoxyethoxy- und der Phenoxypropoxyrest genannt.

Phen-$C_1$-$C_4$-alkyl steht für $C_1$-$C_4$-Alkyl, das durch einen Phenylrest substituiert ist. Beispielsweise seien der Phenethyl und insbesondere der Benzylrest genannt.

Phen-$C_1$-$C_4$-alkoxy steht für $C_1$-$C_4$-Alkoxy, das durch einen Phenylrest substituiert ist. Beispielsweise seien der 2-Phenyl-ethoxy- und der Benzyloxyrest genannt.

Als ganz oder überwiegend durch Fluor substituiertes $C_1$-$C_4$-Alkoxy seien beispielsweise der 1,1,2-Trifluorethoxy-, der Perfluorpropoxy-, der Perfluorethoxy- und insbesondere der 1,1,2,2-Tetrafluorethoxy-, der Trifluormethoxy-, der 2,2,2-Trifluorethoxy- und der Difluormethoxyrest genannt.

Als ganz oder teilweise durch Fluor substituiertes $C_1$-$C_2$-Alkylendioxy seien beispielsweise der 1,1-Difluorethylendioxy- ($-0-CF_2-CH_2-O-$), der 1,1,2,2-Tetrafluorethylendioxy- ($-O-CF_2-CF_2-O-$) und insbesondere der Difluormethylendioxy- ($-O-CF_2-O-$) und der 1,1,2-Trifluorethylendioxyrest ($-O-CF_2-CHF-O-$) genannt.

Eine unter physiologischen Bedingungen leicht abspaltbare Gruppe R5 ist ein Substituent, der durch - gegebenenfalls enzymatisch katalysierte - Hydrolyse vom Stickstoffatom unter Ausbildung einer N-H-Bindung abgetrennt wird, wobei er selbst - unter Anbindung einer Hydroxylgruppe - in eine physiologisch unbedenkliche und insbesondere pharmakologisch verträgliche Verbindung umgewandelt wird. Als abspaltbare Gruppen R5 seien insbesondere alle Arten von substituierten Carbonylgruppen genannt, wie die Alkylcarbonyl-, Arylcarbonyl-, Aralkylcarbonyl-, Alkoxycarbonyl-, Aryloxycarbonyl-, Aralkoxycarbonyl- oder die gegebenenfalls substituierte Carbamoylgruppe. Beispielsweise seien die Methoxycarbonyl-, t-Butoxycarbonyl-, Benzoyl-, Phenylcarbamoyl- und die Dimethylcarbamoylgruppe genannt.

$C_2$-$C_5$-Alkenyloxy steht für Alkenyloxyreste wie den Pent-2-enyloxy-, den But-1-enyloxy- und insbeson-

dere den Allyloxyrest.

$C_2$-$C_5$-Alkinyloxy steht für Alkinyloxyreste wie den Ethinyloxy- und insbesondere den Prop-2-inyloxyrest.

Als Salze kommen für Verbindungen der Formel I, in denen n die Zahl 0 bedeutet (Sulfide), bevorzugt alle Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze, die beispielsweise bei der Herstellung der erfindungsgemäßen Verbindungen im industriellen Maßstab als Verfahrensprodukte zunächst anfallen können, werden durch dem Fachmann bekannte Verfahren in pharmakologisch verträgliche Salze übergeführt. Als solche eignen sich beispielsweise wasserlösliche und wasserunlösliche Säureadditionssalze, wie das Hydrochlorid, Hydrobromid, Hydroiodid, Phosphat, Nitrat, Sulfat, Acetat, Citrat, Gluconat, Benzoat, Hibenzat, Fendizoat, Butyrat, Sulfosalicylat, Maleat, Laurat, Malat, Fumarat, Succinat, Oxalat, Tartrat, Amsonat, Embonat, Metembonat, Stearat, Tosilat, 2-Hydroxy-3-naphthoat, 3-Hydroxy-2-naphthoat oder Mesilat.

Für Verbindungen der Formel I, in denen n die Zahl 1 bedeutet (Sulfoxide), kommen als Salze bevorzugt basische Salze in Betracht, insbesondere pharmakologisch verträgliche Salze mit in der Galenik üblicherweise verwendeten anorganischen und organischen Basen. Als Beispiele für basische Salze seien Lithium-, Natrium-, Kalium-, Calcium-, Aluminium-, Magnesium-, Titan-, Ammonium- oder Guanidiniumsalze erwähnt.

Wenn R3 und R4 gemeinsam ganz oder teilweise durch Fluor substituiertes $C_1$-$C_2$-Alkylendioxy oder Chlortrifluorethylendioxy bedeuten, so sind die Substituenten R3 und R4 in Nachbarpositionen am Benzoteil des Benzimidazolringes gebunden.

Ein Aspekt der Erfindung sind Verbindungen der Formel I, worin

R1, R2, R3 und R4 an beliebigen Positionen im Benzoteil des Benzimidazols stehen können und worin

- R1 Wasserstoff oder $C_1$-$C_6$-Alkyl bedeutet,
- R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,
- R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,
- R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,
- R5 Wasserstoff oder eine unter physiologischen Bedingungen leicht abspaltbare Gruppe bedeutet,
- R6 Wasserstoff oder $C_1$-$C_4$-Alkyl bedeutet,
- R7 Wasserstoff oder $C_1$-$C_6$-Alkyl bedeutet,
- R8 Wasserstoff, $C_1$-$C_6$-Alkyl, $C_1$-$C_6$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,
- R9 Wasserstoff oder $C_1$-$C_6$-Alkyl bedeutet,
- R10 Wasserstoff oder $C_1$-$C_6$-Alkyl bedeutet und
- n die Zahlen 0 oder 1 darstellt,

und die Salze dieser Verbindungen, wobei

ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy

haben kann, wenn

- R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und
- R5 Wasserstoff und
- R6 Wasserstoff und
- R7 Wasserstoff oder $C_1$-$C_6$-Alkyl und
- R8 Wasserstoff oder $C_1$-$C_6$-Alkyl oder $C_1$-$C_6$-Alkoxy und
- R9 Wasserstoff oder $C_1$-$C_6$-Alkyl und
- R10 Wasserstoff

bedeutet und in 4- und 7-Position des Benzimidazols Wasserstoffatome stehen.

Ein weiterer Aspekt der Erfindung sind Verbindungen der Formel I, worin

R1, R2, R3 und R4 an beliebigen Positionen im Benzoteil des Benzimidazols stehen können und worin

R1 Wasserstoff oder $C_1$-$C_6$-Alkyl bedeutet,

R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,

R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,

R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,

R5 Wasserstoff oder eine unter physiologischen Bedingungen leicht abspaltbare Gruppe bedeutet,

R6 Wasserstoff oder $C_1$-$C_4$-Alkyl bedeutet,

R8 Wasserstoff, $C_1$-$C_6$-Alkyl, $C_1$-$C_6$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,

einer der Substituenten R7 und R9 $C_1$-$C_6$-Alkoxy und der andere Wasserstoff, $C_1$-$C_6$-Alkyl oder $C_1$-$C_6$-Alkoxy bedeutet,

R10 Wasserstoff oder $C_1$-$C_6$-Alkyl bedeutet und

n die Zahlen 0 oder 1 darstellt,

und die Salze dieser Verbindungen, wobei

ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy

haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und

R5 Wasserstoff und

R6 Wasserstoff und

R8 Wasserstoff oder $C_1$-$C_6$-Alkyl oder $C_1$-$C_6$-Alkoxy und

einer der Substituenten R7 und R9 $C_1$-$C_6$-Alkoxy und der andere Wasserstoff oder $C_1$-$C_6$-Alkyl oder $C_1$-$C_6$-Alkoxy und

R10 Wasserstoff

bedeutet und in 4- und 7-Position des Benzimidazols Wasserstoffatome stehen.

Eine Ausgestaltung (Ausgestaltung a) der Erfindung sind Verbindungen der Formel I, worin R6 Wasserstoff und R10 Wasserstoff bedeutet und R1, R2, R3, R4, R5, R7, R8, R9 und n die eingangs angegebenen Bedeutungen haben, und ihre Salze.

Eine weitere Ausgestaltung (Ausgestaltung b) der Erfindung sind Verbindungen der Formel I, worin R6 $C_1$-$C_4$-Alkyl und R10 Wasserstoff bedeutet und R1, R2, R3, R4, R5, R7, R8, R9 und n die eingangs angegebenen Bedeutungen haben, und ihre Salze.

Hervorzuhebende erfindungsgemäße Verbindungen sind solche der Formel I, in denen

R1 Wasserstoff oder $C_1$-$C_4$-Alkyl bedeutet,

R2 Wasserstoff, Halogen, $C_1$-$C_4$-Alkyl, $C_1$-$C_4$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,

R3 Wasserstoff, $C_1$-$C_4$-Alkyl, $C_1$-$C_4$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,

R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,

R5 Wasserstoff bedeutet,

R6 Wasserstoff oder $C_1$-$C_4$-Alkyl bedeutet,

R7 Wasserstoff, $C_1$-$C_4$-Alkyl oder $C_1$-$C_4$-Alkoxy bedeutet,

R8 Wasserstoff, $C_1$-$C_4$-Alkyl, $C_1$-$C_4$-Alkoxy, $C_1$-$C_4$-Alkoxy-$C_1$-$C_4$-alkoxy, $C_2$-$C_5$-Alkenyloxy oder $C_2$-

$C_5$-Alkinyloxy bedeutet,

R9 Wasserstoff, $C_1$-$C_4$-Alkyl oder $C_1$-$C_4$-Alkoxy bedeutet,

R10 Wasserstoff oder $C_1$-$C_4$-Alkyl bedeutet und

n die Zahlen 0 oder 1 darstellt,

und die Salze dieser Verbindungen, wobei

ein in 5- bzw. 6-Position im Benzimidazol stehender Substituent R1, R2 oder R3 nicht die Bedeutung Wasserstoff, Halogen, $C_1$-$C_4$-Alkyl, $C_1$-$C_4$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy

haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und

R5 Wasserstoff und

R6 Wasserstoff und

R7 Wasserstoff oder $C_1$-$C_4$-Alkyl oder $C_1$-$C_4$-Alkoxy und

R8 Wasserstoff oder $C_1$-$C_4$-Alkyl oder $C_1$-$C_4$-Alkoxy und

R9 Wasserstoff oder $C_1$-$C_4$-Alkyl oder $C_1$-$C_4$-Alkoxy und

R10 Wasserstoff

bedeutet und in 4- und 7-Position des Benzimidazols Wasserstoffatome stehen.

Ein Aspekt der hervorzuhebenden erfindungsgemäßen Verbindungen sind solche der Formel I, in denen

R1 Wasserstoff oder $C_1$-$C_4$-Alkyl bedeutet,

R2 Wasserstoff, Halogen, $C_1$-$C_4$-Alkyl, $C_1$-$C_4$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,

R3 Wasserstoff, $C_1$-$C_4$-Alkyl, $C_1$-$C_4$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,

R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,

R5 Wasserstoff bedeutet,

R6 Wasserstoff oder $C_1$-$C_4$-Alkyl bedeutet,

R7 Wasserstoff oder $C_1$-$C_4$-Alkyl bedeutet,

R8 Wasserstoff, $C_1$-$C_4$-Alkyl, $C_1$-$C_4$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,

R9 Wasserstoff oder $C_1$-$C_4$-Alkyl bedeutet,

R10 Wasserstoff oder $C_1$-$C_4$-Alkyl bedeutet und

n die Zahlen 0 oder 1 darstellt,

und die Salze dieser Verbindungen, wobei

ein in 5- bzw. 6-Position im Benzimidazol stehender Substituent R1, R2 oder R3 nicht die Bedeutung Wasserstoff, Halogen, $C_1$-$C_4$-Alkyl, $C_1$-$C_4$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy

haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und

R5 Wasserstoff und

R6 Wasserstoff und

R7 Wasserstoff oder $C_1$-$C_4$-Alkyl und

R8 Wasserstoff oder $C_1$-$C_4$-Alkyl oder $C_1$-$C_4$-Alkoxy und

R9 Wasserstoff oder $C_1$-$C_4$-Alkyl und

R10 Wasserstoff

bedeutet und in 4- und 7-Position des Benzimidazols Wasserstoffatome stehen.

Ein weiterer Aspekt der hervorzuhebenden erfindungsgemäßen Verbindungen sind solche der Formel I,

in denen

R1 Wasserstoff oder $C_1$-$C_4$-Alkyl bedeutet,

R2 Wasserstoff, Halogen, $C_1$-$C_4$-Alkyl, $C_1$-$C_4$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,

R3 Wasserstoff, $C_1$-$C_4$-Alkyl, $C_1$-$C_4$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,

R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,

R5 Wasserstoff bedeutet,

R6 Wasserstoff oder $C_1$-$C_4$-Alkyl bedeutet,

R8 Wasserstoff, $C_1$-$C_4$-Alkyl, $C_1$-$C_4$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,

einer der Substituenten R7 und R9 $C_1$-$C_4$-Alkoxy und der andere Wasserstoff, $C_1$-$C_4$-Alkyl oder $C_1$-$C_4$-Alkoxy bedeutet,

R10 Wasserstoff oder $C_1$-$C_4$-Alkyl bedeutet und

n die Zahlen 0 oder 1 darstellt,

und die Salze dieser Verbindungen, wobei
ein in 5- bzw. 6-Position im Benzimidazol stehender Substituent R1, R2 oder R3 nicht die Bedeutung Wasserstoff, Halogen, $C_1$-$C_4$-Alkyl, $C_1$-$C_4$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy
haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und

R5 Wasserstoff und

R6 Wasserstoff und

R8 Wasserstoff oder $C_1$-$C_4$-Alkyl oder $C_1$-$C_4$-Alkoxy und

einer der Substituenten R7 und R9 $C_1$-$C_4$-Alkoxy und der andere Wasserstoff oder $C_1$-$C_4$-Alkyl oder $C_1$-$C_4$-Alkoxy und

R10 Wasserstoff

bedeutet und in 4- und 7-Position des Benzimidazols Wasserstoffatome stehen.

Die Ausgestaltung a der hervorzuhebenden Verbindungen betrifft solche hervorzuhebende Verbindungen der Formel I, in denen R6 Wasserstoff bedeutet und R10 Wasserstoff bedeutet.

Die Ausgestaltung b der hervorzuhebenden Verbindungen betrifft solche hervorzuhebende Verbindungen der Formel I, in denen R6 $C_1$-$C_4$-Alkyl bedeutet und R10 Wasserstoff bedeutet.

Bevorzugte erfindungsgemäße Verbindungen sind solche, in denen

R1 Wasserstoff, Methyl oder Ethyl bedeutet,

R2 Wasserstoff, Chlor, Fluor, Methyl, Ethyl, Methoxy oder Ethoxy, bedeutet,

R3 Wasserstoff, Methyl, Ethyl, Methoxy, Ethoxy, 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R4 Difluormethylendioxy, 1,1,2-Trifluorethylendioxy oder 1-Chlor-1,2,2-trifluorethylendioxy bedeutet,

R4 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R3 Difluormethylendioxy, 1,1,2-Trifluorethylendioxy oder 1-Chlor-1,2,2-trifluorethylendioxy bedeutet,

R5 Wasserstoff bedeutet,

R6 Wasserstoff, Methyl oder Ethyl bedeutet,

R7 Wasserstoff, Methyl, Ethyl, Methoxy oder Ethoxy bedeutet,

R8 Wasserstoff, Methyl, Ethyl, Methoxy, Ethoxy oder Allyloxy bedeutet,

R9 Wasserstoff, Methyl, Ethyl, Methoxy oder Ethoxy bedeutet,

R10 Wasserstoff, Methyl oder Ethyl bedeutet und

n die Zahlen 0 oder 1 darstellt, und die Salze dieser Verbindungen, wobei

ein in 5- bzw. 6-Position im Benzimidazol stehender Substituent R1, R2 oder R3 nicht die Bedeutung Wasserstoff, Chlor, Fluor, Methyl, Ethyl, Methoxy, Ethoxy, 1,1,2,2-Tetrafluorethoxy, Trifluormethoxy, 2,2,2-Trifluorethoxy, Difluormethoxy oder gemeinsam mit R4 - wenn dieses in 6- bzw. 5-Position steht - Difluormethylendioxy, 1,1,2-Trifluorethylendioxy oder 1-Chlor-1,2,2-trifluorethylendioxy
haben kann, wenn

- R4 in 6- bzw. 5-Position steht und 1,1,2,2-Tetrafluorethoxy, Trifluormethoxy, 2,2,2-Trifluorethoxy, Difluormethoxy oder gemeinsam mit R3 - wenn dieses in 5- bzw. 6-Position steht - Difluormethylendioxy, 1,1,2-Trifluorethylendioxy oder 1-Chlor-1,2,2-trifluorethylendioxy und
- R5 Wasserstoff und
- R6 Wasserstoff und
- R7 Wasserstoff oder Methyl oder Ethyl oder Methoxy oder Ethoxy und
- R8 Wasserstoff oder Methyl oder Ethyl oder Methoxy oder Ethoxy und
- R9 Wasserstoff oder Methyl oder Ethyl oder Methoxy oder Ethoxy und
- R10 Wasserstoff

bedeutet und in 4- und 7-Position des Benzimidazols Wasserstoffatome stehen.

Ein Aspekt der bevorzugten erfindungsgemäßen Verbindungen sind solche der Formel I, in denen

- R1 Wasserstoff, Methyl oder Ethyl bedeutet,
- R2 Wasserstoff, Chlor, Fluor, Methyl, Ethyl, Methoxy oder Ethoxy, bedeutet,
- R3 Wasserstoff, Methyl, Ethyl, Methoxy, Ethoxy, 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R4 Difluormethylendioxy, 1,1,2-Trifluorethylendioxy oder 1-Chlor-1,2,2-trifluorethylendioxy bedeutet,
- R4 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R3 Difluormethylendioxy, 1,1,2-Trifluorethylendioxy oder 1-Chlor-1,2,2-trifluorethylendioxy bedeutet,
- R5 Wasserstoff bedeutet,
- R6 Wasserstoff, Methyl oder Ethyl bedeutet,
- R7 Wasserstoff, Methyl oder Ethyl bedeutet,
- R8 Wasserstoff, Methyl, Ethyl, Methoxy, Ethoxy oder Allyloxy bedeutet,
- R9 Wasserstoff, Methyl oder Ethyl bedeutet,
- R10 Wasserstoff, Methyl oder Ethyl bedeutet und
- n die Zahlen 0 oder 1 darstellt, und die Salze dieser Verbindungen,

wobei
ein in 5- bzw. 6-Position im Benzimidazol stehender Substituent R1, R2 oder R3 nicht die Bedeutung Wasserstoff, Chlor, Fluor, Methyl, Ethyl, Methoxy, Ethoxy, 1,1,2,2-Tetrafluorethoxy, Trifluormethoxy, 2,2,2-Trifluorethoxy, Difluormethoxy oder gemeinsam mit R4 - wenn dieses in 6- bzw. 5-Position steht - Difluormethylendioxy, 1,1,2-Trifluorethylendioxy oder 1-Chlor-1,2,2-trifluorethylendioxy
haben kann, wenn

- R4 in 6- bzw. 5-Position steht und 1,1,2,2-Tetrafluorethoxy, Trifluormethoxy, 2,2,2-Trifluorethoxy, Difluormethoxy oder gemeinsam mit R3 - wenn dieses in 5- bzw. 6-Position steht - Difluormethylendioxy, 1,1,2-Trifluorethylendioxy oder 1-Chlor-1,2,2-trifluorethylendioxy und
- R5 Wasserstoff und
- R6 Wasserstoff und
- R7 Wasserstoff oder Methyl oder Ethyl und
- R8 Wasserstoff oder Methyl oder Ethyl oder Methoxy oder Ethoxy und
- R9 Wasserstoff oder Methyl oder Ethyl und
- R10 Wasserstoff

bedeutet und in 4- und 7-Position des Benzimidazols Wasserstoffatome stehen.

Ein weiterer Aspekt der bevorzugten erfindungsgemäßen Verbindungen sind solche der Formel I, in denen

- R1 Wasserstoff, Methyl oder Ethyl bedeutet,
- R2 Wasserstoff, Chlor, Fluor, Methyl, Ethyl, Methoxy oder Ethoxy, bedeutet,
- R3 Wasserstoff, Methyl, Ethyl, Methoxy, Ethoxy, 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R4 Difluormethylendioxy, 1,1,2-Trifluorethylendioxy oder 1-Chlor-1,2,2-trifluorethylendioxy bedeutet,
- R4 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R3 Difluormethylendioxy, 1,1,2-Trifluorethylendioxy oder 1-Chlor-1,2,2-trifluorethylendioxy bedeutet,
- R5 Wasserstoff bedeutet,
- R6 Wasserstoff, Methyl oder Ethyl bedeutet,
- R8 Wasserstoff, Methyl, Ethyl, Methoxy, Ethoxy oder Allyloxy bedeutet,

einer der Substituenten R7 und R9 Methoxy oder Ethoxy und der andere Wasserstoff, Methyl, Ethyl, Methoxy oder Ethoxy bedeutet,

R10 Wasserstoff, Methyl oder Ethyl bedeutet und

n die Zahlen 0 oder 1 darstellt, und die Salze dieser Verbindungen,

wobei

ein in 5- bzw. 6-Position im Benzimidazol stehender Substituent R1, R2 oder R3 nicht die Bedeutung Wasserstoff, Chlor, Fluor, Methyl, Ethyl, Methoxy, Ethoxy, 1,1,2,2-Tetrafluorethoxy, Trifluormethoxy, 2,2,2-Trifluorethoxy, Difluormethoxy oder gemeinsam mit R4 - wenn dieses in 6- bzw. 5-Position steht - Difluormethylendioxy, 1,1,2-Trifluorethylendioxy oder 1-Chlor-1,2,2-trifluorethylendioxy

haben kann, wenn

R4 in 6- bzw. 5-Position steht und 1,1,2,2-Tetrafluorethoxy, Trifluormethoxy, 2,2,2-Trifluorethoxy, Difluormethoxy oder gemeinsam mit R3 - wenn dieses in 5- bzw. 6-Position steht - Difluormethylendioxy, 1,1,2-Trifluorethylendioxy oder 1-Chlor-1,2,2-trifluorethylendioxy und

R5 Wasserstoff und

R6 Wasserstoff und

R8 Wasserstoff oder Methyl oder Ethyl oder Methoxy oder Ethoxy und

einer der Substituenten R7 und R9 Methoxy oder Ethoxy und der andere Wasserstoff, Methyl, Ethyl, Methoxy oder Ethoxy und

R10 Wasserstoff

bedeutet und in 4- und 7-Position des Benzimidazols Wasserstoffatome stehen.

Die Ausgestaltung a der bevorzugten Verbindungen betrifft solche bevorzugte Verbindungen der Formel I, in denen R6 Wasserstoff und R10 Wasserstoff bedeutet.

Die Ausgestaltung b der bevorzugten Verbindungen betrifft solche bevorzugte Verbindungen der Formel I, in denen R6 Methyl oder Ethyl und R10 Wasserstoff bedeutet.

Besonders bevorzugte erfindungsgemäße Verbindungen sind solche der Formel I, worin

R1 Wasserstoff bedeutet,

R2 Wasserstoff, Methyl oder Methoxy bedeutet,

R3 Wasserstoff, Methyl, Methoxy, 1,1,2,2-Tetrafluorethoxy, Difluormethoxy, 2-Chlor-1,1,2-trifluorethoxy oder gemeinsam mit R4 Difluormethylendioxy bedeutet,

R4 1,1,2,2-Tetrafluorethoxy, Difluormethoxy, 2-Chlor-1,1,2-trifluorethoxy oder gemeinsam mit R3 Difluormethylendioxy bedeutet,

R5 Wasserstoff bedeutet,

R6 Wasserstoff oder Methyl bedeutet,

R7 Wasserstoff, Methyl oder Methoxy bedeutet,

R8 Methoxy bedeutet,

R9 Wasserstoff bedeutet,

R10 Wasserstoff bedeutet und

n die Zahlen 0 oder 1 darstellt, und die Salze dieser Verbindungen,

wobei

ein in 5- bzw. 6-Position im Benzimidazol stehender Substituent R1, R2 oder R3 nicht die Bedeutung Wasserstoff, Methyl, Methoxy, 1,1,2,2-Tetrafluorethoxy,Difluormethoxy oder gemeinsam mit R4 - wenn dieses in 6- bzw. 5-Position steht - Difluormethylendioxy

haben kann, wenn

R4 in 6- bzw. 5-Position steht und 1,1,2,2-Tetrafluorethoxy, Difluormethoxy oder gemeinsam mit R3 - wenn dieses in 5- bzw. 6-Position steht - Difluormethylendioxy und

R5 Wasserstoff und

R6 Wasserstoff und

R7 Wasserstoff, Methyl oder Methoxy und

R8 Methoxy und

R9 Wasserstoff und

R10 Wasserstoff

bedeutet und in 4- und 7-Position des Benzimidazols Wasserstoffatome stehen.

Ein Aspekt der besonders bevorzugten erfindungsgemäßen Verbindungen sind solche der Formel I, worin

R1 Wasserstoff bedeutet,

R2 Wasserstoff, Methyl oder Methoxy bedeutet,

R3 Wasserstoff, Methyl, Methoxy, 1,1,2,2-Tetrafluorethoxy, Difluormethoxy, 2-Chlor-1,1,2-trifluorethoxy oder gemeinsam mit R4 Difluormethylendioxy bedeutet,

R4 1,1,2,2-Tetrafluorethoxy, Difluormethoxy, 2-Chlor-1,1,2-trifluorethoxy oder gemeinsam mit R3 Difluormethylendioxy bedeutet,
R5 Wasserstoff bedeutet,
R6 Wasserstoff oder Methyl bedeutet,
R7 Wasserstoff oder Methyl bedeutet,
R8 Methoxy bedeutet,
R9 Wasserstoff bedeutet,
R10 Wasserstoff bedeutet und
n die Zahlen 0 oder 1 darstellt, und die Salze dieser Verbindungen,

wobei
ein in 5- bzw. 6-Position im Benzimidazol stehender Substituent R1, R2 oder R3 nicht die Bedeutung Wasserstoff, Methyl, Methoxy, 1,1,2,2-Tetrafluorethoxy,Difluormethoxy oder gemeinsam mit R4 - wenn dieses in 6- bzw. 5-Position steht - Difluormethylendioxy
haben kann, wenn
R4 in 6- bzw. 5-Position steht und 1,1,2,2-Tetrafluorethoxy, Difluormethoxy oder gemeinsam mit R3 - wenn dieses in 5- bzw. 6-Position steht - Difluormethylendioxy und
R5 Wasserstoff und
R6 Wasserstoff und
R7 Wasserstoff oder Methyl und
R8 Methoxy und
R9 Wasserstoff und
R10 Wasserstoff
bedeutet und in 4- und 7-Position des Benzimidazols Wasserstoffatome stehen.

Ein weiterer Aspekt der besonders bevorzugten erfindungsgemäßen Verbindungen sind solche der Formel I, worin
R1 Wasserstoff bedeutet,
R2 Wasserstoff, Methyl oder Methoxy bedeutet,
R3 Wasserstoff, Methyl, Methoxy, 1,1,2,2-Tetrafluorethoxy, Difluormethoxy, 2-Chlor-1,1,2-trifluorethoxy oder gemeinsam mit R4 Difluormethylendioxy bedeutet,
R4 1,1,2,2-Tetrafluorethoxy, Difluormethoxy, 2-Chlor-1,1,2-trifluorethoxy oder gemeinsam mit R3 Difluormethylendioxy bedeutet,
R5 Wasserstoff bedeutet,
R6 Wasserstoff oder Methyl bedeutet,
R7 Methoxy bedeutet,
R8 Methoxy bedeutet,
R9 Wasserstoff bedeutet und
R10 Wasserstoff bedeutet und
n die Zahlen 0 oder 1 darstellt, und die Salze dieser Verbindungen,

wobei
ein in 5- bzw. 6-Position im Benzimidazol stehender Substituent R1, R2 oder R3 nicht die Bedeutung Wasserstoff, Methyl, Methoxy, 1,1,2,2-Tetrafluorethoxy,Difluormethoxy oder gemeinsam mit R4 - wenn dieses in 6- bzw. 5-Position steht - Difluormethylendioxy
haben kann, wenn
R4 in 6- bzw. 5-Position steht und 1,1,2,2-Tetrafluorethoxy, Difluormethoxy oder gemeinsam mit R3 - wenn dieses in 5- bzw. 6-Position steht - Difluormethylendioxy und
R5 Wasserstoff und
R6 Wasserstoff und
R7 Methoxy und
R8 Methoxy und
R9 Wasserstoff und
R10 Wasserstoff
bedeutet und in 4- und 7-Position des Benzimidazols Wasserstoffatome stehen.

Bei den bevorzugten und besonders bevorzugten erfindungsgemäßen Verbindungen bzw. bei ihren Ausgestaltungen a und b sind die Substituenten R3 und R4 insbesondere in 5- und 6-Position des Benzimidazols.

Bei den Verbindungen der Formel I, worin R5 Wasserstoff bedeutet, ist - bedingt durch die Tautomerie im Imidazolring - im Benzimidazol die 4-Position mit der 7-Position, und die 5-Position mit der 6-Position identisch.

Als erfindungsgemäße Verbindungen seien beispielsweise genannt:
5-Difluormethoxy-2-[(4-methoxy-3-methyl-2-pyridyl)methylthio]-4,6-dimethyl-1H-benzimidazol,
5-Difluormethoxy-2-[(4-methoxy-3,5-dimethyl-2-pyridyl)methylthio]-4,6-dimethyl-1H-benzimidazol,
5-Difluormethoxy-2-{[4-(2-methoxyethoxy)-3-methyl-2-pyridyl]methyl thio}-4,6-dimethyl-1H-benzimidazol,
5-Difluormethoxy-4,6-dimethyl-2-[(4-methyl-2-pyridyl)methylthio]-1H-benzimidazol,
5-Difluormethoxy-4,6-dimethyl-2-[(3-methyl-2-pyridyl)methylthio]-1H-benzimidazol,
5-Difluormethoxy-4,6-dimethyl-2-[(5-methyl-2-pyridyl)methylthio]-1H-benzimidazol,
2-[(4-Allyloxy-3-methyl-2-pyridyl)methylthio]-5-difluormethoxy-4,6-dimethyl-1H-benzimidazol,
5-Difluormethoxy-4,6-dimethoxy-2-[(4-methoxy-3-methyl-2-pyridyl)methylthio]-1H-benzimidazol,
5-Difluormethoxy-4,6-dimethoxy-2-[(4-methoxy-3,5-dimethyl-2-pyridyl)-methylthio]-1H-benzimidazol,
5-Difluormethoxy-4,6-dimethoxy-2-[(3,5-dimethyl-2-pyridyl)methylthio]-1H-benzimidazol,
2-[(4-Allyloxy-3-methyl-2-pyridyl)methylthio]-5-difluormethoxy-4,6-dimethoxy-1H-benzimidazol,
4-Difluormethoxy-2-[(4-methoxy-2-pyridyl)methlthio]-6-methyl-1H-benzimidazol,
4-Difluormethoxy-2-[(4-methoxy-3-methyl-2-pyridyl)methylthio]-6-methyl-1H-benzimidazol,
4-Difluormethoxy-2-[(4-methoxy-3-methyl-2-pyridyl)methylthio]-7-methyl-1H-benzimidazol,
5-Difluormethoxy-2-[(4-methoxy-2-pyridyl)methylthio]-4,6,7-trimethyl-1H-benzimidazol,
2-[(4-Allyloxy-3-methyl-2-pyridyl)methylthio]-5-difluormethoxy-6-methyl-1H-benzimidazol,
5-Difluormethoxy-2-[(4-methoxy-2-pyridyl)methylthio]-6,7-dimethyl-1H-benzimidazol,
5-Difluormethoxy-2-[(4-methoxy-3-methyl-2-pyridyl)methylthio]-6,7-dimethyl-1H-benzimidazol,
5-Difluormethoxy-2-[(4-methoxy-3-methyl-2-pyridyl)methylthio]-6-(2-methoxyethoxy)-1H-benzimidazol,
5-(2-Chlor-1,1,2-trifluorethoxy)-2-[(4-methoxy-2-pyridyl)methylthio]-4,6-dimethyl-1H-benzimidazol,
5-(2-Chlor-1,1,2-trifluorethoxy)-2-[(4-methoxy-3-methyl-2-pyridyl)methylthio]-4,6-dimethyl-1H-benzimidazol,
2-[(4-Allyloxy-3-methyl-2-pyridyl)methylthio]-5-(2-chlor-1,1,2-trifluorethoxy)-1H-benzimidazol,
5-(2-Chlor-1,1,2-trifluorethoxy)-2-[(4-methoxy-2-pyridyl)methylthio]-1H-benzimidazol,
5-(2-Chlor-1,1,2-trifluorethoxy)-2-[(4-methoxy-3-methyl-2-pyridyl)methylthio]-1H-benzimidazol,
5-(2-Chlor-1,1,2-trifluorethoxy)-2-[(4-methoxy-5-methyl-2-pyridyl)methylthio]-1H-benzimidazol,
5-(2-Chlor-1,1,2-trifluorethoxy)-6-methoxy-2-[(4-methoxy-2-pyridyl)methylthio]-1H-benzimidazol,
2-[(4-Allyloxy-3-methyl-2-pyridyl)methylthio]-5-methoxy-6-(1,1,2,2-tetrafluorethoxy)-1H-benzimidazol,
2-[(4-Allyloxy-3-methyl-2-pyridyl)methylthio]-5-(1,1,2,2-tetrafluorethoxy)-1H-benzimidazol,
2-[(4-Allyloxy-2-pyridyl)methylthio]-5-(1,1,2,2-tetrafluorethoxy)-1H-benzimidazol,
2-[(4-Allyloxy-3-methyl-2-pyridyl)methylthio]-5-difluormethoxy-6-methoxy-1H-benzimidazol,
2-[(4-Allyloxy-3-methyl-2-pyridyl]methylthio]-5-difluormethoxy-1H-benzimidazol,
2-[(4-Allyloxy-3,5-dimethyl-2-pyridyl)methylthio]-5-trifluormethoxy-1H-benzimidazol,
6-[(4-Allyloxy-2-pyridyl)methylthio]-2,2-difluor-5H-[1.3]-dioxolo-[4.5-f]-benzimidazol,
6-[(4-Allyloxy-3-methyl-2-pyridyl]methylthio]-2,2-difluor-5H-[1.3]-dioxolo[4.5-f]benzimidazol,
6-[(4-Allyloxy-3,5-dimethyl-2-pyridyl)methylthio]-2,2-difluor-5H-[1.3]-dioxolol[4.5-f]benzimidazol,
2,2-Difluor-6-{[4-(2-methoxyethoxy)-3-methyl-2-pyridyl]methylthio}-5H-[1.3]-dioxolo[4.5-f]benzimidazol,
2-[(4-Allyloxy-2-pyridyl)methylthio]-6,6,7-trifluor-6,7-dihydro-1H-[1.4]-dioxino[2.3-f]benzimidazol
2,2-Difluor-6-[(4-methoxy-2-pyridyl)methylthio]-4-methyl-5H-[1.3]-dioxolo[4.5-f]benzimidazol,
2,2-Difluor-6-[(4-methoxy-3-methyl-2-pyridyl)methylthio]-4-methyl-5H-[1.3]-dioxolo[4.5-f]benzimidazol,
2,2-Difluor-4-methoxy-6-[(4-methoxy-3-methyl-2-pyridyl)methylthio]-5H-[1.3]-dioxolo[4.5-f]-benzimidazol,
4-Difluormethoxy-7-methoxy-2-[(4-methoxy-3-methyl-2-pyridyl)methylthio]-5-methyl-1H-benzimidazol
5-Difluormethoxy-2-[(4-methoxy-3-methyl-2-pyridyl)methylsulfinyl]-4,6-dimethyl-1H-benzimidazol,
5-Difluormethoxy-2-[(4-methoxy-3,5-dimethyl-2-pyridyl)methylsulfinyl]-4,6-dimethyl-1H-benzimidazol,
5-Difluormethoxy-2-{[4-(2-methoxyethoxy)-3-methyl-2-pyridyl]methylsulfinyl}-4,6-dimethyl-1H-benzimidazol,
5-Difluormethoxy-4,6-dimethyl-2-[(4-methyl-2-pyridyl)methylsulfinyl]-1H-benzimidazol,
5-Difluormethoxy-4,6-dimethyl-2-[(3-methyl-2-pyridyl)methylsulfinyl]-1H-benzimidazol,
5-Difluormethoxy-4,6-dimethyl-2-[(5-methyl-2-pyridyl)methylsulfinyl]-1H-benzimidazol,
2-[(4-Allyloxy-3-methyl-2-pyridyl)methylsulfinyl]-5-difluormethoxy-4,6-dimethyl-1H-benzimidazol,
5-Difluormethoxy-4,6-dimethoxy-2-[(4-methoxy-3-methyl-2-pyridyl)methylsulfinyl]-1H-benzimidazol,
5-Difluormethoxy-4,6-dimethoxy-2-[(4-methoxy-3,5-dimethyl-2-pyridyl)methylsulfinyl]-1H-benzimidazol,
5-Difluormethoxy-4,6-dimethoxy-2-[(3,5-dimethyl-2-pyridyl)methylsulfinyl]-1H-benzimidazol,
2-[(4-Allyloxy-3-methyl-2-pyridyl)methylsulfinyl]-5-difluormethoxy-4,6-dimethoxy-1H-benzimidazol,
4-Difluormethoxy-2-[(4-methoxy-2-pyridyl)methylsulfinyl]-6-methyl-1H-benzimidazol,
4-Difluormethoxy-2-[(4-methoxy-3-methyl-2-pyridyl)methylsulfinyl]-6-methyl-1H-benzimidazol,
4-Difluormethoxy-2-[(4-methoxy-3-methyl-2-pyridyl)methylsulfinyl]-7-methyl-1H-benzimidazol,
5-Difluormethoxy-2-[(4-methoxy-2-pyridyl)methylsulfinyl]-4,6,7-trimethyl-1H-benzimidazol,
2-[(4-Allyloxy-3-methyl-2-pyridyl)methylsulfinyl]-5-difluormethoxy-6-methyl-1H-benzimidazol,

5-Difluormethoxy-2-[(4-methoxy-2-pyridyl)methylsulfinyl]-6,7-dimethyl-1H-benzimidazol,
5-Difluormethoxy-2-[(4-methoxy-3-methyl-2-pyridyl)methylsulfinyl]-6,7-dimethyl-1H-benzimidazol,
5-Difluormethoxy-2-[(4-methoxy-3-methyl-2-pyridyl)methylsulfinyl]-6-(2-methoxyethoxy)-1H-benzimidazol,
5-(2-Chlor-1,1,2-trifluorethoxy)-2-[(4-methoxy-2-pyridyl)methylsulfinyl]-4,6-dimethyl-1H-benzimidazol,
5-(2-Chlor-1,1,2-trifluorethoxy)-2-[(4-methoxy-3-methyl-2-pyridyl)methylsulfinyl]-4,6-dimethyl-1H-benzimidazol,
2-[(4-Allyloxy-3-methyl-2-pyridyl)methylsulfinyl]-5-(2-chlor-1,1,2-trifluorethoxy)-1H-benzimidazol,
5-(2-Chlor-1,1,2-trifluorethoxy)-2-[(4-methoxy-2-pyridyl)methylsulfinyl]-1H-benzimidazol,
5-(2-Chlor-1,1,2-trifluorethoxy)-2-[(4-methoxy-3-methyl-2-pyridyl)methylsulfinyl]-1H-benzimidazol,
5-(2-Chlor-1,1,2-trifluorethoxy)-2-[(4-methoxy-5-methyl-2-pyridyl)methylsulfinyl]-1H-benzimidazol,
5-(2-Chlor-1,1,2-trifluorethoxy)-6-methoxy-2-[(4-methoxy-2-pyridyl)methylsulfinyl]-1H-benzimidazol,
2-[(4-Allyloxy-3-methyl-2-pyridyl)methylsulfinyl]-5-methoxy-6-(1,1,2,2-tetrafluorethoxy)-1H-benzimidazol,
2-[(4-Allyloxy-3-methyl-2-pyridyl)methylsulfinyl]-5-(1,1,2,2-tetrafluorethoxy)-1H-benzimidazol,
2-[(4-Allyloxy-2-pyridyl)methylsulfinyl]-5-(1,1,2,2-tetrafluorethoxy)-1H-benzimidazol,
2-[(4-Allyloxy-3-methyl-2-pyridyl)methylsulfinyl]-5-difluormethoxy-6-methoxy-1H-benzimidazol,
2-[(4-Allyloxy-3-methyl-2-pyridyl)methylsulfinyl]-5-difluormethoxy-1H-benzimidazol,
2-[(4-Allyloxy-3,5-dimethyl-2-pyridyl)methylsulfinyl]-5-trifluormethoxy-1H-benzimidazol,
6-[(4-Allyloxy-2-pyridyl)methylsulfinyl]-2,2-difluor-5H-[1.3]-dioxolo-[4.5-f]-benzimidazol,
6-[(4-Allyloxy-3-methyl-2-pyridyl)methylsulfinyl]-2,2-difluor-5H-[1.3]-dioxolo[4.5-f]benzimidazol,
6-[(4-Allyloxy-3,5-dimethyl-2-pyridyl)methylsulfinyl]-2,2-difluor-5H-[1.3]-dioxolo[4.5-f]benzimidazol,
2,2-Difluor-6-{[4-(2-methoxyethoxy)-3-methyl-2-pyridyl]methylsulfinyl}-5H-[1.3]-dioxolo[4.5-f]benzimidazol,
2-[(4-Allyloxy-2-pyridyl)methylsulfinyl]-6,6,7-trifluor-6,7-dihydro-1H-[1.4]-dioxino[2.3-f]benzimidazol
2,2-Difluor-6-[(4-methoxy-2-pyridyl)methylsulfinyl]-4-methyl-5H-[1.3]-dioxolo[4.5-f]benzimidazol,
2,2-Difluor-6-[(4-methoxy-3-methyl-2-pyridyl)methylsulfinyl]-4-methyl-5H-[1.3]-dioxolo[4.5-f]benzimidazol,
2,2-Difluor-4-methoxy-6-[(4-methoxy-3-methyl-2-pyridyl)methylsulfinyl]-5H-[1.3]-dioxolo[4.5-f]-benzimidazol,
4-Difluormethoxy-7-methoxy-2-[(4-methoxy-3-methyl-2-pyridyl)methylsulfinyl]-5-methyl-1H-benzimidazol
und ihre Salze.

Ein weiterer Gegenstand der Erfindung ist ein Verfahren zur Herstellung der Verbindungen der Formel I, worin R1, R2, R3, R4, R5, R6, R7, R8, R9, R10 und n die oben angegebenen Bedeutungen haben, und ihrer Salze.

Das Verfahren ist dadurch gekennzeichnet, daß man

a) Mercaptobenzimidazole der Formel II mit Picolinderivaten III,

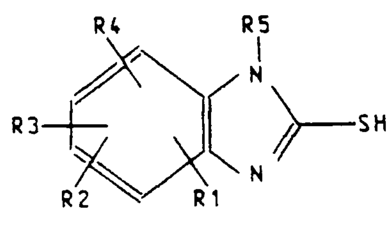

(II),

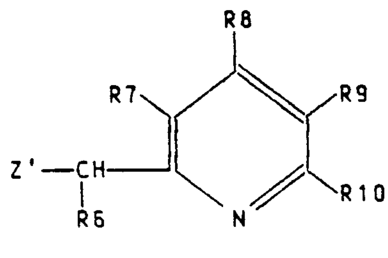

(III),

oder

b) Benzimidazole der Formel IV mit Mercaptopicolinen V,

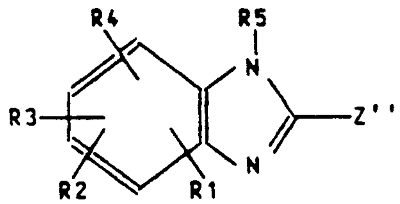

(IV),

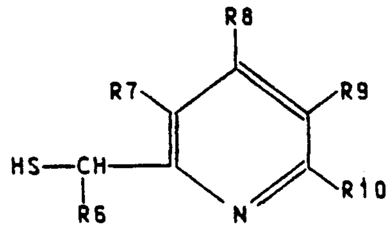

(V),

oder

c) o-Phenylendiamine der Formel VI mit Ameisensäurederivaten VII

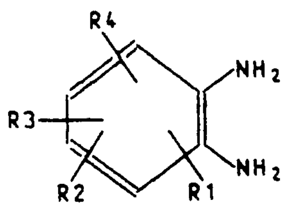

(VI),

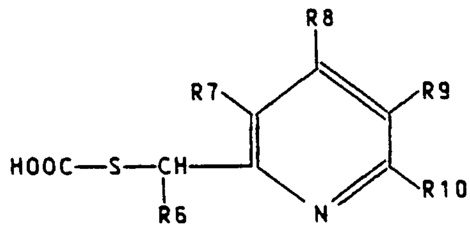

(VII),

umsetzt und (falls Verbindungen der Formel I mit n = 1 die gewünschten Endprodukte sind) anschließend die nach a), b) oder c) erhaltenen 2-Benzimidazolyl-2-pyridylmethyl-sulfide der Formel VIII (= Verbindungen der Formel I mit n = 0)

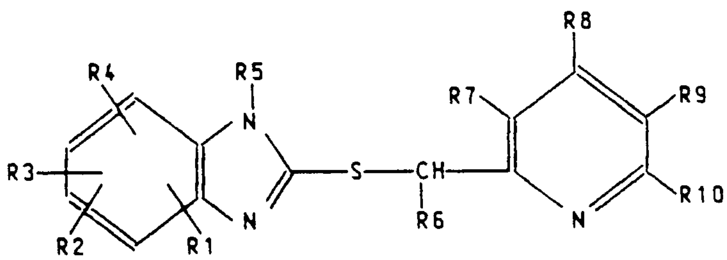

(VIII),

oxidiert und/oder gewünschtenfalls in die Salze überführt,

oder daß man

d) Benzimidazole der Formel IX mit Pyridinderivaten X

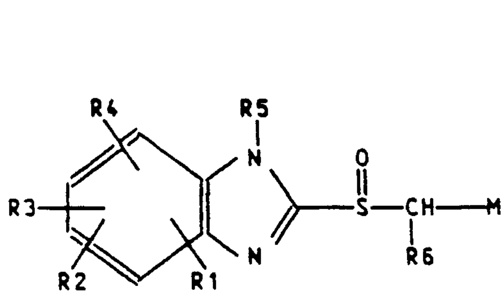

(IX),

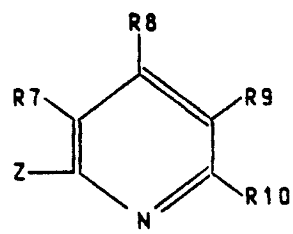

(X),

oder

e) Sulfinylderivate der Formel XI mit 2-Picolinderivaten XII

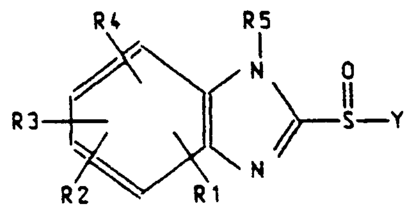

(XI),

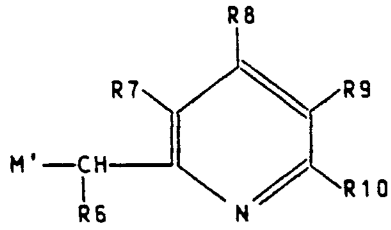

(XII),

umsetzt und gewünschtenfalls anschließend in die Salze überführt, oder

f) - falls Verbindungen der Formel I, worin R5 eine unter physiologischen Bedingungen leicht abspaltbare Gruppe darstellt, die herzustellenden Verfahrensprodukte sind - daß man Verbindungen der Formel I, worin R5 Wasserstoff bedeutet, mit Verbindungen der Formel R5'-Y' (XIII), worin R5' die gewünschte, unter physiologischen Bedingungen leicht abspaltbare Gruppe oder gemeinsam mit Y' ihr Vorläufer ist, umsetzt und gewünschtenfalls anschließend in die Salze überführt, oder

g) - falls Verbindungen der Formel I, worin R5 Wasserstoff bedeutet, die herzustellenden Verfahrensprodukte sind - daß man Verbindungen der Formel I, worin R5 eine unter physiologischen Bedingungen leicht abspaltbare Gruppe darstellt, solvolysiert, und die erhaltenen Produkte gewünschtenfalls in die Salze überführt, oder

h) - falls Verbindungen der Formel I, worin R6 $C_1$-$C_6$-Alkyl bedeutet, die herzustellenden Verfahrensprodukte sind - daß man Verbindungen der Formel I, worin R6 Wasserstoff bedeutet, mit Verbindungen der Formel R6-Y'' (XIV), worin R6 $C_1$-$C_6$-Alkyl bedeutet, alkyliert und gewünschtenfalls anschließend in die Salze überführt,

wobei Y, Y'', Z, Z' und Z'' geeignete Abgangsgruppen darstellen, Y' eine Abgangs- bzw. Reaktivgruppe darstellt, M für ein Alkalimetallatom (Li, Na oder K) steht, M' für das Äquivalent eines Metallatoms steht und R1, R2, R3, R4, R5, R6, R7, R8, R9, R10 und n (sofern nicht anders definiert) die oben angegebenen Bedeutungen haben.

Bei den vorstehend aufgeführten Umsetzungen können die Verbindungen II-XIV als solche oder gegebenenfalls in Form ihrer Salze eingesetzt werden.

Die Herstellungsverfahren a), b) und c) führen zu den erfindungsgemäßen Sulfiden, die Oxidation der Verbindungen VIII und die Verfahren d) und e) liefern die erfindungsgemäßen Sulfoxide, die Verfahren f), g) und h) führen zu beiden Produktklassen.

Welche Abgangsgruppen Y, Y', Y'', Z, Z' bzw. Z'' geeignet sind, ist dem Fachmann aufgrund seines Fachwissens geläufig. Eine geeignete Abgangsgruppe Y ist beispielsweise eine Gruppe, die zusammen mit der Sulfinylgruppe, an die sie gebunden ist, ein reaktives Sulfinsäurederivat bildet. Als geeignete Abgangsgruppe Y seien beispielsweise Alkoxy-, Dialkylamino-oder Alkylmercaptogruppen genannt. Eine geeignete Abgangs- bzw. Reaktivgruppe Y' ist eine Gruppe, die mit einer sekundären Aminogruppe unter Abspaltung von HY' oder unter Anlagerung zu reagieren in der Lage ist. Bei Verbindungen der Formel XIII, in denen R5' insbesondere eine substituierte Carbonylgruppe darstellt, ist Y' beispielsweise eine Abgangsgruppe, die zusammen mit der Carbonylgruppe, an die sie gebunden ist, ein reaktives Carbonsäurederivat, z.B. ein Säurehalogenid, bildet. Erfindungsgemäß sind durch die allgemeine Formel R5'-Y' (XIII) auch solche Verbindungen (Vorläufer der unter physiologischen Bedingungen leicht abspaltbaren Gruppe R5) umfaßt, bei denen Y' eine Reaktivgruppe darstellt (z.B. Isonitrile), die bei der Reaktion mit der sekundären Aminogruppe keine Kondensation unter Abspaltung von HY' sondern eine Addition unter Bildung der gewünschten abspaltbaren Gruppe R5 durchlaufen.

Die Abgangsgruppe Y'' ist eine dem Fachmann für Alkylierungsreaktionen geläufige Gruppe, die bei der Alkylierung - z.B. mit Dialkylsulfat, Fluorsulfonsäurealkylester oder Alkyliodid - abgeht.

Als geeignete Abgangsgruppen Z, Z' bzw. Z'' seien beispielsweise Halogenatome, insbesondere Chloratome, oder durch Veresterung (z.B. mit p-Toluolsulfonsäure) aktivierte Hydroxylgruppen genannt.

Das Äquivalent eines Metallatoms M' ist beispielsweise ein Alkalimetall (Li, Na oder K), oder ein Erdalkalimetallatom (z.B. Mg), das durch ein Halogenatom (z.B. Br, Grignard-Reagenz) substituiert ist, oder irgendein anderes, gegebenenfalls substituiertes Metallatom, von dem bekannt ist, daß es bei Substitutionsreaktionen metallorganischer Verbindungen wie die obenerwähnten Metalle reagiert.

Die Umsetzung von II mit III erfolgt in an sich bekannter Weise in geeigneten, vorzugsweise polaren protischen oder aprotischen Lösungsmitteln (wie Methanol, Isopropanol, Dimethylsulfoxid, Aceton, Dimethylformamid oder Acetonitril) unter Zusatz oder unter Ausschluß von Wasser. Sie wird beispielsweise in Gegenwart eines Protonenakzeptors durchgeführt. Als solche eignen sich Alkalimetallhydroxide, wie Natriumhydroxid, Alkalimetallcarbonate, wie Kaliumcarbonat, oder tertiäre Amine, wie Pyridin, Triethylamin oder Ethyldiisopropylamin. Alternativ kann die Umsetzung auch ohne Protonenakzeptor durchgeführt werden, wobei - je nach Art der Ausgangsverbindungen - gegebenenfalls zunächst die Säureadditionssalze in besonders reiner Form abgetrennt werden können. Die Reaktionstemperatur kann zwischen $0^0$ und $150^0C$ liegen, wobei in Gegenwart von Protonenakzeptoren Temperaturen zwischen $20^0$ und $80^0C$ und ohne Protonenakzeptoren zwischen $60^0$ und $120^0C$ - insbesondere die Siedetemperatur der verwendeten Lösungsmittel - bevorzugt sind. Die Reaktionszeiten liegen zwischen 0,5 und 12 Stunden.

Bei der Umsetzung von IV mit V, die in an sich bekannter Weise erfolgt, kommen ähnliche Reaktionsbedingungen wie bei der Umsetzung von II mit III zur Anwendung.

Die Reaktion von VI mit VII wird bevorzugt in polaren, gegebenenfalls wasserhaltigen Lösungsmitteln in Gegenwart einer starken Säure, z.B. Salzsäure, insbesondere bei der Siedetemperatur des verwendeten Lösungsmittels durchgeführt.

Die Oxidation der Sulfide VIII erfolgt in an sich bekannter Weise und - unter den Bedingungen, wie sie dem Fachmann für die Oxidation von Sulfiden zu Sulfoxiden geläufig sind {siehe hierzu z.B. J. Drabowicz und M. Mikolajczyk, Organic preparations and procedures int. 14(1-2), 45-89(1982) oder E. Block in S. Patai, The Chemistry of Functional Groups, Supplement E. Part 1, S. 539-608, John Wiley and Sons (Interscience Publication), 1980}. Als Oxidationsmittel kommen alle für die Oxidation von Sulfiden zu Sulfoxiden üblicherweise verwendeten Reagenzien in Frage, insbesondere Peroxysäuren, wie z.B. Peroxyessigsäure, Trifluorperoxyessigsäure, 3,5-Dinitroperoxybenzoesäure, Peroxymaleinsäure oder bevorzugt m-Chlorperoxybenzoesäure.

Die Reaktionstemperatur liegt (je nach Reaktivität des Oxidationsmittels und Verdünnungsgrad) zwischen $-70^0C$ und der Siedetemperatur des verwendeten Lösungsmittels, bevorzugt jedoch zwischen $-30^0$ und $+20^0C$. Als vorteilhaft hat sich auch die Oxidation mit Halogenen bzw. mit Hypohalogeniten (z.B. mit wäßriger Natriumhypochloritlösung) erwiesen, die zweckmäßigerweise bei Temperaturen zwischen $0^0$ und $30^0C$ durchgeführt wird. Die Reaktion wird zweckmäßigerweise in inerten Lösungsmitteln, z. B. aromatischen oder chlorierten Kohlenwasserstoffen, wie Benzol, Toluol, Dichlormethan oder Chloroform, vorzugsweise in Estern oder Ethern, wie Essigsäureethylester, Essigsäureisopropylester oder Dioxan durchgeführt.

Die Umsetzung von IX mit X erfolgt bevorzugt in inerten Lösungsmitteln, wie sie auch für die Reaktion von Enolationen mit Alkylierungsmitteln üblicherweise verwendet werden. Beispielsweise seien aromatische Lösungsmittel wie Benzol oder Toluol genannt. Die Reaktionstemperatur liegt (je nach Art des Alkalimetallatoms M und der Abgangsgruppe Z) in der Regel zwischen $0^0$ und $120^0C$, wobei die Siedetemperatur des verwendeten Lösungsmittels bevorzugt ist. Beispielsweise [wenn M Li(Lithium) und Z Cl(Chlor) darstellt und die Umsetzung in Benzol durchgeführt wird] ist die Siedetemperatur von Benzol ($80^0C$) bevorzugt.

Die Verbindungen XI werden mit den Verbindungen XII in an sich bekannter Weise umgesetzt, wie sie dem Fachmann für die Reaktion metallorganischer Verbindungen geläufig ist.

Die Umsetzung gemäß Verfahrensvariante f) erfolgt in dem Fachmann bekannter Weise in geeigneten Lösungsmitteln wie Tetrahydrofuran oder Acetonitril, gegebenenfalls unter Zusatz einer Base (falls Y' eine Abgangsgruppe darstellt) oder auch ohne Basenzusatz (falls Y' eine Reaktivgruppe darstellt). Bei unsymmetrischer Verteilung der Substituenten R1, R2, R3 und R4 liefert diese Reaktion Isomerengemische, die durch geeignete Trennverfahren (z.B. Chromatographie) aufgetrennt werden müssen.

Die Solvolyse gemäß Verfahrensvariante g) erfolgt in dem Fachmann bekannter Weise in geeigneten, Wasser enthaltenden oder Wasser abspaltenden alkalischen oder sauren Lösungen, bei Raumtemperatur oder gewünschtenfalls unter Erwärmen bis zur Siedetemperatur des eingesetzten Lösungsmittels.

Die Alkylierung gemäß Verfahrensvariante h) erfolgt - gegebenenfalls nach vorheriger Deprotonierung - in dem Fachmann geläufiger Weise in geeigneten inerten Lösungsmitteln.

Je nach Art der Ausgangsverbindungen, die gegebenenfalls auch in Form ihrer Salze eingesetzt werden können, und in Abhängigkeit von den Reaktionsbedingungen werden die erfindungsgemäßen Verbindungen zunächst entweder als solche oder in Form ihrer Salze gewonnen.

Im übrigen erhält man die Salze durch Auflösen der freien Verbindungen in einem geeigneten Lösungsmittel, z.B. in einem chlorierten Kohlenwasserstoff, wie Methylenchlorid oder Chloroform, einem niedermolekularen aliphatischen Alkohol (Ethanol, Isopropanol), einem Ether (Diisopropylether), einem Keton (Aceton) oder Wasser, das die gewünschte Säure bzw. Base enthält, oder dem die gewünschte Säure bzw. Base - gegebenenfalls in der genau berechneten stöchiometrischen Menge - anschließend zugegeben wird.

Die Salze werden durch Filtrieren, Umfällen, Ausfällen oder durch Verdampfen des Lösungsmittels

gewonnen.

Erhaltene Salze können durch Alkalisieren bzw. Ansäuern, z.B. mit wäßrigem Natriumhydrogencarbonat bzw. mit verdünnter Salzsäure, in die freien Verbindungen umgewandelt werden, welche wiederum in die Salze übergeführt werden können. Auf diese Weise lassen sich die Verbindungen reinigen, oder es lassen sich pharmakologisch nicht verträgliche Salze in pharmakologisch verträgliche Salze umwandeln.

Die erfindungsgemäßen Sulfoxide sind optisch aktive Verbindungen. Je nach Art der Substituenten R1 bis R10 können noch weitere Chiralitätszentren im Molekül sein (z.B. wenn R6 kein Wasserstoff ist). Die Erfindung umfaßt daher sowohl die Enantiomeren und Diastereomeren als auch ihre Mischungen und Racemate. Die Enantiomeren können in an sich bekannter Weise (beispielsweise durch Herstellung und Trennung entsprechender diastereoisomerer Verbindungen) separiert werden. Die Enantiomeren können aber auch durch asymmetrische Synthese, beispielsweise durch Reaktion optisch aktiver reiner Verbindungen XI oder diastereoisomer reiner Verbindungen XI mit Verbindungen XII hergestellt werden [siehe hierzu K.K. Andersen, Tetrahedron Lett., 93 (1962)].

Die erfindungsgemäßen Verbindungen werden bevorzugt durch Umsetzung von II mit III und gegebenenfalls anschließende Oxidation des entstandenen Sulfids VIII synthetisiert.

Die Verbindungen II, IV, VI, IX und XI sind neu. Sie können nach bekannten Vorschriften ausgehend von bekannten oder in bekannter Weise erhältlichen Vorprodukten in analoger Weise erhalten werden [siehe z.B. DE-OS 28 48 531; J. Org. Chem. 44, 2907-2910 (1979); J. Org. Chem. 29, 1-11 (1964); DE-OS 20 29 556; J. Fluorine Chem. 18, 281-91 (1981); Synthesis 1980, 727-8; Bull. Soc. Chim. France 4, 581-592 (1957); C.A. 60, 13352h (1964)].

Die Verbindungen III, V, VII, X und XII sind teils bekannt oder können nach bekannten Vorschriften analog hergestellt werden (siehe z. B. DE-OS 34 04 610), bzw. sie können - je nach Substitutionsmuster - z.B. wie folgt hergestellt werden:

1. Verbindungen III mit R7 und R8 = 1-3C-Alkoxy und R9 = Wasserstoff oder 1-3C-Alkyl.

Diese Verbindungen werden z.B. ausgehend von bekannten oder auf bekanntem Wege herstellbaren 3-Hydroxy- bzw. 3-Hydroxy-5-alkyl-pyridinen durch Benzylierung der Hydroxygruppe (z.B. mit Kaliumhydroxid und Benzylchlorid in Dimethylsulfoxid), N-Oxidation (z.B. mit 30 %-igem Wasserstoffperoxid), Nitrierung in 4-Position (z.B. mit Nitriersäure), Austausch der Nitrogruppe gegen die 1-3C-Alkoxygruppe (z.B. durch Umsetzung mit Alkali-alkoxid), reduktive Debenzylierung und gleichzeitige N-Deoxygenierung (z.B. mit Wasserstoff an Palladium/Kohle in saurem Medium), Einführung der Hydroxymethylgruppe in o-Position zum Pyridinstickstoff (z.B. durch Umsetzung mit alkalischer Formalinlösung), Umwandlung der 3-Hydroxy- in eine 1-3C-Alkoxygruppe (z.B. durch Alkylierung mit 1-3C-Alkyliodid in basischem Medium) und Einführung der Fluchtgruppe Z' (z.B. durch Umsetzung mit Thionylchlorid) hergestellt. In einer bevorzugten Alternative werden die Verbindungen ausgehend von bekannten oder auf bekanntem Wege herstellbaren 3-Hydroxy-2-alkyl- bzw. 3-Hydroxy-2,5-dialkyl-pyridinen durch Alkylierung der Hydroxygruppe (z.B. mit Kaliumhydroxid und Methyliodid in Dimethylsulfoxid), N-Oxidation (z.B. mit 30%igem Wasserstoffperoxid), Nitrierung in 4-Position (z.B. mit Salpetersäure), Austausch der Nitrogruppe gegen die 1-3C-Alkoxygruppe (z.B. durch Umsetzung mit Alkalialkoxid), Umwandlung in das 2-Acetoxymethylpyridin (z.B. mit heißem Essigsäureanhydrid), Verseifung (z.B. mit verdünnter Natronlauge) zur Hydroxymethylgruppe und Einführung der Fluchtgruppe Z' (z.B. durch Umsetzung mit Thionylchlorid) hergestellt.

2. Verbindungen III mit R8 und R9 = 1-3C-Alkoxy und R7 = Wasserstoff.

Diese Verbindungen werden z.B. ausgehend vom bekannten 5-Hydroxy-2-methylpyridinen durch Alkylierung der Hydroxygruppe (z.B. mit 1-3C-Alkyliodid und Kaliumhydroxid in Dimethylsulfoxid), N-Oxidation (z.B. mit 30 %-igem Wasserstoffperoxid), Nitrierung in 4-Position (z.B. mit Nitriersäure), Austausch der Nitrogruppe gegen die 1-3C-Alkoxygruppe (z.B. durch Umsetzung mit Alkalialkoxid), Umwandlung in das 2-Acetoxymethylpyridin (z.B. mit heißem Essigsäureanhydrid), Verseifung (z.B. mit verdünnter Natronlauge) zur 2-Hydroxymethylgruppe und Einführung der Fluchtgruppe Z' (z.B. durch Umsetzung mit Thionychlorid) hergestellt.

3. Verbindungen III mit R8 und R9 = 1-3C-Alkoxy und R7 = 1-3C-Alkyl.

Diese Verbindungen werden z.B. ausgehend von bekannten oder auf bekanntem Weg herstellbaren 2-Methyl-3-alkyl-4-alkoxypyridinen (siehe z.B. EP-A 0 080 602) durch N-Oxidation (z.B. mit 30 %-igem Wasserstoffperoxid), gezielte Acetoxylierung und anschließende Verseifung in 5-Postition (z.B. mit Essigsäureanhydrid und anschließend Natronlauge), Alkylierung der 5-Hydroxygruppe (z.B. mit 1-3C-Alkyliodid und Natronlauge in Dimethylsulfoxid), N-Oxidation (z.B. mit m-Chlorperoxibenzoesäure), Umwandlung in das 2-Acetoxymethylpyridin (z.B. mit heißem Essigsäureanhydrid), Verseifung (z.B. mit verdünnter Natronlauge) zur 2-Hydroxymethylgruppe und Einführung der Fluchtgruppe Z' (z.B. durch Umsetzung mit Thionylchlorid) hergestellt.

Welche Reaktionsbedingungen (Temperaturen, Reaktionszeiten, Lösungsmittel etc.) bei den oben skizzierten Synthesewegen für die Herstellung der Verbindungen III im einzelnen erforderlich sind, ist dem Fachmann aufgrund seines Fachwissens geläufig. Die genaue Herstellung einzelner Vertreter der Verbindungen III ist in den Beispielen angegeben. Die Herstellung weiterer Vertreter erfolgt in analoger Weise.

Verbindungen der Formel III, in denen R6 eine von Wasserstoff verschiedene Bedeutung hat, werden z.B. aus den entsprechenden Pyridin-2-alkylverbindungen durch Umsetzung mit Acetanhydrid, anschließende Reaktion mit Natronlauge und Umwandlung des entstandenen Alkohols in die entsprechende Halogenverbindung (z.B. mit Thionylchlorid) in dem Fachmann bekannter Weise erhalten.

Die Verbindungen V, VII und XII werden beispielsweise ausgehend von den Verbindungen III auf für den Fachmann bekannten Wegen hergestellt. Die Verbindungen X werden in Anlehnung an Z. Talik, Roczniki Chem. 35, 475 (1961) hergestellt.

Die folgenden Beispiele erläutern die Erfindung näher ohne sie einzu schränken. Die in den Beispielen namentlich aufgeführten Verbindungen der allgemeinen Formel I sowie die Salze dieser Verbindungen sind be vorzugter Gegenstand der Erfindung. Schmp. bedeutet Schmelzpunkt, Kp. bedeutet Siedepunkt, für Stunde(n) wird die Abkürzung h und für Minuten die Abkürzung Min. verwendet. Unter "Ether" wird Diethylether verstanden.

## Beispiele

### Endprodukte

1. 5-Difluormethoxy-2-[(4-methoxy-3-methyl-2-pyridyl)methylsulfinyl]-4,6-dimethyl-1H-benzimidazol

Zu einer Lösung von 0,80 g 5-Difluormethoxy-2-[(4-methoxy-3-methyl-2-pyridyl)methylthio]-4,6-dimethyl-1H-benzimidazol in 40 ml Dichlormethan tropft man bei -40°C eine Lösung von 0,47 g 81%ige m-Chlorperoxybenzoesäure in 40 ml Dichlormethan. Man rührt noch 2 h bei -40°C setzt bei -20°C 0,31 ml Triethylamin zu und extrahiert bei 0°C mit Kaliumbicarbonatlösung und Wasser. Man trocknet, engt ein und läßt aus Diisopropylether kristallisieren. Man erhält 0,6 g (73%) der Titelverbindung vom Schmp. 182-183°C (Zersetzung).

Analog erhält man aus
5-Difluormethoxy-2-[(4-methoxy-2-pyridyl)methylthio]-4,6-dimethyl-1H-benzimidazol,
4-Difluormethoxy-5,6-dimethoxy-2-[(4-methoxy-3-methyl-2-pyridyl)methylthio]-1H-benzimidazol,
4-Difluormethoxy-5,6-dimethoxy-2-[(4-methoxy-2-pyridyl)methylthio]-1H-benzimidazol,
5-Difluormethoxy-6-methoxy-2-{[1-(4-methoxy-2-pyridyl)ethyl]thio}-1H-benzimidazol,
5-Difluormethoxy-2-{[1-(4-methoxy-2-pyridyl)ethyl]thio}-6-methyl-1H-benzimidazol und
5-(2-Chlor-1,1,2-trifluorethoxy)-2-{[1-(4-methoxy-2-pyridyl)ethyl]thio}-1H-benzimidazol
durch Oxidation mit m-Chlorperoxybenzoesäure
5-Difluormethoxy-2-[(4-methoxy-2-pyridyl)methylsulfinyl]-4,6-dimethyl-1H-benzimidazol (Schmp. 163- 164°C unter Zersetzung),
4-Difluormethoxy-5,6-dimethoxy-2-[(4-methoxy-3-methyl-2-pyridyl)methylsulfinyl]-1H-benzimidazol (Schmp. 180-181°C unter Zersetzung),
4-Difluormethoxy-5,6-dimethoxy-2-[(4-methoxy-2-pyridyl)methylsulfinyl]-1H-benzimidazol (Schmp. 154-155°C unter Zersetzung),
5-Difluormethoxy-6-methoxy-2-{[1-(4-methoxy-2-pyridyl)ethyl]sulfinyl}-1H-benzimidazol (Schmp. 152-153°C unter Zersetzung; aus Essigester),
5-Difluormethoxy-2-{[1-(4-methoxy-2-pyridyl)ethyl]sulfinyl}-6-methyl-1H-benzimidazol (Schmp. 144-145°C unter Zersetzung) und
5-(2-Chlor-1,1,2-trifluorethoxy)-2-{[1-(4-methoxy-2-pyridyl)ethyl]sulfinyl}-1H-benzimidazol (Schmp. 139-140°C unter Zersetzung, aus Essigester/Diethylether).

2. 5-Difluormethoxy-2-[(4-methoxy-3-methyl-2-pyridyl)methylthio]-4,6-dimethyl-1H-benzimidazol

2,0 g 5-Difluormethoxy-4,6-dimethyl-1H-benzimidazol-2-thiol und 1,72 g 2-Chlormethyl-4-methoxy-3-methylpyridin-hydrochlorid werden in 30 ml 2-Propanol 4 h zum Sieden erhitzt. Man erhält 3,6 g des Dihydrochlorids der Titelverbindung. Man löst in Wasser und konzentrierter Salzsäure, klärt mit Aktivkohle, fällt mit verdünnter Natronlauge und kristallisiert den Niederschlag aus Butanol um. Man erhält 2,6 g (85%) der Titelverbindung (Schmp. 190-191°C).

Analog erhält man durch Umsetzung des Thiols mit 2-Chlormethyl-4-methoxy-pyridin-hydrochlorid

5-Difluormethoxy-2-[(4-methoxy-2-pyridyl)methylthio]-4,6-dimethyl-1H-benzimidazol (Schmp. 168-169$^0$C).

Analog erhält man durch Umsetzung von
5-(2-Chlor-1,1,2-trifluorethoxy-1H-benzimidazol-2-thiol mit 2-Chlormethyl-4-methoxypyridin-hydrochlorid
5-(2-Chlor-1,1,2-trifluorethoxy)-2-[(4-methoxy-2-pyridyl)methylthio]-1H-benzimidazol (Schmp. 120$^0$C, aus Essigester/Toluol; Dihydrochlorid Schmp. 197-198$^0$C)
und mit 2-Chlormethyl-4-methoxy-3-methylpyridin-hydrochlorid
5-(2-Chlor-1,1,2-trifluorethoxy)-2-[(4-methoxy-3-methyl-2-pyridyl)-methylthio]-1H-benzimidazol (Schmp. 111$^0$C, aus Essigester).

3. <u>4-Difluormethoxy-5,6-dimethoxy-2-[(4-methoxy-3-methyl-2-pyridyl)methylthio]-1H-benzimidazol</u>

1,0 g 4-Difluormethoxy-5,6-dimethoxy-1H-benzimidazol-2-thiol, 0,75 g 2-Chlormethyl-4-methoxy-3-methylpyridin-hydrochlorid, 0,3 g Natriumhydroxid, 2 ml Wasser und 10 ml Ethanol werden 2,5 h bei 60$^0$C gerührt. Man gießt auf 100 ml Wasser und kühlt im Eisbad. Man erhält 1,3 g (87%) der Titelverbindung (Schmp. 177-179$^0$C, aus Diisopropylether).

Analog erhält man durch Umsetzung des Thiols mit 2-Chlormethyl-4-methoxypyridin-hydrochlorid
4-Difluormethoxy-5,6-dimethoxy-2-[(4-methoxy-2-pyridyl)methylthio]-1H-benzimidazol (Schmp. 112-114$^0$C).

Analog erhält man durch Umsetzung von
5-Difluormethoxy-6-methoxy-1H-benzimidazol-2-thiol,
5-Difluormethoxy-6-methyl-1H-benzimidazol-2-thiol und
5-(2-Chlor-1,1,2-trifluorethoxy)-1H-benzimidazol-2-thiol mit 2-(1-Chlorethyl)-4-methoxypyridin-hydrochlorid
5-Difluormethoxy-6-methoxy-2-{[1-(4-methoxy-2-pyridyl)ethyl]thio}-1H-benzimidazol (Schmp. 128-131$^0$C, aus Toluol),
5-Difluormethoxy-2-{[1-(4-methoxy-2-pyridyl)ethyl]thio}-6-methyl-1H-benzimidazol (Schmp. 183-184$^0$C, aus Essigester/Toluol) und
5-(2-Chlor-1,1,2-trifluorethoxy)-2-{[1-(4-methoxy-2-pyridyl)ethyl]thio}-1H-benzimidazol (Schmp. 120-122$^0$C, aus Toluol/Diisopropylether).

4. <u>5-(2-Chlor-1,1,2-trifluorethoxy)-2-[(4-methoxy-2-pyridyl)methylsulfinyl]-1H-benzimidazol</u>

Zu einer Lösung von 5,0 g 5-(2-Chlor-1,1,2-trifluorethoxy-2-[(4-methoxy-2-pyridyl)methylthio]-1H-benzimidazol in 50 ml Essigester tropft man bei -10$^0$C 2,6 ml 4 n Natronlauge und eine Lösung von 24 ml Natriumhypochloritlösung (6,6% aktives Chlor) in 9,3 ml 6 n Natronlauge, rührt noch 20 Min. und zerstört das Oxidationsmittel mit wäßriger Natriumthiosulfatlösung. Man trennt die organische Schicht ab, neutralisiert mit Kaliumdihydrogenphosphatlösung, trocknet die organische Schicht mit Magnesiumsulfat, klärt mit Aktivkohle und engt im Vakuum zur Trockne ein. Der Rückstand wird aus Essigester und Diisopropylether umkristallisiert. Man erhält 2,9 g (56%) der Titelverbindung vom Schmp. 109-110$^0$C (Zersetzung).

Analog erhält man aus
5-(2-Chlor-1,1,2-trifluorethoxy)-2-[(4-methoxy-3-methyl-2-pyridyl)-methylthio]-1H-benzimidazol durch Umsetzung mit Natriumhypochloritlösung 5-(2-Chlor-1,1,2-trifluorethoxy)-2-[(4-methoxy-3-methyl-2-pyridyl)-methylsulfinyl]-1H-benzimidazol (Schmp. 147-148$^0$C unter Zersetzung, Natrium-Salz 252-254$^0$C unter Zersetzung).

5. <u>5-Difluormethoxy-2-[(4-methoxy-3-methyl-2-pyridyl)methylsulfinyl]-4,6-dimethyl-1H-benzimidazol-Natriumsalz und Calciumsalz</u>

Zu 0,638 g 5-Difluormethoxy-2-[(4-methoxy-3-methyl-2-pyridyl)methylsulfinyl]-4,6-dimethyl-1H-benzimidazol in 10 ml Ethanol fügt man 0,30 ml 30%iges Natriummethanolat in Methanol und rührt bis zur Auflösung. Man engt am Rotationsverdampfer ein und versetzt die eine Hälfte des Rückstands mit 15 ml Wasser, engt im Vakuum zur Trokene ein und trocknet im Hochvakuum bei 70-120$^0$C. Man erhält das Natriumsalz vom Schmp. 250-252$^0$C (Zersetzung).
Die andere Hälfte wird in 15 ml Wasser gelöst und mit einer Lösung von 60 mg Calciumchlorid in 5 ml Wasser gefällt und der Niederschlag mit Wasser gewaschen und bei 70$^0$C im Hochvakuum getrocknet. Man erhält das Calciumsalz vom Schmp. 213-215$^0$C (Zersetzung).

6. <u>5-Difluormethoxy-2-[(3,4-dimethoxy-2-pyridyl)methylsulfinyl]-4,6-dimethyl-1H-benzimidazol</u>

Zu einer Lösung von 0,39 g (1 mmol) 5-Difluormethoxy-2-[(3,4-dimethoxy-2-pyridyl)methylthio]-4,6-dimethyl-1H-benzimidazol in 20 ml Methylenchlorid werden bei -40°C 5 ml einer 0,2 m Lösung von m-Chlorperoxybenzoesäurein Methylenchlorid zugetropft und 15 Min. gerührt. Anschließend gibt man 0,5 ml Triethylamin zu und gießt die Reaktionsmischung in 20 ml eines 1:1 Gemisches aus jeweils 5%iger Natriumcarbonat- und Natriumthiosulfatlösung. Nach Phasentrennung wird noch zweimal mit je 30 ml Methylenchlorid extrahiert, die vereinigten, organischen Phasen mit Natriumcarbonatlösung gewaschen, über Magnesiumsulfat getrocknet, filtriert und auf 2 ml eingeengt. Nach Kristallisation aus Diisopropylether wird filtriert und bis zur Gewichtskonstanz getrocknet. Man erhält 0,3 g (74% der Theorie) der Titelverbindung als farblosen Feststoff vom Schmp. 163-164°C (Zersetzung).

Analog erhält man durch Oxidation von 5-[(2-Chlor-1,1,2-trifluor)ethoxy]-2-[(3,4-dimethoxy-2-pyridyl)-methylthio]-1H-benzimidazol mit m-Chlorperoxybenzoesäure 5-[(2-Chlor-1,1,2-trifluor)ethoxy]-2-[(3,4-dimethoxy-2-pyridyl)methylsulfinyl]-1H-benzimidazol.

7. <u>5-Difluormethoxy-2-[(3,4-dimethoxy-2-pyridyl)methylthio]-4,6-dimethyl-1H-benzimidazol</u>

0,45 g (1,8 mmol) 5-Difluormethoxy-4,6-dimethyl-2-mercaptobenzimidazol und 0,41 g (1,8 mmol) 2-Chlormethyl-3,4-dimethoxypyridinium-chlorid werden in 20 ml trockenem Isopropanol unter Rückfluß zum Sieden erhitzt. Dabei entsteht zwischenzeitlich eine klare Lösung, aus der ein farbloser Feststoff ausfällt. Nach 3 h läßt man abkühlen, filtriert, wäscht den Filterrückstand mit Isopropanol, löst ihn anschließend in Wasser und stellt mit 1 N Natronlauge unter Rühren auf pH 9. Das ausgefallene Produkt wird filtriert, einmal in 20 ml Wasser suspendiert, erneut filtriert und neutral gewachen. Nach Trocknung bis zur Gewichtskonstanz im Vakuum erhält man 0,50 g (69% der Theorie) der Titelverbindung als farblosen Feststoff; Schmp. 137-138°C.

Analog erhält man durch Umsetzung von 5-(2-Chlor-1,1,2-trifluorethoxy)-2-mercaptobenzimidazol mit 2-Chlormethyl-3,4-dimethoxypyridiniumchlorid 5-[(2-Chlor-1,1,2-trifluor)ethoxy]-2-[(3,4-dimethoxy-2-pyridyl)-methylthio]-1H-benzimidazol (Schmp. 99-102°C).

8. <u>5-(1,1,2,2-Tetrafluorethoxy)-2-{[1-(4-methoxy-2-pyridyl)ethyl]sulfinyl}-1H-benzimidazol</u>

Zu einer Lösung von 19,5 g ±-5-(1,1,2,2-Tetrafluorethoxy)-2-{[1-(4-methoxy-2-pyridyl)ethyl]thio}-1H-benzimidazol in 1,5 l Dichlormethan werden bei -65°C 9,8 g 80%ige m-Chlorperoxybenzoesäure in 200 ml Dichlormethan zugetropft. Man rührt noch 2 h bei -40°C und rührt das Reaktionsgemisch anschließend in eine Natriumcarbonatlösung ein. Die organische Phase wird abgetrennt, die wäßrige Phase nochmals mit Dichlormethan extrahiert. Die vereinigten organischen Phasen werden über Natriumsulfat getrocknet und eingeengt. Dabei kristallisiert ein schwerlösliches Diastereomer aus. In der Mutterlauge verbleibt das andere Diastereomer, das entweder chromatographisch gereinigt werden kann oder durch Stehenlassen, bevorzugt in Gegenwart von Basen, in das schwerer lösliche Diastereomer umgelagert werden kann. Umkristallisation des schwer löslichen Produktes aus Essigester/Diisopropylether liefert 10 g (51%) der Titelverbindung vom Schmp. 156-157°C.

Analog erhält man aus
(±)-2,2-Difluor-6-{[1-(4-methoxy-2-pyridyl)ethyl]thio}-5H-[1,3]-dioxolo[4,5-f]benzimidazol bzw. aus
(±)-5-(2,2,2-Trifluorethoxy)-2-{[1-(4-methoxy-2-pyridyl)ethyl]thio}-1H-benzimidazol,
durch Oxidation mit m-Chlorperoxybenzoesäure 2,2-Difluor-6-{[1-(4-methoxy-2-pyridyl)ethyl]sulfinyl}-5H-[1,3]-dioxolo[4,5-f]benzimidazol (Schmp. 170°C) bzw. 5-(2,2,2-Trifluorethoxy)-2-{[1-(4-methoxy-2-pyridyl)-ethyl]sulfinyl}-1H-benzimidazol (Öl).

9. <u>(±)-2,2-Difluor-6-{[1-(4-methoxy-2-pyridyl)ethyl]thio}-5H-[1,3]-dioxolo[4,5-f]benzimidazol</u>

4 g 2,2-Difluor-5H-[1,3]-dioxolo[4,5-f]benzimidazol-6-thiol, 3,6 g (±)-2-(1-Chlorethyl)-4-methoxy-pyridin-hydrochlorid, 1,4 g Natriumhydroxid, 3 ml Wasser und 50 ml Ethanol werden 4 h bei 65°C gerührt. Das Reaktionsgemisch wird zur Trockene eingeengt, in 100 ml 2 N Salzsäure gelöst, Verunreinigungen mit Essigester ausgeschüttelt, die wäßrige Phase wird mit Natronlauge auf pH 10 eingestellt und mit Essigester extrahiert. Die organische Phase wird getrocknet und eingeengt. Umkristallisation aus Toluol liefert 4,3 g (80,5%) der Titelverbindung vom Schmp. 173°-175°C.

Analog erhält man durch Umsetzen des Pyridinhydrochlorids mit 2-Mercapto-5-(1,1,2,2-tetrafluorethoxy)-1H-benzimidazol bzw.
2-Mercapto-5-(2,2,2-trifluorethoxy)-1H-benzimidazol,
(±)-5-(1,1,2,2-Tetrafluorethoxy)-2-{[1-(4-methoxy-2-pyridyl)ethyl]thio}-1H-benzimidazol (Schmp. 78°-81°C) bzw.

(±)-5-(2,2,2-Trifluorethoxy)-2-{[1-(4-methoxy-2-pyridyl)ethyl]thio}-1H-benzimidazol (Öl).

Ausgangsverbindungen

A. Pyridine

Die Pyridine werden in Analogie zu den in der DE-OS 34 04 610, EP-A-0 005 129 und EP-A-0 080 602 beschriebenen Verfahren bzw. wie folgt hergestellt:

A1. 2-Chlormethyl-4,5-dimethoxy-pyridinium-chlorid

a) 2-Chlormethyl-4,5-dimethoxy-pyridinium-chlorid

Zu einer auf 0°C gekühlten Lösung von 5 g 2-Hydroxymethyl-4,5-dimethoxypyridin in 40 ml Methylenchlorid tropft man innerhalb einer Stunde 3 ml Thionylchlorid, gelöst in 10 ml Methylenchlorid zu, rührt anschließend 4 Stunden bei 20°C, wobei sich die Reaktionsmischung rot färbt, setzt 5 ml Toluol zu und engt am Rotationsverdampfer vollständig ein (30°C / 5 mbar). Der ölige Rückstand wird in 50 ml warmem Isopropanol gelöst, mit wenig Tonsil® geklärt, filtriert und erneut eingeengt. Man nimmt in 10 ml Toluol auf und bringt die Lösung mit Petrolether zur Kristallisation. Nach Abkühlung im Eisbad wird abgesaugt, mit Petrolether gewaschen und getrocknet. Man erhält 4,6 g (70 % d Th.) der Titelverbindung 2-Chlormethyl-4,5-dimethoxy-pyridinium-chlorid als farblosen Feststoff; Zers. bei 160-61°C.

b) 2-Hydroxymethyl-4,5-dimethoxy-pyridin

19 g 4,5-Dimethoxy-2-methylpyridin-1-oxid werden innerhalb von 30 Minuten in der Weise zu 60 ml auf 80°C erwärmten Essigsäureanhydrid zudosiert, daß die Temperatur nicht über 100°C steigt. Nach weiteren 45 Minuten bei 85°C wird überschüssiges Essigsäureanhydrid im Vakuum abdestilliert und der ölige dunkle Rückstand, der im wesentlichen aus dem Zwischenprodukt 2-Acetoxymethyl-4,5-dimethoxypyridin besteht, mit 80 ml 2n Natronlauge 1 Stunde lang bei 80°C gerührt. Nach Verdünnen mit 80 ml Wasser und Abkühlung wird achtmal mit je 100 ml Methylenchlorid extrahiert, die vereinigten organischen Phasen zweimal mit in Natronlauge gewaschen, getrocknet, eingeengt und der kristalline, bräunliche Rückstand aus Toluol umkristallisiert. Man erhält 14 g (74 % d.Th.) 2-Hydroxymethyl-4,5-dimethoxy-pyridin vom Schmp. 122-24°C.

c) 4,5-Dimethoxy-2-methylpyridin-1-oxid

Zu einer Suspension von 16,9 g 5-Methoxy-2-methyl-4-nitropyridin-1-oxidin 170 ml trockenem Methanol werden 20 ml einer 30 %-igen Natriummethylatlösung zugetropft, 15 Stunden bei 20°C und anschließend 4 Stunden bei 50°C gerührt. Man stellt durch vorsichtige Zugabe von konzentrierter Schwefelsäure unter Eiskühlung auf pH 7, engt ein, rührt den Rückstand mit 200 ml Methylenchlorid aus, filtriert von unlöslichen Bestandteilen, versetzt mit 10 ml Toluol und engt erneut zur Trockne ein. Man erhält 15,2 g (98 % d.Th.) 4,5-Dimethoxy-2-methylpyridin-1-oxid als farbloses Kristallisat vom Schmp. 118-121°C.

d) 5-Methoxy-2-methyl-4-nitropyridin-1-oxid

Zu 35 ml auf 60°C erwärmte 65 %-ige Salpetersäure werden 21,2 g 5-Methoxy-2-methylpyridin-1-oxid in der Weise zudosiert, daß die Temperatur der Reaktionsmischung 80°C nicht übersteigt. Man rührt 1 Stunde bei 80°C, setzt zur vollständigen Umsetzung noch 13 ml 100 %-ige Salpetersäure zu und rührt weitere 2 Stunden bei 60-70°C. Zur Aufarbeitung gießt man auf 300 g Eis. Der ausgefallene gelbe Niederschlag wird über ein Nutsche filtriert, mit Wasser gewaschen und getrocknet. Der trockene Feststoff wird mit 200ml Methylenchlorid ausgekocht, filtriert und getrocknet. Durch Konzentrierung des Filtrats wird weiteres DC-reines Produkt isoliert. Man erhält 22,3 g (87 % d.Th.) 5-Methoxy-2-methyl-4-nitropyridin-1-oxid vom Schmp. 201-202°C; gelbe Kristalle.

e) 5-Methoxy-2-methylpyridin-1-oxid

Zu einer Lösung von 60,9 g 5-Methoxy-2-methylpyridin in 300 ml Eisessig werden bei 60°C 120 g 30 %-ige Wasserstoffperoxidlösung innerhalb von 1 Stunde zugetropft und 3 Stunden nachgerführt. Nach

Zerstörung von überschüssigen Perverbindungen durch Zugabe von aktivem Mangandioxid wird filtriert, eingeengt, der Rückstand in 500 ml Essigsäureethylester heiß geklärt, erneut eingeengt und bei 0,3 mbar destilliert. Man erhält 54 g (77 % d.Th.) 5-Methoxy-2-methylpyridin-1-oxid als rasch erstarrendes Öl (Sdp. 130°C); Schmp. 80-84°C.

f) 5-Methoxy-2-methylpyridin

Zu einer Lösung von 84 g Kaliumhydroxid in 400 ml Methanol und 500 ml Dimethylsulfoxid werden innerhalb einer Stunde 150 ml 3-Hydroxy-6-methylpyridinzudosiert. Nach Entfernung des Methanols am Rotationsverdampfer tropft man unter Eiskühlung 213 g Methyliodid, gelöst in 100 ml Dimethylsulfoxid zu, rührt 15 Stunden bei 20°C und unterwirft das Reaktionsgemisch einer Wasserdampfdestillation. Das Destillat wird am Extraktor kontinuierlich mit Methylenchlorid extrahiert und der Extrakt eingeengt. Man erhält 85 g (56 % d.Th.) 5-Methoxy-2-methylpyridin als farbloses Öl.

A2. 2-Chlormethyl-4,5-dimethoxy-3-methylpyridinium-chlorid

a) 2-Chlormethyl-4,5-dimethoxy-3-methylpyridinium-chlorid

Nach der in Beispiel A1a) beschriebenen Arbeitsweise erhält man durch Umsetzung von 2,7 g 2-Hydroxymethyl-4,5-dimethoxy-3-methylpyridin mit 4 g Thionylchlorid in 25 ml Methylenchlorid nach 1 Stunde Reaktionszeit und nach einer vereinfachten Aufarbeitungsmethode, nämlich durch Zusatz von 10 ml Toluol, abdestillieren des Methylenchlorids und überschüssigen Thionylchlorids, Absaugung des ausgefallenen Kristallisats und Trocknung 3,45 g (99 % d.Th) der Titelverbindung als farblose Kristalle; Zers. bei 125-26°C.

b) 2-Hydroxymethyl-4,5-dimethoxy-3-methylpyridin

4,5 g 4,5-Dimethoxy-2,3-dimethylpyridin-1-oxid werden in 20 ml Essigsäureanhydrid 30 Minuten auf 110°C erwärmt und anschließend am Rotationsverdampfer eingeengt. Der ölige Rückstand, der aus dem Zwischen produkt 2-Acetoxymethyl-4,5-dimethoxy-3-methylpyridin besteht, wird in 30 ml 3n Natronlauge 2 Stunden bei 80°C gerührt, nach Abkühlung fünfmal mit je 30 ml Methylenchlorid extrahiert, die vereinigten organischen Phasen zweimal mit 2n Natronlauge gewaschen, getrocknet, eingeengt, der Rückstand mit Petrolether verrührt, abgesaugt und getrocknet. Man erhält 4,0 g (89 % d.Th.) 2-Hydroxymethyl-4,5-dimethoxy-3-methylpyridin vom Schmp. 91-92°C.

c) 4,5-Dimethoxy-2,3-dimethylpyridin-1-oxid

6,3 g 4,5-Dimethoxy-2,3-dimethylpyridin werden in 120 ml Methylenchlorid gelöst, sukzessive 20 g m-Chlorperoxibenzoesäure zugegeben, erst 2 Stunden bei 20°C und anschließend 4 Stunden bei 40°C gerührt. Nach Zusatz von 20 ml 5n Natronlauge wird dreimal mit einem Gemisch aus einer 5 %-igen Natriumthiosulfat- und 5 %-igen Natriumcarbonatlösung gewaschen, die Wasserphasen zweimal mit Methylenchlorid rückextrahiert, die vereinigten organischen Phasen über Magnesiumsulfat getrocknet und eingeengt. Man erhält 4,6 g (66 % d.Th.) 4,5-Dimethoxy-2,3-dimethylpyridin-1-oxid. Der Rf-Wert in Methylenchlorid/Methanol 19:1 beträgt 0,25.

d) 4,5-Dimethoxy-2,3-dimethylpyridin

Nach der in Beispiel A1f) beschriebenen Arbeitsweise erhält man durch Umsetzung von 9,18 g 5-Hydroxy-4-methoxy-2,3-dimethylpyridin in 50 ml Dimethylsulfoxid zuerst mit 3,6 g Natriumhydroxid, anschließend mit 8,95 g Methyliodid 7,4 g (74 % d.Th.) 4,5-Dimethoxy-2,3-dimethylpyridin als farbloses, allmählich kristallisierendes Öl, Schmp. 36-38°C.

e) 5-Hydroxy-4-methoxy-2,3-dimethylpyridin

1000 g 4-Methoxy-2,3-dimethylpyridin-1-oxid werden bei 100°C unter Rühren innerhalb von 7 Stunden zu 3 l Essigsäureanhydrid zudosiert und weitere 3 Stunden bei 100°C nachgerührt. Man läßt abkühlen, engt bei 70°C/10 mbar vollständig ein und destilliert anschließend bei $10^{-2}$ mbar. Die Fraktion mit einem Siedeintervall von 95-145°C (Gemisch aus dem Zwischenprodukt 5-Acetoxy-4-methoxy-2,3-dimethylpyridin

und 2-Acetoxymethyl-4-methoxy-3-methylpyridin) wird abgenommen (952 g) und zu 3,5 l auf 50°C erwärmte 2n Natronlauge innerhalb von 30 Minuten zugegeben.
Man rührt bei 50°C bis zur Bildung einer klaren Lösung (ca. 3 Stunden), läßt abkühlen und extrahiert dreimal mit je 1 l Methylenchlorid. Die vereinigten organischen Phasen werden zweimal mit je 0,5 l 1n Natronlauge rückextrahiert und anschließend die vereinigten Wasserphasen mit halbkonzentrierter Salzsäure unter Rühren auf pH 7,5 gestellt. Man filtriert vom ausgefallenen Feststoff, wäscht nach und trocknet bis zur Gewichtskonstanz. Man erhält 5-Hydroxy-4-methoxy-2,3-dimethylpyridin vom Schmp. 274-76°C.

A3. <u>2-Chlormethyl-3,4-dimethoxy-pyridinium-chlorid</u>

a) 2-Chlormethyl-3,4-dimethoxy-pyridiniumchlorid

Nach der in Beispiel A1a beschriebenen Arbeitsweise erhält man durch Umsetzung von 3,38 g 2-Hydroxymethyl-3,4-dimethoxypyridin mit 2 ml Thionylchlorid in 30 ml Methylenchlorid nach 2,5 Stunden Reaktionszeit und nach der in Beispiel A2a beschriebenen Art der Aufarbeitung 4,2 g (93% d.Th.) der Titelverbindung als farblosen Feststoff vom Schmp. 151-152°C (Zers.)

b) 2-Hydroxymethyl-3,4-dimethoxypyridin

4,8 g 2-Acetoxymethyl-3,4-dimethoxypyridin werden nach Zusatz von 15 ml 2n Natronlauge bei 80°C kräftig gerührt, wobei sich aus dem anfänglichen Zweiphasengemisch eine homogene Lösung bildet. Nach 2 h läßt man abkühlen, extrahiert fünfmal mit je 30 ml Methylenchlorid, wäscht die vereinigten organischen Phasen zweimal mit je 5 ml 0,3 n Natronlauge, trocknet über Kaliumcarbonat, filtriert, engt ein und verrührt den Destillationsrückstand mit Petrolether. Man erhält 3,6 g (96% d.Th.) 2-Hydroxymethyl-3,4-dimethoxypyridin als farblosen Feststoff vom Schmp. 87-89°C.

c) 2-Acetoxymethyl-3,4-dimethoxypyridin

Zu 25 ml Essigsäureanhydrid werden bei 85°C innerhalb von einer Stunde 4,8 g (28 mMol) 3,4-Dimethoxy-2-methylpyridin-1-oxid zudosiert, eine Stunde bei der selben Temperatur gerführt, im Vakuum vollständig eingeengt und der braune, ölige Rückstand in einer Kugelrohrdestille bei 1 Pa destilliert. Man erhält 5,3 g (90% d.Th.) 2-Acetoxymethyl-3,4-dimethoxypyridin; Sdp. 125-130°C.

d) 3,4-Dimethoxy-2-methylpyridin-1-oxid

4,5 g (25 mMol) 3-Methoxy-2-methyl-4-nitropyridin-1-oxid werden in 75 ml trockenem Methanol nach Zusatz von 4,7 ml 30%iger Natriummethylatlösung 16 Stunden bei 40°C gerührt. Anschließend kühlt man ab, stellt mit konz. Schwefelsäure auf pH 7, filtriert, engt im Vakuum vollständig ein, nimmt den öligen, rötlichen Rückstand in 50 ml Toluol auf, filtriert erneut von unlöslichen Bestandteilen und engt das Filtrat zur Trockene ein. Der gelbe, ölige Rückstand kristallisiert im Eisbad und wird abschließend mit 30 ml Petrolether (50/70) bei 40°C ausgerührt. Nach Filtration und Trocknung im Exsiccator erhält man 5,2 g (88% d.Th.) 3,4-Dimethoxy-2-methylpyridin-1-oxid in Form blaßgelber Kristalle vom Schmp. 111-113°C.

e) 3-Methoxy-2-methyl-4-nitropyridin-1-oxid

Zu 5,4 g 3-Methoxy-2-methylpyridin-1-oxid in 12 ml Eisessig werden bei 80°C innerhalb von 6h in vier Portionen von je 2 ml 8 ml konz. Salpetersäure zugegeben, über Nacht bei der selben Temperatur gerührt, nochmals in drei Portionen innerhalb von 6 Stunden 8 ml Salpetersäure zugegeben und weitere 15 Stunden gerührt. Nach Abkühlung gießt man auf Eis (40g), stellt mit 10n Natronlauge auf pH 6, filtriert vom ausgefallenen Nebenprodukt (3-Methoxy-2-methyl-4-nitropyridin) und extrahiert viermal mit 50 ml Methylenchlorid. Nach Trocknung werden die vereinigten organischen Phasen vollständig eingeengt und der Rückstand aus wenig Methylenchlorid/Petrolether umkristallisiert. Man erhält 4,2 g (57% d.Th.) der Titelverbindung in Form gelber Kristalle vom Schmp. 103-104°C.

f) 3-Methoxy-2-methylpyridin-1-oxid

15,3 g (0,124 Mol) 3-Methoxy-2-methylpyridin werden in 100 ml Eisessig gelöst und bei 80°C in 4 Portionen 40 ml 30%iges Wasserstoffperoxid innerhalb von 6 Stunden zugegeben. Man rührt weitere drei

Stunden und engt anschließend im Vakuum (1,5 kPa) ein, setzt zweimal je 50 ml Essigsäure zu und engt jeweils vollständig ein. Nach negativem Nachweis auf Perverbindungen wird im Kugelrohrofen destilliert. Die bei 120$^0$C (1,5 Pa) destillierende Fraktion wird in wenig Diethylether ausgerührt, der Feststoff filtriert und getrocknet. Man erhält 12 g (60% d.Th.) 3-Methoxy-2-methylpyridin-1-oxid in Form farbloser Kristalle vom Schmp. 72-78$^0$C.

g) 3-Methoxy-2-methylpyridin

Nach der in Beispiel A1f beschriebenen Arbeitsweise erhält man durch Umsetzung von 13,7 g (125 mMol) 3-Hydroxy-2-methylpyridin mit 9,2 ml Methyliodid unter Zusatz von 46 ml 3m methanolischer Kaliumhydroxidlösung nach einer Reaktionszeit von 3 Stunden 15,5 g (90% d.Th.) 3-Methoxy-2-methylpyridin als farbloses Öl.

A4. <u>2-(1-Chlorethyl)-4-methoxy-pyridin-hydrochlorid</u>

a) 2-(1-Chlorethyl)-4-methoxy-pyridin-hydrochlorid

Zu einer gekühlten Suspension von 57 g (±)-1-[4-Methoxy-2-pyridyl]-ethanol in 400 ml Dichlormethan werden 36 ml Thionylchlorid so zugetropft, daß die Innentemperatur 15$^0$C nicht übersteigt. Anschließend erhitzt man 2,5 h unter Rückfluß, versetzt mit 300 ml Toluol und engt auf 150 ml ein. Das ausgefallene Reaktionsprodukt wird abgesaugt und mit kaltem Toluol und Petrolether nachgewaschen. Man erhält 76,4 g (98,2%) der Titelverbindung vom Schmp. 113$^0$-116$^0$C.

b) (±)-1-(4-Methoxy-2-pyridyl)-ethanol

114 g (±)-Essigsäure-1-[4-methoxy-2-pyridyl]-ethylester werden bei 70$^0$C zu 220 ml 4 N Natronlauge zugetropft. Anschließend wird noch 2 h bei 75$^0$C gerührt, abgekühlt und viermal mit je 200 ml Dichlormethan extrahiert. Die organische Phase wird mit 2 N Natronlauge und Wasser gewaschen, über Natriumsulfat getrocknet und zur Trockene eingeengt. Nach Ausrühren mit 250 ml Diisopropylether erhält man 64 g (72%) der Titelverbindung vom Schmp. 92$^0$C.

c) (±)-Essigsäure-1-[4-methoxy-2-pyridyl]-ethylester

171 g 2-Ethyl-4-methoxy-pyridin-N-oxid werden im Verlauf von 3 h zu 312 ml auf 90$^0$C erwärmtem Acetanhydrid so zugetropft, daß die Innentemperatur 115$^0$C nicht übersteigt. Anschließend läßt man noch 2,5 h bei 100$^0$C stehen und zieht überschüssiges Acetanhydrid ab. Destillation des Rückstandes liefert 171 g (78%) der Titelverbindung vom Kp. 100$^0$C/1 Pa.

d) 2-Ethyl-4-methoxypyridin-N-oxid

Zu einer Suspension von 192 g 4-Nitro-2-ethylpyridin-N-oxid in 1,1 l absolutem Methanol werden im Verlauf von 2,5 h 236 ml 30%ige Natriummethylatlösung (in Methanol) so zugetropft, daß die Innentemperatur 30$^0$C nicht überschreitet. Anschließend wird 12 h weitergerührt, mit etwa 10 ml konzentrierter Schwefelsäure auf pH 6,5-7 eingestellt, filtriert und eingeengt. Der Rückstand wird dreimal mit heißem Toluol ausgewaschen, der Extrakt wird mit wenig Tonsil geklärt. Durch Einengen erhält man als Rückstand 172 g der Titelverbindung als Öl.

<u>B. Benzimidazole</u>

B1. <u>5-(1,1,2,2-Tetrafluorethoxy)-1H-benzimidazol-2-thiol</u>

a) 55 g 1-Nitro-4-(1,1,2,2-tetrafluorethoxy)benzol werden in 300 ml Ethanol an 0,5 g 10%iger Palladiumkohle in einer Umlaufhydrierungsapparatur unter Atmosphärendruck 1 h bei 20-45°C hydriert, der Katalysator abfiltriert und die Lösung bei 40$^0$C im Vakuum eingeengt. Man verdünnt das 4-(1,1,2,2-Tetrafluorethoxy)anilin mit 100 ml Eisessig und tropft 23 ml Essigsäureanhydrid bei Raumtemperatur zu, versetzt nach 30 Min. mit 2 ml Wasser, engt nach kurzer Zeit die Lösung bei 50$^0$C im Vakuum ein und versetzt mit 500 ml Eiswasser. Man erhält 56 g (97%) N-[4-(1,1,2,2-tetrafluorethoxy)phenyl]-acetamid vom Schmp. 121-122$^0$C.

b) Man löst 55 g der voranstehenden Verbindung in 380 ml Dichlormethan, tropft 55 ml 100%ige Salpetersäure in 10 Min. bei Raumtemperatur zu und rührt noch 6 h. Die organische Lösung wird dann mit wässriger Natriumcarbonatlösung und Wasser gewaschen, mit Magnesiumsulfat getrocknet und eingeengt. Man erhält 65 g (100%) N-[2-Nitro-4-(1,1,2,2-tetrafluorethoxy)phenyl]-acetamid vom Schmp. 80-81°C (aus Cyclohexan).
c) Man löst 63 g der voranstehenden Verbindung in 450 ml Methanol, tropft bei Raumtemperatur 106 ml 6 m Natronlauge zu, kühlt im Eisbad und fällt durch Zutropfen von 900 ml Wasser 53 g (98%) 2-Nitro-4-(1,1,2,2-tetrafluorethoxy)-anilin (Schmp. 85-86°C).
d) 33 g der voranstehenden Verbindung werden in ca. 600 ml Isopropanol an 1 g10%iger Palladiumkohle in einer Umlaufhydrierungsapparatur drucklos bei Raumtemperatur hydriert. Man saugt den Katalysator ab und fällt mit 4 m Chlorwasserstoff in Ether 34 g (89%) 4-(1,1,2,2-Tetrafluorethoxy)-1,2-phenylendiamin -dihydrochlorid vom Schmp. 275-276°C (Zersetzung).
e) 33 g der voranstehenden Verbindung werden mit 330 ml Ethanol, 60 ml Wasser, 8,9 g Natriumhydroxid und 23 g Kalium-O-ethyldithiocarbonat (umkristallisiert aus Isopropanol) versetzt und 15 h unter Rückfluß zum Sieden erhitzt. Man versetzt mit 1,2 l Eiswasser, stellt mit Natronlauge auf pH 13-14, klärt mit Aktivkohle und fällt mit verdünnter Salzsäure bis pH 3,5. Man erhält 27 g (91%) der Titelverbindung vom Schmp. 316-319°C (aus Isopropanol).

B2. 5-Trifluormethoxy-1H-benzimidazol-2-thiol

Analog Beispiel B1e erhält man durch Umsetzen von 4-Trifluormethoxy-1,2-phenylendiamin-dihydrochlorid (vgl. C.A. 55, 23408d, 1961) mit Kalium-0-ethyldithiocarbonat und Natronlauge in Ethanol in 75% Ausbeute die Titelverbindung vom Schmp. 305-307°C (Zersetzung, aus Toluol).

B3. 5-(2,2,2-Trifluorethoxy)-1H-benzimidazol-2-thiol

a) 50 g 1-(2,2,2-Trifluorethoxy)-4-nitrobenzol (Synthesis 1980, Seite 727) werden analog Beispiel B1a hydriert und acetyliert. Man erhält 50 g (95%) N-[4-(2,2,2-Trifluorethoxy)phenyl]-acetamid (Schmp. 140-141°C).
b) Man rührt 42 g der voranstehenden Verbindung mit 9,7 ml 100% Salpetersäure in 290 ml Eisessig 18 h bei Raumtemperatur und fällt mit Wasser. Man erhält 47 g (94%) N-[2-Nitro-4-(2,2,2-trifluorethoxy)-phenyl]-acetamid (Schmp. 117-118°C).
c) Man hydrolysiert 47 g der voranstehenden Verbindung analog Beispiel B1c und erhält 38,7 g (97%) 2-Nitro-4-(2,2,2-trifluorethoxy)-anilin (Schmp. 84-85°C).
d) Man hydriert 37 g der voranstehenden Verbindung analog Beispiel B1d und erhält 41 g (94%) 4-(2,2,2-Trifluorethoxy)-1,2-phenylendiamindihydrochlorid vom Schmp. 230-233°C (Zersetzung).
e) Analog Beispiel B1e erhält man aus 36 g der voranstehenden Verbindung 30 g (94%) der Titelverbindung (Schmp. 288-290°C).

B4. 5-Chlordifluormethoxy-1H-benzimidazol-2-thiol

a) 10,0 g N-[4-(Chlordifluormethoxy)phenyl]-acetamid (Schmp. 101-103°C, aus 4-Chlordifluormethoxyanilin und Essigsäureanhydrid) und 12,3 ml 100 Salpetersäure werden in 80 ml Dichlormethan 4 h bei 20°C gerührt. Man neutralisiert mit wäßriger Kaliumhydrogencarbonatlösung, engt die organische Schicht ein und erhält 11,4 g (96%) N-(4-Chlordifluormethoxy-2-nitrophenyl)-acetamid (Schmp. 89-91°C).
b) Man tropft bei 5°C zu 10,5 g der voranstehenden Verbindung in 200 ml Methanol 8,6 ml einer 30%igen Lösung von Natriummethylat in Methanol, rührt 2 h ohne Kühlung, versetzt mit Eiswasser, stellt auf pH 8 und erh 8,7 g (97%) 4-Chlordifluormethoxy-2-nitroanilin (Schmp. 40-42°C).
c) Man hydriert 8,5 g der voranstehenden Verbindung an 0,8 g 10%iger Palladiumkohle drucklos in 200 ml Methanol, versetzt mit konzentrierter Salzsäure, filtriert, engt ein und verrührt mit Diisopropylether. Man erhält 8,5 g (97%) 4-Chlordifluormethoxy-1,2-phenylendiamin-dihydrochlorid.
d) Aus 8,5 g der voranstehenden Verbindung werden analog Beispiel B1e 6,3 g (72%) der Titelverbindung vom Schmp. 268-270°C (Zersetzung) erhalten.

B5. 5-Difluormethoxy-1H-benzimidazol-2-thiol

a) 11,8 g N-(4-Difluormethoxyphenyl)-acetamid [L.M. Jagupol'skii et al., J.General Chemistry (USSR) 39, 190 (1969)] werden in 200 ml Dichlormethan mit 12,1 ml 100%iger Salpetersäure 1,5 h bei Raumtemperatur gerührt. Analog Beispiel B1b erhält man 13,3 g (92%) N-[(4-Difluormethoxy-2-nitro)phenyl]-acetamid (Schmp. 71-73°C).

b) Analog Beispiel B4b erhält man daraus in 96%iger Ausbeute 4-Difluormethoxy-2-nitroanilin (Schmp. 68-70°C).

c) Analog Beispiel B4c erhält man in 94% Ausbeute 4-Difluormethoxy-1,2-phenylendiamin-dihydrochlorid.

d) Analog Beispiel B1e erhält man in 78% Ausbeute die Titelverbindung vom Schmp. 250-252°C (aus 2-Propanol).

B6. 5,6-Bis(difluormethoxy)-1H-benzimidazol-2-thiol

a) In eine Lösung von 100 g Brenzkatechin, 220 g Natriumhydroxid und 60 g Natriumdithionit in 500 ml Wasser und 400 ml Dioxan leitet man bei 50-55°C 275 g Chlordifluormethan analog L.N. Sedova et al., Zh. Org. Khim. 6, 568 (1970) ein. Man erhält nach Destillation bei 61-62°C/1,0-1,1kPa eine Mischung von 1,2-Bis(difluormethoxy)benzol und 2-Difluormethoxyphenol, die durch Chromatographie an Kieselgel mittels Cyclohexan/Essigsäureethylester (4:1) getrennt werden.

b) Eine Lösung von 15 g 1,2-Bis(difluormethoxy)benzol und 15 ml 100%ige Salpetersäure in 150 ml Dichlormethan wird 7 h bei Raumtemperatur gerührt. Man neutralisiert mit Kaliumhydrogencarbonatlösung, trennt die organische Schicht ab und chromatographiert an Kieselgel mittels Cyclohexan/Essigsäureethylester (4:1). Man erhält 1,2-Bis(difluormethoxy)-4-nitrobenzol. Dieses hydriert und acetyliert man analog Beispiel B1a zu N-[3,4-Bis(difluormethoxy)phenyl]acetamid (Schmp. 81-83°C). Analog Beispiel B1 erhält man ferner N-[4,5-Bis(difluormethoxy)-2-nitrophenyl]acetamid (Schmp. 65-67°C), N-[4,5-Bis(difluormethoxy)-2-nitro]anilin (Schmp. 107-109°C), 4,5-Bis(difluormethoxy)-1,2-phenylendiamin-dihydrochlorid und die Titelverbindung vom Schmp. 285-287°C (Zersetzung; aus 2-Propanol).

B7. 5-Difluormethoxy-6-methoxy-1H-benzimidazol-2-thiol

a) In eine Lösung von 55,5 g Guajacol und 130 g Natriumhydroxid in 300 ml Wasser und 300 ml Dioxan werden bei 60°C ca. 58 g Chlordifluormethan eingeleitet. Man filtriert die Mischung bei 10°C, trennt die organische Schicht ab, trocknet mit wasserfreiem Kaliumcarbonat und destilliert. Man erhält 56 g (73%) 1-Difluormethoxy-2-methoxybenzol vom Siedepunkt 75-76°C/0,9kPa.

b) Zu einer Lösung von 47 g der voranstehenden Verbindung in 230 ml Dichlormethan wird bei 0-5°C eine Lösung von 33,8 ml 100%iger Salpetersäure in 90 ml Dichlormethan getropft, nach 30 Min. mit 250 ml Eiswasser versetzt und mit Kaliumhydrogencarbonat neutralisiert. Die getrocknete organische Phase wird im Vakuum eingeengt und der Rückstand aus Cyclohexan umkristallisiert. Man erhält 53 g (90%) 1-Difluormethoxy-2-methoxy-5-nitrobenzol (Schmp. 48-49°C). Dieses wird analog Beispiel B1a hydriert und acetyliert. Man erhält in 90% Ausbeute N-(3-Difluormethoxy-4-methoxyphenyl)acetamid (Schmp. 129-130°C).

c) 46 g der voranstehenden Verbindung werden mit 33 ml 100%iger Salpetersäure in Dichlormethan analog der voranstehenden Vorschrift nitriert. Man erhält in 99% Ausbeute N-(5-Difluormethoxy-4-methoxy-2-nitrophenyl)acetamid (Schmp. 116-117°C).

d) 54 g der voranstehenden Verbindung werden in 810 ml Methanol 1 h mit 44,8 ml 30%iger methanolischer Natriummethylatlösung bei Raumtemperatur gerührt. Man engt im Vakuum ein, versetzt mit Eiswasser und Eisessig bis pH 8 und erhält in 99% Ausbeute 5-Difluormethoxy-4-methoxy-2-nitroanilin (Schmp. 144-145°C).

e) 25 g der voranstehenden Verbindung werden in 300 ml Methanol an 1,25 g 10%iger Palladiumkohle entsprechend Beispiel B1d hydriert. Man erhält 26 g (88%) 3-Difluormethoxy-4-methoxy-1,2-phenylendiamindihydrochlorid vom Schmp. 218-220°C (Zersetzung).

f) 25 g der voranstehenden Verbindung werden mit 19 g Kalium-0-ethyldithiocarbonat entsprechend Beispiel B1e umgesetzt. Man erhält 20 g (89%) der Titelverbindung vom Schmp. 280-282°C (Zersetzung; aus 2-Propanol).

B8. 5-Difluormethoxy-6-fluor-1H-benzimidazol-2-thiol

a) Analog Beispiel B7a erhält man aus 2-Fluorphenol und Chlordifluormethan 1-Difluormethoxy-2-fluorbenzol (Kp. 76°C/10 kPa; $n_D^{20}$ = 1,4340)

b) Zu 30 g der voranstehenden Verbindung in 300 ml Dichlormethan tropft man bei -10°C 38,4 ml

100%ige Salpetersäure, rührt 1 h bei -10°C und 2, bei 0°C. Man versetzt mit Eiswasser, stellt neutral und chromatographie über Kieselgel mit Essigester/Cyclohexan (4:1). Man erhält 34 g eines Öles, das ca. 90% 1-Difluormethoxy-2-fluor-4-nitrobenzol und 10% 1-Difluormethoxy-2-fluor-5-nitrobenzol (NMR-Spektrum) enthält.

c) 30 g der voranstehenden Mischung wird analog Beispiel B1a hydriert und acetyliert. Nach Umkristallisieren aus Toluol erhält man 21 g (65%) N-(4-Difluormethoxy-3-fluorphenyl)acetamid vom Schmp. 112-113°C.

d) Zu 20 g der voranstehenden Verbindung in 200 ml Dichlormethan werden bei 20°C 22,5 ml 100%ige Salpetersäure in 30 Min. zugetropft und 15 h bei Raumtemperatur nachgerührt. Analog Beispiel B7c erhält man in 89% Ausbeute N-(4-Difluormethoxy-5-fluor-2-nitrophenyl)acetamid vom Schmp. 72-74°C (aus Cyclohexan). Durch mehrstündiges Rühren mit 1 m Salzsäure in Methanol bei 60°C erhält man in 95% Ausbeute 4-Difluormethoxy-5-fluor-2-nitroanilin vom Schmp. 95-97,5°C und analog Beispiel B7e in 85% Ausbeute 4-Difluormethoxy-5-fluor-1,2-phenylendiamin-dihydrochlorid. Zersetzung ab 210°C.

e) 15 g der voranstehenden Verbindung werden mit 11,8 g Kalium-0-ethyldithiocarbonat entsprechend Beispiel B1e umgesetzt. Man erhält 11,1 g (84%) der Titelverbindung vom Schmp. 275-276°C (Zersetzung aus 2-Propanol).

B9. <u>2,2-Difluor-5H-[1,3]-dioxolo[4,5-f]benzimidazol-6-thiol</u>

a) Man hydriert 30 g 4-Amino-2,2-difluor-5-nitro-1,3-benzodioxol in 300 ml Methanol an 0,5 g 10%iger Palladiumkohle in einer Umlaufhydrierungsapparatur bei Atmosphärendruck und Raumtemperatur, versetzt mit 2,5 Äquivalenten methanolischer Chlorwasserstofflösung, filtriert, engt die Lösung im Vakuum ein, versetzt mit 2-Propanol und Ether und erhält 35 g (97 %) 2,2-Difluor-1,3-benzodioxol-4,5-diamin-dihyrochlorid vom Schmp. 232-233°C (Zersetzung).

b) Man versetzt 30 g der voranstehenden Verbindung in 300 ml Ethanol mit 24 g Kalium-0-ethyldithiocarbonat (umkristallisiert aus 2-Propanol) und 9,2 g Natriumhydroxid in 55 ml Wasser und erhitzt 15 h unter Rückfluß zum Sieden. Man gießt auf 1,5 l Wasser, stellt mit Natronlauge auf pH 14, klärt mit Aktivkohle, fällt mit konzentrierter Salzsäure in der Wärme und saugt den Niederschlag in der Kälte ab. Man erhält 24 g (91%) der Titelverbindung vom Schmp. 365-370°C (Zersetzung).

B10. <u>6,6,7-Trifluor-6,7-dihydro-1H-[1,4]-dioxino[2,3-f]benzimidazol-2-thiol</u>

a) Zu 50 g 2,2,3-Trifluor-2,3-dihydro-1,4-benzodioxin wird bei 5°C in 1 h eine Mischung von 39,5 ml 69%iger Salpetersäure und 46 ml 97%iger Schwefelsäure getropft. Man rührt 1 h bei 10°C, 1 h bei 20°C und 5 Min. bei 40°C und gießt auf 200 g Eis, extrahiert mit Dichlormethan, wäscht mit Wasser, trocknet mit Magnesiumsulfat und destilliert im Vakuum. Man erhält 58 g (94 %) einer Mischung von 2,2,3-Trifluor-2,3-dihydro-6-nitro-(und 7-nitro)-1,4-benzodioxin vom Kp. 68,5°C (0,15 mbar) und $n_D^{20}$ 1,5080. Ein Gaschromatogramm mit einer 10 m Fused Silica Säule (Fa. Chrompack) zeigt zwei Peaks im Verhältnis 2:3.

b) Man hydriert 35 g des Isomerengemisches in 400 ml Ethanol an 3 g 10%iger Palladiumkohle bei Atmosphärendruck und 20-30°C in einer Umlaufhydrierungsapparatur, filtriert und engt im Vakuum ein. Man erhält 30,5 g (100 %) einer flüssigen Mischung von 6-Amino-(und 7-Amino)-2,2,3-trifluor-2,3-dihydro-1,4-benzodioxin.

c) Zu 28 g der voranstehenden Isomerenmischung tropft man bei 20-30°C eine Mischung aus 15,3 g Essisäureanhydrid und 15 ml Eisessig, rührt 30 Min. bei 30°C, setzt 1 ml Wasser zu, rührt 30 Min. bei 30°C und destilliert das Lösungsmittel im Vakuum ab. Durch Umkristallisation aus Toluol erhält man 19 g einer Fraktion des Gemisches der isomeren Acetaminoderivate vom Schmp. 128-133°C.

d) Zu 17 g des Isomerengemisches der Acetaminoderivate, suspendiert in 200 ml Dichlormethan, tropft man bei -6° bis -8°C 14 ml 100%ige Salpetersäure, gelöst in 60 ml Dichlormethan, rührt 2 h bei 0°C und dann über Nacht bei Raumtemperatur. Man gießt auf 110 g Eis, trennt die organische Phase ab, wäscht mit Wasser und engt im Vakuum ein. Der Rückstand (19,8 g) wird aus 20 ml Ethanol umkristallisiert. Man erhält 15,9 g einer Mischung von 6-Acetamino-2,2,3-trifluor-2,3-dihydro-7-nitro-1,4-benzodioxin und 7-Acetamino-2,2,3-trifluor-2,3-dihydro-6-nitro-1,4-benzodioxin.

e) Man suspendiert 14,5 g des voranstehenden Produktgemisches in 80 ml Methanol und tropft unter Erwärmung auf 30°C 30 ml 5m Natronlauge zu. Man rührt noch 0,5 h bei Raumtemperatur, gießt auf 200 g Eis und erhält 11,7 g einer Mischung von 6-Amino-2,2,3-trifluor-2,3-dihydro-7-nitro-1,4-benzodioxin und 7-Amino-2,2,3-trifluor-2,3-dihydro-6-nitro-1,4-benzdioxin. Eine Probe wird an einer Kieselgelsäule mit Cyclohexan/Essigsäu ethylester (4:1) in zwei reine Isomeren mit den Schmelzpunkten 110,5-111,5°C und

120-121°C getrennt, deren NMR-Spektren an einem 60 MHz-Gerä in Deuterochloroform praktisch identisch sind.

f) 10,9 g des voranstehenden Isomerengemisches werden in 300 ml Methanol bei Raumtemperatur und Atmosphärendruck an 1 g 10%iger Palladiumkohle in 2,5 h hydriert. Man setzt 30 ml 4 m Chlorwasserstoff in Methanol zu, filtriert, engt im Vakuum ein und verrührt mit 100 ml Ether. Man erhält 12,6 g (98 %) 2,2,3-Trifluor-2,3-dihydro-1,4-benzodioxin-6,7-diamin-dihydrochlorid (Schmp. >250°C).

g) 12 g der voranstehenden Verbindung und 8,5 g Kalium-0-ethyldithiocarbonat (umkristallisiert aus Isopropanol) werden in 120 ml Ethanol mit 20,5 ml 4 m wäßriger Kaliumhydroxidlösung versetzt und 17 h unter Rückfluß zum Sieden erhitzt. Man gießt auf 300 g Eis, stellt mit Kaliumhydroxidlösung auf pH 12-13, klärt mit Aktivkohle und fällt mit konzentrierter Salzsäure. Nach erneuter Fällung mit Säure aus alkalischer wäßrig-alkoholischer Lösung erhält man 10 g (93 %) der Titelverbindung vom Schmp. 309-310°C (Zersetzung).

B11. <u>6-Chlor-6,7,7-trifluor-6,7-dihydro-1H-[1,4]-dioxino[2,3-f]benzimidazol-2-thiol</u>

a) Zu 18 g 2-Chlor-2,3,3-trifluor-2,3-dihydro-1,4-benzodioxin tropft man bei 5°C eine Mischung von 18,3 ml 65%iger Salpetersäure und 15,4 ml 97%ige Schwefelsäure, rührt 2 h bei 5-10°C und gießt auf Eis. Man extrahiert mit Methylenchlorid und erhält 21,3 g einer Mischung von 2-Chlor-2,3,3-trifluor-2,3-dihydro-6-nitro-(und 7-nitro)-1,4-benzodioxin als Öl.

b) Analog Beispiel B10b erhält man daraus in 95% Ausbeute eine ölige Mischung von 2-Chlor-2,3,3-trifluor-2,3-dihydro-1,4-benzodioxin-6-(und 7-)amin, welche entsprechend Beispiel B10c zu einer Mischung der entsprechenden Acetaminoderivate quantitativ umgesetzt wird.

c) 19 g der voranstehenden Mischung wird in 190 ml Dichlormethan mit 16 ml 100%iger Salpetersäure gerührt und das Reaktionsprodukt durch Chromatographie an Kieselgel mittels Cyclohexan/Essigsäureethylester (4:1) gereinigt. Man erhält 15 g einer Mischung von 6-Acetamino-2-chlor-2,3,3 trifluor-6,7-dihydro-7-nitro-1,4-benzodioxin und 7-Acetamino-2-chlor-2,3,3-trifluor-6,7-dihydro-6-nitro-1,4-benzodioxin als hellgelbes Öl.

d) Zu 14,5 g der voranstehenden Mischung in 100 ml Methanol tropft man bei 5°C 10,2 ml einer 30%igen Lösung von Natriummethylat in Methanol, rührt 1,5 h ohne Kühlung, gießt auf Eis, neutralisiert mit verdünnter Salzsäure, extrahiert mit Dichlormethan und engt im Vakuum ein. Man erhält 12,7 g einer Mischung von 6-Amino-2-chlor-2,3,3-trifluor-2,3-dihydro-7-nitro-1,4-benzodioxin und 7-Amino-2-chlor-2,3,3-trifluor-2,3-di hydro-6-nitro-1,4-benzodioxin als orangefarbenes Öl.

e) 12,4 g der voranstehenden Mischung werden analog Beispiel B10f hydriert. Man erhält 12,6 g (99%) 2-Chlor-2,3,3-trifluor-2,3-dihydro-1,4-benzodioxin-6,7-diamin-dihydrochlorid.

f) 12,4 g der voranstehenden Verbindung werden analog Beispiel B10g mit 9,1 g Kalium-O-ethyldithiocarbonat und Kaliumhydroxidlösung in Ethanol umgesetzt. Man erhält 9,6 g (74%) der Titelverbindung vom Schmp. 288-290°C (Zersetzung).

B12. <u>5-Difluormethoxy-4,6-dimethyl-1H-benzimidazol-2-thiol</u>

a) In eine Lösung von 99,7 g 2,6-Dimethylphenol und 246 g Natriumhydroxid in 500 ml Wasser und 500 ml Dioxan leitet man bei 60-70°C 7 h lang Chlordifluormethan ein. Man filtriert, schüttelt mit Ether aus und fraktioniert die organische Phase. Man erhält 98 g (70%) 1-Difluormethoxy-2,6-dimethylbenzol (Kp. 68°C/15 mbar; ($n_D^{20}$ = 1.4631).

b) Zu 93,9 g der voranstehenden Verbindung in 500 ml Dichlormethan tropft man bei 5-10°C 113 ml 100%ige Salpetersäure und rührt bei Raumtemperatur über Nacht. Man wäscht die organische Lösung mit Wasser und Natriumbicarbonatlösung und engt ein. Man erhält 118 g (100%) 1-Difluormethoxy-2,6-dimethyl-3-nitrobenzol ($n_D^{20}$ = 1.5075).

c) 116 g der voranstehenden Verbindung werden analog Beispiel B1a hydriert und acetyliert. Man erhält 99 g (81%) (3-Difluormethoxy-2,4-dimethylphenyl)acetamid (Schmp. 136-137°C).

d) 20 g der voranstehenden Verbindung werden mit 14,5 ml 100%iger Salpetersäure analog Beispiel B7c in Dichlormethan nitriert. Man erhält 21g (89%) N-(3-Difluormethoxy-2,4-dimethyl-6-nitrophenyl)acetamid (Schmp. 184-185°C).

e) 17,5 g der voranstehenden Verbindung werden in 260 ml ca. 8 m Lösung von Chlorwasserstoff in Methanol 12 h bei 60°C gerührt, 60 ml konzentrierte Salzsäure zugegeben und 18 h bei 70°C gerührt. Man stellt mit Natronlauge auf pH 8 und erhält 14,6 (98%) 3-Difluormethoxy-2,4-dimethyl-6-nitroanilin (Schmp. 95-96°C).

f) 14 g der voranstehenden Verbindung werden analog Beispiel B7e hydriert. Man erhält 15,6 g (94%) 4-Difluormethoxy-3,5-dimethyl-1,2-phenylendiamin-dihydrochlorid vom Schmp. 252°C (Zersetzung).
g) 15,6 g der voransteheden Verbindung werden analog Beispiel B1e umgesetzt. Man erhält 9,8 g (71%) der Titelverbindung (Schmp. > 250°C).

B13. 4-Difluormethoxy-5,6-dimethoxy-1H-benzimidazol-2-thiol

a) 50 g 2,3-Dimethoxyphenol werden analog Beispiel B12a mit 80 g Kaliumhydroxid und Chlordifluormethan in Dioxan/Wasser umgesetzt. Man schüttelt mit Diisopropylether aus und wäscht mit Natronlauge und erhält 35 g eines Produkts (Kp. 70-74°C/0,3 mbar), welches analog Beispiel B7b mit 100%iger Salpetersäure umgesetzt wird. Das Umsetzungsprodukt wird über eine Kieselgelsäule mit Cyclohexan/Ethylacetat (3:2) chromatographiert, wonach man in 48% Ausbeute 1-Difluormethoxy-2,3-dimethoxy-5-nitrobenzol (Schmp. 72-73°C) isoliert.
b) 17 g der voranstehenden Verbindung werden analog Beispiel B1a hydriert und acetyliert. Man erhält 15,8 g (88%) (3-Difluormethoxy-4,5-dimethoxyphenyl)acetamid (Schmp. 104-105°C).
c) 10 g der voranstehenden Verbindung werden in 120 ml Dichlormethan mit 5,1 ml 100%iger Salpetersäure 1h bei 0°C gerührt. Man erhält 10,1 g (86%) N-(3-Difluormethoxy-4,5-dimethoxy-2-nitrophenyl)acetamid (Schmp. 144-145°C).
d) 8,3 g der voranstehenden Verbindung werden mit 47 ml konzentrierter Salzsäure und 120 ml Methanol 1,5 h bei 70°C gerührt. Man neutralisiert, extrahiert mit Dichlormethan und läßt aus Toluol kristallisieren. Man erhält 5,7 g (80%) 3-Difluormethoxy-4,5-dimethoxy-2-nitroanilin (Schmp. 141-142°C).
e) Analog Beispiel B7e erhält man aus der voranstehenden Verbindung in 92% Ausbeute 3-Difluormethoxy-4,5-dimethoxy-1,2-phenylendiamin-dihydrochlorid vom Schmp. 233-234°C (Zersetzung).
f) Analog Beispiel B1e erhält man aus der voranstehenden Verbindung in 84% Ausbeute die Titelverbindung (Schmp. 223-224°C).

B14. 5-Difluormethoxy-6-methyl-1H-benzimidazol-2-thiol

a) Analog Beispiel B12. erhält man aus 2-Methylphenol und Chlordifluormethan in 51% Ausbeute 1-Difluormethoxy-2-methylbenzol (Kp. 45-47°C/-11-12 mbar) und daraus mit 100%iger Salpetersäure ein Gemisch von 1-Difluormethoxy-2-methyl-4-nitrobenzol und 1-Difluormethoxy-2-methyl-5-nitrobenzol (Kp. 70-74°C/1-1,5 mbar; 80% Ausbeute), welches reduziert, acetyliert und nitriert wird. Das Rohprodukt wird aus Ethanol umkristallisiert. Man erhält in 42% N-(4-Difluormethoxy-5-methyl-2-nitrophenyl)acetamid (Schmp. 96-97°C).
b) 110,4 g der voranstehenden Verbindung werden in 1,6 l Methanol bei 10-25°C mit 84 g 30% Natriummethanolat in Methanol versetzt und nach 1 h die Mischung auf Eis gegossen. Man erhält 82,5 g (89%) 4-Difluormethoxy-5-methyl-2-nitroanilin (Schmp. 73-74°C).
c) 82 g der voranstehenden Verbindung werden in 400 ml Ethanol an Palladiumkohle hydriert und die filtrierte Lösung von 4-Difluormethoxy-5-methyl-1,2-phenylendiamin mit 82,4 g Kalium-0-ethyldithiocarbonat und 70 ml Wasser 15 h unter Rückfluß erhitzt und analog B1e aufgearbeitet. Man erhält 71 g (82%) der Titelverbindung vom Schmp. 327-329°C (aus Ethanol).

B15. 5-[2-Chlor-1,1,2-trifluorethoxy)-1H-benzimidazol-2-thiol

120 g N-[4-(2-Chlor-1,1,2-trifluorethoxy)phenyl]acetamid werden analog B1 zu N-[4-(2-Chlor-1,1,2-trifluorethoxy)-2-nitrophenyl]acetamid (96%, langsam kristallisierendes Öl) nitriert und analog B14b zu 4-(2-Chlor-1,1,2-trifluorethoxy-2-nitroanilin (96%, Schmp. 65-66°C) entacetyliert. Analog B14c erhält man über 4-(2-Chlor-1,1,2-trifluorethoxy)-1,2-phenylendiamin in 90% die Titelverbindung vom Schmp. 297-298°C (aus Toluol).

Gewerbliche Anwendbarkeit

Die erfindungsgemäßen Verbindungen der Formel I und ihre Salze besitzen wertvolle pharmakologische Eigenschaften, die sie gewerblich verwertbar machen. Sie hemmen deutlich die Magensäuresekretion von Warmblütern und weisen darüberhinaus eine ausgezeichnete Magen- und Darmschutzwirkung bei Warmblütern auf. Diese Magen- und Darmschutzwirkung wird teilweise bereits bei der Verabreichung von Dosen beobachtet, die unterhalb der säuresekretionshemmenden Dosen liegen. Darüberhinaus zeichnen sich die erfindungsgemäßen Verbindungen durch das Fehlen wesentlicher Nebenwirkungen und eine große therapeutische Breite aus. Ein weiterer erfindungswesentlicher Aspekt besteht darin, daß die Verbindungen der

Formel I im jeweils erwünschten pH-Bereich eine hohe chemische Stabilität und ein signifikantes Wirkungsmaximum aufweisen.

Unter "Magen- und Darmschutz" wird in diesem Zusammenhang die Verhütung und Behandlung gastrointestinaler Krankheiten, insbesondere gastrointestinaler entzündlicher Krankheiten und Läsionen (wie z.B. Ulcus ventriculi, Ulcus duodeni, Gastritits, hyperazider oder medikamentös bedingter Reizmagen) verstanden, die beispielsweise durch Mikroorganismen, Bakterientoxine, Medikamente (z.B. bestimmte Antiphlogistika und Antirheumatika), Chemikalien (z.B. Ethanol), Magensäure oder Streßsituationen verursacht werden können.

In ihren ausgezeichneten Eigenschaften erweisen sich die erfindungsgemäßen Verbindungen überraschenderweise den aus dem Stand der Technik bekannten Verbindungen deutlich überlegen. Aufgrund dieser Eigenschaften sind die erfindungsgemäßen Verbindungen und ihre pharmakologisch verträglichen Salze für den Einsatz in der Human- und Veterinärmedizin hervorragend geeignet, wobei sie insbesondere zur Behandlung und Prophylaxe von Krankheiten des Magens und Darms und solcher Krankheiten, die auf einer überhöhten Magensäuresekretion beruhen, verwendet werden. Die hohe Lagerstabilität der erfindungsgemäßen Verbindungen ermöglicht dabei ihren problemlosen Einsatz in pharmazeutischen Zubereitungen.

Ein weiterer Gegenstand der Erfindung sind die erfindungsgemäßen Verbindungen zur Anwendung bei der Behandlung und Prophylaxe der vorstehend genannten Krankheiten.

Ebenso umfaßt die Erfindung die Verwendung der erfindungsgemäßen Verbindungen bei der Herstellung von Arzneimitteln, die zur Behandlung und Prophylaxe der vorstehend genannten Krankheiten eingesetzt werden.

Ein weiterer Gegenstand der Erfindung sind Arzneimittel, die ein oder mehrere erfindungsgemäße Verbindungen der Formel I und/oder ihre pharmakologisch verträglichen Salze enthalten.

Die Arzneimittel werden nach an sich bekannten, dem Fachmann geläufigen Verfahren hergestellt. Als Arzneimittel werden die erfindungsgemäßen pharmakologisch wirksamen Verbindungen (= Wirkstoffe) entweder als solche, oder vorzugsweise in Kombination mit geeigneten pharmazeutischen Hilfsstoffen in Form von Tabletten, Dragées, Kapseln, Suppositorien, Pflastern (z.B. als TTS), Emulsionen, Suspensionen oder Lösungen eingesetzt, wobei der Wirkstoffgehalt vorteilhafterweise zwischen 0,1 und 95 % beträgt.

Welche Hilfsstoffe für die gewünschten Arzneimittelformulierungen geeignet sind, ist dem Fachmann aufgrund seines Fachwissens geläufig. Neben Lösemitteln, Gelbildnern, Suppositoriengrundlagen, Tabletten-Hilfsstoffen und anderen Wirkstoffträgern können beispielsweise Antioxidantien, Dispergiermittel, Emulgatoren, Entschäumer, Geschmackskorrigentien, Konservierungsmittel, Lösungsvermittler, Farbstoffe oder insbesondere Permeationspromotoren und Komplexbildner (z.B. Cyclodextrine) verwendet werden.

Die Wirkstoffe können oral, parenteral oder percutan appliziert werden.

Im allgemeinen hat es sich in der Humanmedizin als vorteilhaft erwiesen, den oder die Wirkstoffe bei oraler Gabe in einer Tagesdosis von etwa 0,05 bis etwa 50, vorzugsweise 0,25 bis 20, insbesondere 0,5 bis 10 mg/kg Körpergewicht, gegebenenfalls in Form mehrerer, vorzugsweise 1 bis 4 Einzelgaben zur Erzielung des gewünschten Ergebnisses zu verabreichen. Bei einer parenteralen Behandlung können ähnliche bzw. (insbesondere bei der intravenösen Verabreichung der Wirkstoffe) in der Regel niedrigere Dosierungen zur Anwendung kommen. Die Festlegung der jeweils erforderlichen optimalen Dosierung und Applikationsart der Wirkstoffe kann durch jeden Fachmann aufgrund seines Fachwissens leicht erfolgen.

Sollen die erfindungsgemäßen Verbindungen und/oder ihre Salze zur Behandlung der oben genannten Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat; Tranquillizer, wie Benzodiazepine, beispielsweise Diazepam; Spasmolytika, wie z.B. Bietamiverin, Camylofin; Anticholinergica, wie z.B. Oxyphencyclimin, Phencarbamid; Lokalanaesthetika, wie z.B. Tetracain, Procain; gegebenenfalls auch Fermente, Vitamine oder Aminosäuren enthalten.

Hervorzuheben ist in diesem Zusammenhang insbesondere die Kombination der erfindungsgemäßen Verbindungen mit anderen, die Säuresekretion hemmenden Pharmaka, wie beispielsweise $H_2$-Blockern (z.B. Cimetidin, Ranitidin), ferner mit sogenannten peripheren Anticholinergika (z.B. Pirenzepin, Telenzepin, Zolenzepin) sowie mit Gastrin-Antagonisten, mit dem Ziel, die Hauptwirkung in additivem oder überadditivem Sinn zu verstärken und/oder die Nebenwirkungen zu eliminieren oder zu verringern.

## Pharmakologie

Die ausgezeichnete Magenschutzwirkung und die magensekretionshemmende Wirkung der erfindungsgemäßen Verbindungen läßt sich tierexperimentell am Modell Shay-Ratte nachweisen. Die untersuchten erfindungsgemäßen Verbindungen sind wie folgt mit Nummern versehen worden:

| Lfd. Nr. | Name der Verbindung |
|---|---|
| 1 | 5-Difluormethoxy-2-[(4-methoxy-3-methyl-2-pyridyl)methylsulfinyl]-4,6-dimethyl-1H-benzimidazol |
| 2 | 5-Difluormethoxy-6-methoxy-2-{[1-(4-methoxy-2-pyridyl)ethyl]sulfinyl}-1H-benzimidazol |
| 3 | 5-(1,1,2,2-Tetrafluorethoxy)-2-{[1-(4-methoxy-2-pyridyl)ethyl]-sulfinyl}-1H-benzimidazol |
| 4 | 5-Difluormethoxy-2-[(3,4-dimethoxy-2-pyridyl)methylsulfinyl]-4,6-dimethyl-1H-benzimidazol |
| 5 | 2,2-Difluor-6-{[1-(4-methoxy-2-pyridyl)ethyl]sulfinyl}-5H-[1,3]-dioxolo[4,5-f]benzimidazol |
| 6 | 5-(2-Chlor-1,1,2-trifluorethoxy)-2-[(4-methoxy-2-pyridyl)methylsulfinyl)-1H-benzimidazol |
| 7 | 5-(2-Chlor-1,1,2-trifluorethoxy)-2-{[1-(4-methoxy-2-pyridyl)ethyl]-sulfinyl}-1H-benzimidazol |
| 8 | 5-Difluormethoxy-2-[(4-methoxy-3-methyl-2-pyridyl)methylthio]-4,6-dimethyl-1H-benzimidazol |
| 9 | 5-Difluormethoxy-6-methoxy-2-{[1-(4-methoxy-2-pyridyl)ethyl]thio}-1H-benzimidazol |
| 10 | 5-(2-Chlor-1,1,2-trifluorethoxy)-2-[(4-methoxy-3-methyl-2-pyridyl)methylsulfinyl]-1H-benzimidazol |

Der Einfluß der untersuchten Verbindungen auf die durch Pylorusligatur und orale Gabe von 100 mg/kg Acetylsalicylsäure ausgelöste Magenläsionsbildung sowie die Magensekretion (HCl) während 4 h bei der Ratte (sog. Shay-Ratte), ist in der folgenden Tabelle dargestellt.

Magenschutzwirkung und Magensekretionshemmung

| Lfd. Nr. | Magenschutzwirkung (Ratte) Hemmung d. Läsionsindexes, ED50+) [mg/kg, p.o.] | Hemmung der HCl-Sekretion d. Magens (Ratte; Summe 4 h) | | |
|---|---|---|---|---|
| | | % Hemmung der HCl-Sekretion ++) | ED25+) [mg/kg, p.o.] | ED50+) |
| 1 | 0.50 | 22 | 0.50 | 1.1 |
| 2 | 0.60 | ~ 20 | ~ 1 | ~ 2 |
| 3 | 0.50 | 35 | 0.35 | 0.8 |
| 4 | ~ 1 | 10 | ~ 1.3 | ~ 2 |
| 5 | 1 | 25 | ~ 1 | 1.7 |
| 6 | 0.55 | 25 | 0.55 | 1.3 |
| 7 | 0.6 | 40 | 0.4 | 0.8 |
| 8 | 0.7 | 15 | 1 | 1 |
| 9 | 1.7 | 35 | < 1 | ~ 4 |
| 10 | 0.4 | 27 | 0.4 | 0.8 |

+) ED25 bzw. ED50 = Dosis, die den Läsionsindex bzw. die HCl-Sekretion (Σ 4h) des Rattenmagens bei der behandelten Gruppe gegenüber der Kontrollgruppe um 25 bzw. 50 % mindert.

++) nach Gabe der antiulcerösen ED50

Die Prüfung der antiulcerogenen Wirkung erfolgte nach der Methode der sogenannten Shay-Ratte:

Die Ulcusprovokation erfolgt bei 24 Stunden nüchtern gehaltenen Ratten (weiblich, 180-200 g, 4 Tiere je Käfig auf hohem Gitterrost) durch Pylorusligatur (unter Diethylethernarkose) und orale Applikation von 100 mg/10 ml/kg Acetylsalicylsäure. Die zu prüfenden Substanzen werden oral (10 ml/kg) eine Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) und später sein HCl-Gehalt (Titration mit Natronlauge) bestimmt wurde; mit einem Stereomikroskop werden bei 10-facher Vergrößerung Anzahl und Größe (=Durchmesser) vorhandener Ulcera ermittelt. Das Produkt aus Schweregrad (gemäß nachfolgender Punkteskala) und Anzahl der Ulcera dient als individueller Läsionsindex.

| Punkteskala: | | |
|---|---|---|
| keine Ulcera | | 0 |
| Ulcusdurchmesser | 0,1 - 1,4 mm | 1 |
| | 1,5 - 2,4 mm | 2 |
| | 2,5 - 3,4 mm | 3 |
| | 3,5 - 4,4 mm | 4 |
| | 4,5 - 5,4 mm | 5 |
| | > 5,5 mm | 6 |

Als Maß für den antiulcerogenen Effekt dient die Minderung des mittlere Läsionsindex jeder behandelten Gruppe gegenüber dem der Kontrollgruppe (=100%). Die ED25 bzw. ED50 bezeichnen diejenigen Dosen, die den mittle Läsionsindex bzw. die HCl-Sekretion gegenüber der Kontrolle um 25% bzw. 50% mindern.

Toxizität

Die LD50 aller geprüften Verbindungen liegt oberhalb von 1000 mg/kg [p.o.] bei der Maus.

**Patentansprüche**

**1.** Neue Picolinderivate der Formel I

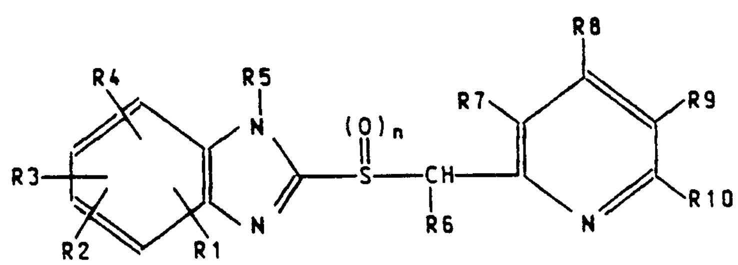

(I),

worin

R1, R2, R3 und R4 an beliebigen Positionen im Benzoteil des Benzimidazols stehen können und worin

- R1 Wasserstoff oder $C_1$-$C_6$-Alkyl bedeutet,
- R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,
- R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,
- R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,

R5 Wasserstoff oder eine unter physiologischen Bedingungen leicht abspaltbare Gruppe bedeutet,
R6 Wasserstoff oder $C_1$-$C_4$-Alkyl bedeutet,
R7 Wasserstoff, $C_1$-$C_6$-Alkyl oder $C_1$-$C_6$-Alkoxy bedeutet,
R8 Wasserstoff, $C_1$-$C_6$-Alkyl, $C_1$-$C_6$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,
R9 Wasserstoff, $C_1$-$C_6$-Alkyl oder $C_1$-$C_6$-Alkoxy bedeutet,
R10 Wasserstoff oder $C_1$-$C_6$-Alkyl bedeutet und
n die Zahlen 0 oder 1 darstellt,

und die Salze dieser Verbindungen, wobei ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und
R5 Wasserstoff und
R6 Wasserstoff und
R7 Wasserstoff oder $C_1$-$C_6$-Alkyl oder $C_1$-$C_6$-Alkoxy und
R8 Wasserstoff oder $C_1$-$C_6$-Alkyl oder $C_1$-$C_6$-Alkoxy und
R9 Wasserstoff oder $C_1$-$C_6$-Alkyl oder $C_1$-$C_6$-Alkoxy und
R10 Wasserstoff

bedeutet und in 4- und 7-Position des Benzimidazols Wasserstoffatome stehen.

**2.** Verbindungen der Formel I nach Anspruch 1, worin R1, R2, R3 und R4 an beliebigen Positionen im Benzoteil des Benzimidazols stehen können und worin

R1 Wasserstoff oder $C_1$-$C_6$-Alkyl bedeutet,
R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,
R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,
R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,
R5 Wasserstoff oder eine unter physiologischen Bedingungen leicht abspaltbare Gruppe bedeutet,
R6 Wasserstoff oder $C_1$-$C_4$-Alkyl bedeutet,
R7 Wasserstoff oder $C_1$-$C_6$-Alkyl bedeutet,
R8 Wasserstoff, $C_1$-$C_6$-Alkyl, $C_1$-$C_6$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,
R9 Wasserstoff oder $C_1$-$C_6$-Alkyl bedeutet,
R10 Wasserstoff oder $C_1$-$C_6$-Alkyl bedeutet und
n die Zahlen 0 oder 1 darstellt,

und die Salze dieser Verbindungen, wobei ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder

Chlortrifluorethylendioxy und
R5 Wasserstoff und
R6 Wasserstoff und
R7 Wasserstoff oder $C_1$-$C_6$-Alkyl und
R8 Wasserstoff oder $C_1$-$C_6$-Alkyl oder $C_1$-$C_6$-Alkoxy und
R9 Wasserstoff oder $C_1$-$C_6$-Alkyl und
R10 Wasserstoff
bedeutet und in 4- und 7-Position des Benzimidazols Wasserstoffatome stehen.

**3.** Verbindungen der Formel I nach Anspruch 1, worin
R1, R2, R3 und R4 an beliebigen Positionen im Benzoteil des Benzimidazols stehen können und worin
R1 Wasserstoff oder $C_1$-$C_6$-Alkyl bedeutet,
R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,
R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,
R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet,
R5 Wasserstoff oder eine unter physiologischen Bedingungen leicht abspaltbare Gruppe bedeutet,
R6 Wasserstoff oder $C_1$-$C_4$-Alkyl bedeutet,
R8 Wasserstoff, $C_1$-$C_6$-Alkyl, $C_1$-$C_6$-Alkoxy, $C_1$-$C_4$-Alkoxy-$C_1$-$C_4$-alkoxy, $C_2$-$C_5$-Alkenyloxy oder $C_2$-$C_5$-Alkinyloxy bedeutet,
einer der Substituenten R7 und R9 $C_1$-$C_6$-Alkoxy und der andere Wasserstoff, $C_1$-$C_6$-Alkyl oder $C_1$-$C_6$-Alkoxy bedeutet,
R10 Wasserstoff oder $C_1$-$C_6$-Alkyl bedeutet und
n die Zahlen 0 oder 1 darstellt,
und die Salze dieser Verbindungen, wobei
ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes $C_1$-$C_4$-Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy haben kann, wenn
R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und
R5 Wasserstoff und
R6 Wasserstoff und
R8 Wasserstoff oder $C_1$-$C_6$-Alkyl oder $C_1$-$C_6$-Alkoxy und
einer der Substituenten R7 und R9 $C_1$-$C_6$-Alkoxy und der andere Wasserstoff oder $C_1$-$C_6$-Alkyl oder $C_1$-$C_6$-Alkoxy und
R10 Wasserstoff
bedeutet und in 4- und 7-Position des Benzimidazols Wasserstoffatome stehen.

**4.** Verbindungen der Formel I nach Anspruch 1 oder 2 oder 3, worin R6 Wasserstoff und R10 Wasserstoff bedeutet und R1, R2, R3, R4, R5, R7, R8, R9 und n die in den Ansprüchen 1 oder 2 oder 3 angegebenen Bedeutungen haben, und ihre Salze.

**5.** Verbindungen der Formel I nach Anspruch 1 oder 2 oder 3, worin R6 $C_1$-$C_4$-Alkyl und R10 Wasserstoff bedeutet und R1, R2, R3, R4, R5, R7, R8, R9 und n die in den Ansprüchen 1 oder 2 oder 3 angegebenen Bedeutungen haben, und ihre Salze.

**6.** Verbindungen der Formel I nach Anspruch 1, worin

R1 Wasserstoff, Methyl oder Ethyl bedeutet,
R2 Wasserstoff, Chlor, Fluor, Methyl, Ethyl, Methoxy oder Ethoxy, bedeutet,
R3 Wasserstoff, Methyl, Ethyl, Methoxy, Ethoxy, 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R4 Difluormethylendioxy, 1,1,2-Trifluorethylendioxy oder 1-Chlor-1,2,2-trifluorethylendioxy bedeutet,
R4 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R3 Difluormethylendioxy, 1,1,2-Trifluorethylendioxy oder 1-Chlor-1,2,2-trifluorethylendioxy bedeutet,
R5 Wasserstoff bedeutet,
R6 Wasserstoff, Methyl oder Ethyl bedeutet,
R7 Wasserstoff, Methyl, Ethyl, Methoxy oder Ethoxy bedeutet,
R8 Wasserstoff, Methyl, Ethyl, Methoxy, Ethoxy oder Allyloxy bedeutet,
R9 Wasserstoff, Methyl, Ethyl, Methoxy oder Ethoxy bedeutet,
R10 Wasserstoff, Methyl oder Ethyl bedeutet und
n die Zahlen 0 oder 1 darstellt, und die Salze dieser Verbindungen, wobei

ein in 5- bzw. 6-Position im Benzimidazol stehender Substituent R1, R2 oder R3 nicht die Bedeutung Wasserstoff, Chlor, Fluor, Methyl, Ethyl, Methoxy, Ethoxy, 1,1,2,2-Tetrafluorethoxy, Trifluormethoxy, 2,2,2-Trifluorethoxy, Difluormethoxy oder gemeinsam mit R4 - wenn dieses in 6- bzw. 5-Position steht - Difluormethylendioxy, 1,1,2-Trifluorethylendioxy oder 1-Chlor-1,2,2-trifluorethylendioxy haben kann, wenn

R4 in 6- bzw. 5-Position steht und 1,1,2,2-Tetrafluorethoxy, Trifluormethoxy, 2,2,2-Trifluorethoxy, Difluormethoxy oder gemeinsam mit R3 - wenn dieses in 5- bzw. 6-Position steht - Difluormethylendioxy, 1,1,2-Trifluorethylendioxy oder 1-Chlor-1,2,2-trifluorethylendioxy und
R5 Wasserstoff und
R6 Wasserstoff und
R7 Wasserstoff oder Methyl oder Ethyl oder Methoxy oder Ethoxy und
R8 Wasserstoff oder Methyl oder Ethyl oder Methoxy oder Ethoxy und
R9 Wasserstoff oder Methyl oder Ethyl oder Methoxy oder Ethoxy und
R10 Wasserstoff

bedeutet und in 4- und 7-Position des Benzimidazols Wasserstoffatome stehen.

**7.** Verbindungen der Formel I nach Anspruch 1, worin

R1 Wasserstoff, Methyl oder Ethyl bedeutet,
R2 Wasserstoff, Chlor, Fluor, Methyl, Ethyl, Methoxy oder Ethoxy, bedeutet,
R3 Wasserstoff, Methyl, Ethyl, Methoxy, Ethoxy, 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R4 Difluormethylendioxy, 1,1,2-Trifluorethylendioxy oder 1-Chlor-1,2,2-trifluorethylendioxy bedeutet,
R4 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R3 Difluormethylendioxy, 1,1,2-Trifluorethylendioxy oder 1-Chlor-1,2,2-trifluorethylendioxy bedeutet,
R5 Wasserstoff bedeutet,
R6 Wasserstoff, Methyl oder Ethyl bedeutet,
R7 Wasserstoff, Methyl oder Ethyl bedeutet,
R8 Wasserstoff, Methyl, Ethyl, Methoxy, Ethoxy oder Allyloxy bedeutet,
R9 Wasserstoff, Methyl oder Ethyl bedeutet,
R10 Wasserstoff, Methyl oder Ethyl bedeutet und
n die Zahlen 0 oder 1 darstellt, und die Salze dieser Verbindungen,

wobei
ein in 5- bzw. 6-Position im Benzimidazol stehender Substituent R1, R2 oder R3 nicht die Bedeutung Wasserstoff, Chlor, Fluor, Methyl, Ethyl, Methoxy, Ethoxy, 1,1,2,2-Tetrafluorethoxy, Trifluormethoxy, 2,2,2-Trifluorethoxy, Difluormethoxy oder gemeinsam mit R4 - wenn dieses in 6- bzw. 5-Position steht - Difluormethylendioxy, 1,1,2-Trifluorethylendioxy oder 1-Chlor-1,2,2-trifluorethylendioxy haben kann, wenn

R4 in 6- bzw. 5-Position steht und 1,1,2,2-Tetrafluorethoxy, Trifluormethoxy, 2,2,2-Trifluorethoxy, Difluormethoxy oder gemeinsam mit R3 - wenn dieses in 5- bzw. 6-Position steht - Difluormethylendioxy, 1,1,2-Trifluorethylendioxy oder 1-Chlor-1,2,2-trifluorethylendioxy und

R5 Wasserstoff und
R6 Wasserstoff und
R7 Wasserstoff oder Methyl oder Ethyl und
R8 Wasserstoff oder Methyl oder Ethyl oder Methoxy oder Ethoxy und
R9 Wasserstoff oder Methyl oder Ethyl und
R10 Wasserstoff
bedeutet und in 4- und 7-Position des Benzimidazols Wasserstoffatome stehen.

**8.** Verbindungen der Formel I nach Anspruch 1, worin
R1 Wasserstoff, Methyl oder Ethyl bedeutet,
R2 Wasserstoff, Chlor, Fluor, Methyl, Ethyl, Methoxy oder Ethoxy, bedeutet,
R3 Wasserstoff, Methyl, Ethyl, Methoxy, Ethoxy, 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R4 Difluormethylendioxy, 1,1,2-Trifluorethylendioxy oder 1-Chlor-1,2,2-trifluorethylendioxy bedeutet,
R4 1,1,2,2-Tetrafluorethoxy, Trifluormethoxy, 2,2,2-Trifluorethoxy, Difluormethoxy, 2-Chlor-1,1,2-trifluorethoxy oder gemeinsam mit R3 Difluormethylendioxy, 1,1,2-Trifluorethylendioxy oder 1-Chlor-1,2,2-trifluorethylendioxy bedeutet,
R5 Wasserstoff bedeutet,
R6 Wasserstoff, Methyl oder Ethyl bedeutet,
R8 Wasserstoff, Methyl, Ethyl, Methoxy, Ethoxy oder Allyloxy bedeutet,
einer der Substituenten R7 und R9 Methoxy oder Ethoxy und der andere Wasserstoff, Methyl, Ethyl, Methoxy oder Ethoxy bedeutet,
R10 Wasserstoff, Methyl oder Ethyl bedeutet und
n die Zahlen 0 oder 1 darstellt, und die Salze dieser Verbindungen,
wobei
ein in 5- bzw. 6-Position im Benzimidazol stehender Substituent R1, R2 oder R3 nicht die Bedeutung Wasserstoff, Chlor, Fluor, Methyl, Ethyl, Methoxy, Ethoxy, 1,1,2,2-Tetrafluorethoxy, Trifluormethoxy, 2,2,2-Trifluorethoxy, Difluormethoxy oder gemeinsam mit R4 - wenn dieses in 6- bzw. 5-Position steht - Difluormethylendioxy, 1,1,2-Trifluorethylendioxy oder 1-Chlor-1,2,2-trifluorethylendioxy
haben kann, wenn
R4 in 6- bzw. 5-Position steht und 1,1,2,2-Tetrafluorethoxy, Trifluormethoxy, 2,2,2-Trifluorethoxy, Difluormethoxy oder gemeinsam mit R3 - wenn dieses in 5- bzw. 6-Position steht - Difluormethylendioxy, 1,1,2-Trifluorethylendioxy oder 1-Chlor-1,2,2-trifluorethylendioxy und
R5 Wasserstoff und
R6 Wasserstoff und
R8 Wasserstoff oder Methyl oder Ethyl oder Methoxy oder Ethoxy und
einer der Substituenten R7 und R9 Methoxy oder Ethoxy und der andere Wasserstoff, Methyl, Ethyl, Methoxy oder Ethoxy und
R10 Wasserstoff bedeutet und in 4- und 7-Position des Benzimidazols Wasserstoffatome stehen.

**9.** Verbindungen der Formel I nach Anspruch 6 oder 7 oder 8, in denen R6 Wasserstoff und R10 Wasserstoff bedeutet.

**10.** Verbindungen der Formel I nach Anspruch 6 oder 7 oder 8, in denen R6 Methyl oder Ethyl und R10 Wasserstoff bedeutet.

**11.** Verbindungen der Formel I nach Anspruch 1, worin
R1 Wasserstoff bedeutet,
R2 Wasserstoff, Methyl oder Methoxy bedeutet,
R3 Wasserstoff, Methyl, Methoxy, 1,1,2,2-Tetrafluorethoxy, Difluormethoxy, 2-Chlor-1,1,2-trifluorethoxy oder gemeinsam mit R4 Difluormethylendioxy bedeutet,
R4 1,1,2,2-Tetrafluorethoxy, Difluormethoxy, 2-Chlor-1,1,2-trifluorethoxy oder gemeinsam mit R3 Difluormethylendioxy bedeutet,
R5 Wasserstoff bedeutet,
R6 Wasserstoff oder Methyl bedeutet,
R7 Wasserstoff, Methyl oder Methoxy bedeutet,
R8 Methoxy bedeutet,

R9 Wasserstoff bedeutet,
R10 Wasserstoff bedeutet und
n die Zahlen 0 oder 1 darstellt, und die Salze dieser Verbindungen,

wobei
ein in 5- bzw. 6-Position im Benzimidazol stehender Substituent R1, R2 oder R3 nicht die Bedeutung Wasserstoff, Methyl, Methoxy, 1,1,2,2-Tetrafluorethoxy,Difluormethoxy oder gemeinsam mit R4 - wenn dieses in 6- bzw. 5-Position steht - Difluormethylendioxy
haben kann, wenn

R4 in 6- bzw. 5-Position steht und 1,1,2,2-Tetrafluorethoxy, Difluormethoxy oder gemeinsam mit R3 - wenn dieses in 5- bzw. 6-Position steht - Difluormethylendioxy und
R5 Wasserstoff und
R6 Wasserstoff und
R7 Wasserstoff, Methyl oder Methoxy und
R8 Methoxy und
R9 Wasserstoff und
R10 Wasserstoff

bedeutet und in 4- und 7-Position des Benzimidazols Wasserstoffatome stehen.

**12.** Verbindung gemäß Anspruch 1 ausgewählt aus der Gruppe bestehend aus
5-Difluormethoxy-2-[(4-methoxy-3-methyl-2-pyridyl)methylsulfinyl]-4,6-dimethyl-1H-benzimidazol,
5-Difluormethoxy-6-methoxy-2-{[1-(4-methoxy-2-pyridyl)ethyl]sulfinyl}-1H-benzimidazol,
5-(1,1,2,2-Tetrafluorethoxy)-2-{[1-(4-methoxy-2-pyridyl)ethyl]sulfinyl}-1H-benzimidazol,
5-Difluormethoxy-2-[(3,4-dimethoxy-2-pyridyl)methylsulfinyl]-4,6-dimethyl-1H-benzimidazol,
2,2-Difluor-6-{[1-(4-methoxy-2-pyridyl)ethyl]sulfinyl}-5H-[1,3]-dioxolo[4,5-f]benzimidazol,
5-(2-Chlor-1,1,2-trifluorethoxy]-2-[(4-methoxy-2-pyridyl)methylsulfinyl]-1H-benzimidazol,
5-(2-Chlor-1,1,2-trifluorethoxy)-2-{[1-(4-methoxy-2-pyridyl)ethyl]sulfinyl}-1H-benzimidazol,
5-Difluormethoxy-2-[(4-methoxy-3-methyl-2-pyridyl)methylthio]-4,6-dimethyl-1H-benzimidazol,
5-Difluormethoxy-6-methoxy-2-{[1-(4-methoxy-2-pyridyl)ethyl]thio}-1H-benzimidazol,
5-(2-Chlor-1,1,2-trifluorethoxy)-2-[(4-methoxy-3-methyl-2-pyridyl)methylsulfinyl]-1H-benzimidazol
und den pharmakologisch verträglichen Salzen dieser Verbindungen.

**13.** Verfahren zur Herstellung der Verbindungen der Formel I nach Anspruch 1 und ihren Salzen, dadurch gekennzeichnet, daß man
a) Mercaptobenzimidazole der Formel II mit Picolinderivaten III,

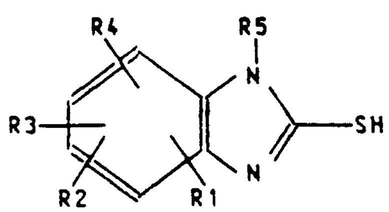

(II),

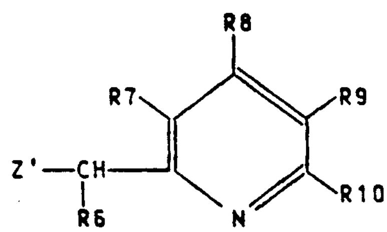

(III),

oder
b) Benzimidazole der Formel IV mit Mercaptopicolinen V,

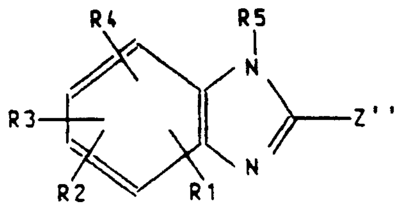

(IV),

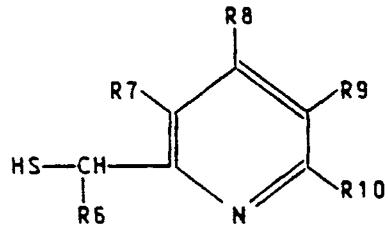

(V),

oder

c) o-Phenylendiamine der Formel VI mit Ameisensäurederivaten VII

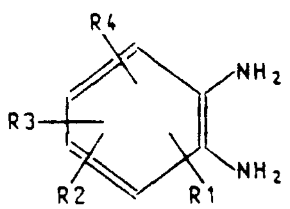

(VI),

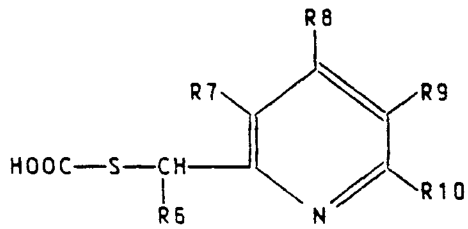

(VII),

umsetzt und (falls Verbindungen der Formel I mit n = 1 die gewünschten Endprodukte sind) anschließend die nach a), b) oder c) erhaltenen 2-Benzimidazolyl-2-pyridylmethyl-sulfide der Formel VIII ( = Verbindungen der Formel I mit n = 0)

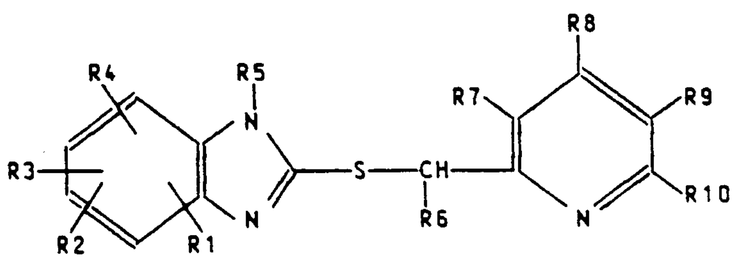

(VIII),

oxidiert und/oder gewünschtenfalls in die Salze überführt, oder daß man

d) Benzimidazole der Formel IX mit Pyridinderivaten X

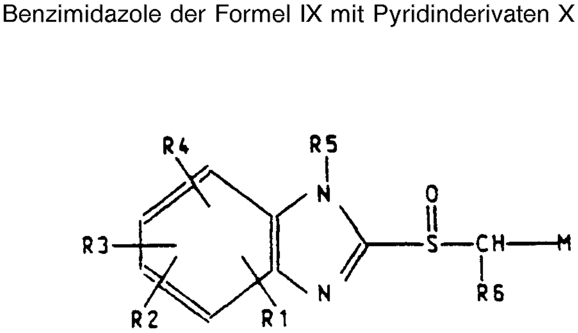

(IX),

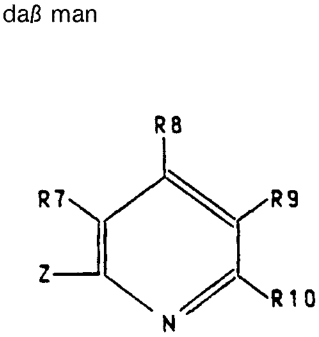

(X),

oder

e) Sulfinylderivate der Formel XI mit 2-Picolinderivaten XII

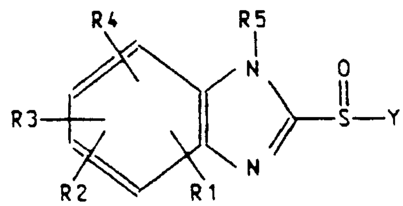
(XI),

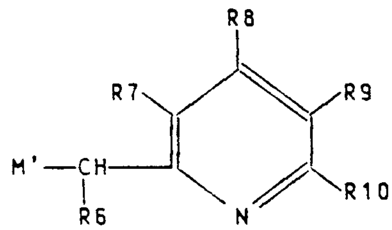
(XII),

umsetzt und gewünschtenfalls anschließend in die Salze überführt, oder

f) - falls Verbindungen der Formel I, worin R5 eine unter physiologischen Bedingungen leicht abspaltbare Gruppe darstellt, die herzustellenden Verfahrensprodukte sind - daß man Verbindungen der Formel I, worin R5 Wasserstoff bedeutet, mit Verbindungen der Formel R5'-Y' (XIII), worin R5' die gewünschte, unter physiologischen Bedingungen leicht abspaltbare Gruppe oder gemeinsam mit Y' ihr Vorläufer ist, umsetzt und gewünschtenfalls anschließend in die Salze überführt, oder

g) - falls Verbindungen der Formel I, worin R5 Wasserstoff bedeutet, die herzustellenden Verfahrensprodukte sind - daß man Verbindungen der Formel I, worin R5 eine unter physiologischen Bedingungen leicht abspaltbare Gruppe darstellt, solvolysiert, und die erhaltenen Produkte gewünschtenfalls in die Salze überführt, oder

h) - falls Verbindungen der Formel I, worin R6 $C_1$-$C_6$-Alkyl bedeutet, die herzustellenden Verfahrensprodukte sind - daß man Verbindungen der Formel I, worin R6 Wasserstoff bedeutet, mit Verbindungen der Formel R6-Y'' (XIV), worin R6 $C_1$-$C_6$-Alkyl bedeutet, alkyliert und gewünschtenfalls anschließend in die Salze überführt,

wobei Y, Y'', Z, Z' und Z'' geeignete Abgangsgruppen darstellen, Y' eine Abgangs- bzw. Reaktivgruppe darstellt, M für ein Alkalimetallatom (Li, Na oder K) steht, M' für das Äquivalent eines Metallatoms steht und R1, R2, R3, R4, R5, R6, R7, R8, R9, R10 und n (sofern nicht anders definiert) die in Anspruch 1 angegebenen Bedeutungen haben.

**14.** Arzneimittel enthaltend eine oder mehrere Verbindungen nach einem oder mehreren der Ansprüche 1 bis 12 und/oder ihre pharmakologisch verträglichen Salze.

**15.** Verbindungen nach einem oder mehreren der Ansprüche 1 bis 12 und ihre pharmakologisch verträglichen Salze zur Anwendung bei der Behandlung und/oder Prophylaxe von Krankheiten des Magens und/oder Darms und solcher Krankheiten, die auf einer erhöhten Magensäuresekretion beruhen.

**16.** Verwendung von Verbindungen nach einem oder mehreren der Ansprüche 1 bis 12 und ihren pharmakologisch verträglichen Salzen zur Herstellung von Arzneimitteln für die Behandlung und/oder Prophylaxe von Krankheiten des Magens und/oder Darms und solcher Krankheiten, die auf einer erhöhten Magensäuresekretion beruhen.

**17.** Mercaptobenzimidazole der Formel II

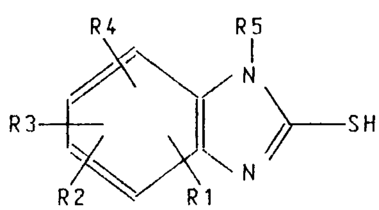
(II)

worin

R1, R2, R3 und R4 an beliebigen Positionen im Benzoteil des Benzimidazols stehen können und worin

R1 Wasserstoff oder $C_1$-$C_6$-Alkyl bedeutet,

R2 Wasserstoff, Halogen, $C_1$-$C_6$-Alkyl, $C_1$-$C_6$-Alkoxy, $C_1$-$C_4$-Alkoxy-$C_1$-$C_4$-alkyl, $C_1$-$C_4$-Alkoxy-

$C_1$-$C_4$-alkoxy, Phenyl, Phenoxy, Phenoxy-$C_1$-$C_4$-alkyl, Phenoxy-$C_1$-$C_4$-alkoxy, Phen-$C_1$-$C_4$-alkyl oder Phen-$C_1$-$C_4$-alkoxy bedeutet,

R3 Wasserstoff, $C_1$-$C_6$-Alkyl, $C_1$-$C_6$-Alkoxy, ganz oder überwiegend durch Fluor substituiertes $C_1$-$C_4$-Alkoxy, Chlordifluormethoxy, 2-Chlor-1,1,2-trifluorethoxy oder gemeinsam mit R4 ganz oder teilweise durch Fluor substituiertes $C_1$-$C_2$-Alkylendioxy oder Chlortrifluorethylendioxy bedeutet,

R4 ganz oder überwiegend durch Fluor substituiertes $C_1$-$C_4$-Alkoxy, Chlordifluormethoxy, 2-Chlor-1,1,2-trifluorethoxy oder gemeinsam mit R3 ganz oder teilweise durch Fluor substituiertes $C_1$-$C_2$-Alkylendioxy oder Chlortrifluorethylendioxy bedeutet und

R5 Wasserstoff oder eine unter physiologischen Bedingungen leicht abspaltbare Gruppe bedeutet,

und die Salze dieser Verbindungen, wobei

ein in 5- bzw. 6-Position im Benzimidazol stehender Substituent R1, R2 oder R3 nicht die Bedeutung Wasserstoff, Halogen, $C_1$-$C_6$-Alkyl, $C_1$-$C_6$-Alkoxy, ganz oder überwiegend durch Fluor substituiertes C -C -Alkoxy, Chlordifluormethoxy oder gemeinsam mit R4 - wenn dieses in 6- bzw. 5-Position steht - ganz oder teilweise durch Fluor substituiertes $C_1$-$C_2$-Alkylendioxy oder Chlortrifluorethylendioxy

haben kann, wenn

R4 in 6- bzw. 5-Position steht und ganz oder überwiegend durch Fluor substituiertes $C_1$-$C_4$-Alkoxy oder Chlordifluormethoxy oder gemeinsam mit R3 - wenn dieses in 5- bzw. 6-Position steht - ganz oder teilweise durch Fluor substituiertes $C_1$-$C_2$-Alkylendioxy oder Chlortrifluorethylendioxy und

R5 Wasserstoff

bedeutet und in 4- und 7-Position des Benzimidazols Wasserstoffatome stehen.

**18.** Verbindungen der Formel II nach Anspruch 17, worin

R1 Wasserstoff bedeutet,

R2 Wasserstoff, Methyl oder Methoxy bedeutet,

R3 Wasserstoff, Methyl, Methoxy, 1,1,2,2-Tetrafluorethoxy, Difluormethoxy, 2-Chlor-1,1,2-trifluorethoxy oder gemeinsam mit R4 Difluormethylendioxy bedeutet,

R4 1,1,2,2-Tetrafluorethoxy, Difluormethoxy, 2-Chlor-1,1,2-trifluorethoxy oder gemeinsam mit R3 Difluormethylendioxy bedeutet und

R5 Wasserstoff bedeutet,

wobei

ein in 5- bzw. 6-Position im Benzimidazol stehender Substituent R1, R2 oder R3 nicht die Bedeutung Wasserstoff, Methyl, Methoxy, 1,1,2,2-Tetrafluorethoxy, Difluormethoxy oder gemeinsam mit R4 - wenn dieses in 6-bzw. 5-Position steht - Difluormethylendioxy

haben kann, wenn

R4 in 6- bzw. 5-Position steht und 1,1,2,2-Tetrafluorethoxy, Difluormethoxy oder gemeinsam mit R3 - wenn dieses in 5- bzw. 6-Positionen steht - Difluormethylendioxy

bedeutet und in 4- und 7-Position des Benzimidazols Wasserstoffatome stehen.

## Claims

**1.** New picoline derivatives of the formula I

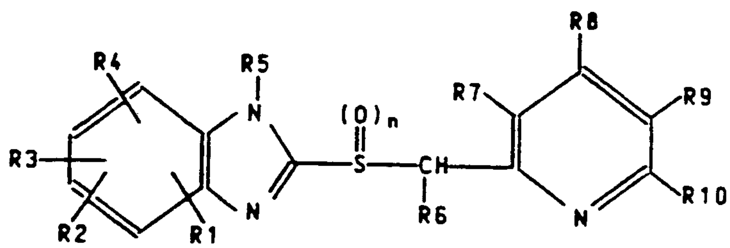

(I),

wherein

R1, R2, R3 and R4 can be in any desired positions in the benzo part of the benzimidazole and wherein

R1 denotes hydrogen or $C_1$-$C_6$-alkyl,

R2 denotes hydrogen, halogen, $C_1$-$C_6$-alkyl, $C_1$-$C_6$-alkoxy, $C_1$-$C_4$-alkoxy-$C_1$-$C_4$-alkyl, $C_1$-$C_4$-alkoxy-$C_1$-$C_4$-alkoxy, phenyl, phenoxy, phenoxy-$C_1$-$C_4$-alkyl, phenoxy-$C_1$-$C_4$-alkoxy, phen-$C_1$-$C_4$-alkyl or phen-$C_1$-$C_4$-alkoxy,

R3 denotes hydrogen, $C_1$-$C_6$-alkyl, $C_1$-$C_6$-alkoxy, $C_1$-$C_4$-alkoxy wholly or predominantly substituted by fluorine, chlorodifluoromethoxy or 2-chloro-1,1,2-trifluoroethoxy or, together with R4, denotes $C_1$-$C_2$-alkylenedioxy wholly or partially substituted by fluorine, or chlorotrifluoroethylenedioxy,

R4 denotes $C_1$-$C_4$-alkoxy wholly or predominantly substituted by fluorine, chlorodifluoromethoxy or 2-chloro-1,1,2-trifluoroethoxy or, together with R3, denotes $C_1$-$C_1$-alkylenedioxy wholly or partially substituted by fluorine, or chlorotrifluoroethylenedioxy,

R5 denotes hydrogen or a group which can readily be eliminated under physiological conditions,

R6 denotes hydrogen or $C_1$-$C_4$-alkyl,

R7 denotes hydrogen, $C_1$-$C_6$-alkyl or $C_1$-$C_6$-alkoxy,

R8 denotes hydrogen, $C_1$-$C_6$-alkyl, $C_1$-$C_6$-alkoxy, $C_1$-$C_4$-alkoxy-$C_1$-$C_4$-alkoxy, $C_2$-$C_5$-alkenyloxy or $C_2$-$C_5$-alkynyloxy,

R9 denotes hydrogen or $C_1$-$C_6$-alkyl or $C_1$-$C_6$-alkoxy

R10 denotes hydrogen or $C_1$-$C_6$-alkyl and

n represents the numbers 0 or 1,

and the salts of these compounds, with the proviso that
a substituent R1, R2 or R3 in the 5- or 6-position in the benzimidazole cannot have the meanings of hydrogen, halogen, $C_1$-$C_6$-alkyl, $C_1$-$C_6$-alkoxy, $C_1$-$C_4$-alkoxy wholly or predominantly substituted by fluorine, chlorodifluoromethoxy or, together with R4 - if this is in the 6- or 5-position - $C_1$-$C_2$-alkylenedioxy wholly or partially substituted by fluorine, or chlorotrifluoroethylenedioxy, if

R4 is in the 6- or 5-position and denotes $C_1$-$C_4$-alkoxy wholly or predominantly substituted by fluorine or chlorodifluoromethoxy or, together with R3 - if this is in the 5- or 6-position - $C_1$-$C_2$-alkylenedioxy wholly or partially substituted by fluorine, or chlorotrifluoroethylenedioxy and

R5 denotes hydrogen and

R6 denotes hydrogen and

R7 denotes hydrogen or $C_1$-$C_6$-alkyl or $C_1$-$C_6$-alkoxy and

R8 denotes hydrogen or $C_1$-$C_6$-alkyl or $C_1$-$C_6$-alkoxy and

R9 denotes hydrogen or $C_1$-$C_6$-alkyl or $C_1$-$C_6$-alkoxy and

R10 denotes hydrogen, and

hydrogen atoms are present in the 4- and 7-positions of the benzimidazole.

**2.** Compounds of the formula I according to Claim 1, wherein
R1, R2, R3 and R4 can be in any desired positions in the benzo part of the benzimidazole and wherein

R1 denotes hydrogen or $C_1$-$C_6$-alkyl,

R2 denotes hydrogen, halogen, $C_1$-$C_6$-alkyl, $C_1$-$C_6$-alkoxy, $C_1$-$C_4$-alkoxy-$C_1$-$C_4$-alkyl, $C_1$-$C_4$-alkoxy-$C_1$-$C_4$-alkoxy, phenyl, phenoxy, phenoxy-$C_1$-$C_4$-alkyl, phenoxy-$C_1$-$C_4$-alkoxy, phen-$C_1$-$C_4$-alkyl or phen-$C_1$-$C_4$-alkoxy,

R3 denotes hydrogen, $C_1$-$C_6$-alkyl, $C_1$-$C_6$-alkoxy, $C_1$-$C_4$-alkoxy wholly or predominantly substituted by fluorine, chlorodifluoromethoxy or 2-chloro-1,1,2-trifluoroethoxy or, together with R4, denotes $C_1$-$C_2$-alkylenedioxy wholly or partially substituted by fluorine, or chlorotrifluoroethylenedioxy,

R4 denotes $C_1$-$C_4$-alkoxy wholly or predominantly substituted by fluorine, chlorodifluoromethoxy or 2-chloro-1,1,2-trifluoroethoxy or, together with R3, denotes $C_1$-$C_2$-alkylenedioxy wholly or partially substituted by fluorine, or chlorotrifluoroethylenedioxy,

R5 denotes hydrogen or a group which can readily be eliminated under physiological conditions,

R6 denotes hydrogen or $C_1$-$C_4$-alkyl,

R7 denotes hydrogen or $C_1$-$C_6$-alkyl and

R8 denotes hydrogen, $C_1$-$C_6$-alkyl, $C_1$-$C_6$-alkoxy, $C_1$-$C_4$-alkoxy-$C_1$-$C_4$-alkoxy, $C_2$-$C_5$-alkenyloxy or $C_2$-$C_5$-alkynyloxy,

R9 denotes hydrogen or $C_1$-$C_6$-alkyl,

R10 denotes hydrogen or $C_1$-$C_6$-alkyl and

n represents the numbers 0 or 1,

and the salts of these compounds, with the proviso that
a substituent R1, R2 or R3 in the 5- or 6-position in the benzimidazole cannot have the meanings of hydrogen, halogen, $C_1$-$C_6$-alkyl, $C_1$-$C_6$-alkoxy, $C_1$-$C_4$-alkoxy wholly or predominantly substituted by fluorine, or chlorodifluoromethoxy or, together with R4 - if this is in the 6- or 5-position - $C_1$-$C_2$-alkylenedioxy wholly or partially substituted by fluorine, or chlorotrifluoroethylenedioxy, if

R4 is in the 6- or 5-position and denotes $C_1$-$C_4$-alkoxy wholly or predominantly substituted by fluorine or chlorodifluoromethoxy or, together with R3 - if this is in the 5- or 6-position - $C_1$-$C_2$-alkylenedioxy wholly or partially substituted by fluorine, or chlorotrifluoroethylenedioxy and
R5 denotes hydrogen and
R6 denotes hydrogen and
R7 denotes hydrogen or $C_1$-$C_6$-alkyl and
R8 denotes hydrogen or $C_1$-$C_6$-alkyl or $C_1$-$C_6$-alkoxy and
R9 denotes hydrogen or $C_1$-$C_6$-alkyl and
R10 denotes hydrogen, and

hydrogen atoms are present in the 4- and 7-positions of the benzimidazole.

**3.** Compounds of the formula I according to Claim 1, wherein
R1, R2, R3 and R4 can be in any desired positions in the benzo part of the benzimidazole and wherein

R1 denotes hydrogen or $C_1$-$C_6$-alkyl,
R2 denotes hydrogen, halogen, $C_1$-$C_6$-alkyl, $C_1$-$C_6$-alkoxy, $C_1$-$C_4$-alkoxy-$C_1$-$C_4$-alkyl, $C_1$-$C_4$-alkoxy-$C_1$-$C_4$-alkoxy, phenyl, phenoxy, phenoxy-$C_1$-$C_4$-alkyl, phenoxy-$C_1$-$C_4$-alkoxy, phen-$C_1$-$C_4$-alkyl or phen-$C_1$-$C_4$-alkoxy,
R3 denotes hydrogen, $C_1$-$C_6$-alkyl, $C_1$-$C_6$-alkoxy, $C_1$-$C_4$-alkoxy wholly or predominantly substituted by fluorine, chlorodifluoromethoxy or 2-chloro-1,1,2-trifluoroethoxy or, together with R4, denotes $C_1$-$C_2$-alkylenedioxy wholly or partially substituted by fluorine, or chlorotrifluoroethylenedioxy,
R4 denotes $C_1$-$C_4$-alkoxy wholly or predominantly substituted by fluorine, chlorodifluoromethoxy or 2-chloro-1,1,2-trifluoroethoxy or, together with R3, denotes $C_1$-$C_2$-alkylenedioxy wholly or partially substituted by fluorine, or chlorotrifluoroethylenedioxy,
R5 denotes hydrogen or a group which can readily be eliminated under physiological conditions,
R6 denotes hydrogen or $C_1$-$C_4$-alkyl,
R8 denotes hydrogen, $C_1$-$C_6$-alkyl, $C_1$-$C_6$-alkoxy, $C_1$-$C_4$-alkoxy-$C_1$-$C_4$-alkoxy, $C_2$-$C_5$-alkenyloxy or $C_2$-$C_5$-alkynyloxy,

one of the substituents R7 and R9 denotes $C_1$-$C_6$-alkoxy and the other denotes hydrogen, $C_1$-$C_6$-alkyl or $C_1$-$C_6$-alkoxy,

R10 denotes hydrogen or $C_1$-$C_6$-alkyl and
n represents the numbers 0 or 1,

and the salts of these compounds, with the proviso that
a substituent R1, R2 or R3 in the 5- or 6-position in the benzimidazole cannot have the meanings of hydrogen, halogen, $C_1$-$C_6$-alkyl, $C_1$-$C_6$-alkoxy, $C_1$-$C_4$-alkoxy wholly or predominantly substituted by fluorine, chlorodifluoromethoxy or, together with R4 - if this is in the 6- or 5-position - $C_1$-$C_2$-alkylenedioxy wholly or partially substituted by fluorine, or chlorotrifluoroethylenedioxy, if

R4 is in the 6- or 5-position and denotes $C_1$-$C_4$-alkoxy wholly or predominantly substituted by fluorine or chlorodifluoromethoxy or, together with R3 - if this is in the 5- or 6-position - $C_1$-$C_2$-alkylenedioxy wholly or partially substituted by fluorine, or chlorotrifluoroethylenedioxy and
R5 denotes hydrogen and
R6 denotes hydrogen and
R8 denotes hydrogen or $C_1$-$C_6$-alkyl or $C_1$-$C_6$-alkoxy and

one of the substituents R7 and R9 denotes $C_1$-$C_6$-alkoxy and the other denotes hydrogen, $C_1$-$C_6$-alkyl or $C_1$-$C_6$-alkoxy and

R10 denotes hydrogen, and

hydrogen atoms are present in the 4- and 7-positions of the benzimidazole.

**4.** Compounds of the formula I according to Claim 1 or 2 or 3, wherein R6 denotes hydrogen and R10 denotes hydrogen and R1, R2, R3, R4, R5, R7, R8, R9 and n have the meanings given in Claims 1 or 2 or 3, and their salts.

5. Compounds of the formula I according to Claim 1 or 2 or 3, wherein R6 denotes $C_1$-$C_4$-alkyl and R10 denotes hydrogen and R1, R2, R3, R4, R5, R7, R8, R9 and n have the meanings given in Claims 1 or 2 or 3, and their salts.

6. Compounds of the formula I according to Claim 1, wherein
   R1 denotes hydrogen, methyl or ethyl,
   R2 denotes hydrogen, chlorine, fluorine, methyl, ethyl, methoxy or ethoxy,
   R3 denotes hydrogen, methyl, ethyl, methoxy, ethoxy, 1,1,2,2-tetrafluoroethoxy, trifluoromethoxy, 2,2,2-trifluoroethoxy, difluoromethoxy or 2-chloro-1,1,2-trifluoroethoxy or, together with R4, denotes difluoromethylenedioxy, 1,1,2-trifluoroethylenedioxy or 1-chloro-1,2,2-trifluoroethylenedioxy,
   R4 denotes 1,1,2,2-tetrafluoroethoxy, trifluoromethoxy, 2,2,2-trifluoroethoxy, difluoromethoxy or 2-chloro-1,1,2-trifluoroethoxy or, together with R3, denotes difluoromethylenedioxy, 1,1,2-trifluoroethylenedioxy or 1-chloro-1,2,2-trifluoroethylenedioxy,
   R5 denotes hydrogen,
   R6 denotes hydrogen, methyl or ethyl,
   R7 denotes hydrogen, methyl, ethyl, methoxy or ethoxy,
   R8 denotes hydrogen, methyl, ethyl, methoxy, ethoxy or allyloxy,
   R9 denotes hydrogen, methyl, ethyl, methoxy or ethoxy,
   R10 denotes hydrogen, methyl or ethyl and
   n represents the numbers 0 or 1,

   and the salts of these compounds, with the proviso that
   a substituent R1, R2 or R3 in the 5- or 6-position in the benzimidazole cannot have the meanings of hydrogen, chlorine, fluorine, methyl, ethyl, methoxy, ethoxy, 1,1,2,2-tetrafluoroethoxy, trifluoromethoxy, 2,2,2-trifluoroethoxy or difluoromethoxy or, together with R4 - if this is in the 6- or 5-position - of difluoromethylenedioxy, 1,1,2-trifluoroethylenedioxy or 1-chloro-1,2,2-trifluoroethylenedioxy, if
   R4 is in the 6- or 5-position and denotes 1,1,2,2-tetrafluoroethoxy, trifluoromethoxy, 2,2,2-trifluoroethoxy or difluoromethoxy or, together with R3 - if this is in the 5- or 6-position - denotes difluoromethylenedioxy, 1,1,2-trifluoroethylenedioxy or 1-chloro-1,2,2-trifluoroethylenedioxy and
   R5 denotes hydrogen and
   R6 denotes hydrogen and
   R7 denotes hydrogen or methyl or ethyl or methoxy or ethoxy and
   R8 denotes hydrogen or methyl or ethyl or methoxy or ethoxy and
   R9 denotes hydrogen or methyl or ethyl or methoxy or ethoxy and
   R10 denotes hydrogen

   and hydrogen atoms are present in the 4- and 7-positions of the benzimidazole.

7. Compounds of the formula I according to Claim 1, wherein
   R1 denotes hydrogen, methyl or ethyl,
   R2 denotes hydrogen, chlorine, fluorine, methyl, ethyl, methoxy or ethoxy,
   R3 denotes hydrogen, methyl, ethyl, methoxy, ethoxy, 1,1,2,2-tetrafluoroethoxy, trifluoromethoxy, 2,2,2-trifluoroethoxy, difluoromethoxy or 2-chloro-1,1,2-trifluoroethoxy or, together with R4, denotes difluoromethylenedioxy, 1,1,2-trifluoroethylenedioxy or 1-chloro-1,2,2-trifluoroethylenedioxy,
   R4 denotes 1,1,2,2-tetrafluoroethoxy, trifluoromethoxy, 2,2,2-trifluoroethoxy, difluoromethoxy or 2-chloro-1,1,2-trifluoroethoxy or, together with R3, denotes difluoromethylenedioxy, 1,2,2-trifluoroethylenedioxy or 1-chloro-1,2,2-trifluoroethylenedioxy,
   R5 denotes hydrogen,
   R6 denotes hydrogen, methyl or ethyl,
   R7 denotes hydrogen, methyl or ethyl,
   R8 denotes hydrogen, methyl, ethyl, methoxy, ethoxy or allyloxy,
   R9 denotes hydrogen, methyl or ethyl,
   R10 denotes hydrogen, methyl or ethyl and
   n represents the numbers 0 or 1, and

   the salts of these compounds, with the proviso that
   a substituent R1, R2 or R3 in the 5- or 6-position in the benzimidazole cannot have the meanings of hydrogen, chlorine, fluorine, methyl, ethyl, methoxy, ethoxy, 1,1,2,2-tetrafluoroethoxy, trifluoromethoxy,

2,2,2-trifluoroethoxy or difluoromethoxy or, together with R4 - if this is in the 6- or 5-position - of difluoromethylenedioxy, 1,1,2-trifluoroethylenedioxy or 1-chloro-1,2,2-trifluoroethylenedioxy, if

R4 is in the 6- or 5-position and denotes 1,1,2,2-tetrafluoroethoxy, trifluoromethoxy, 2,2,2-trifluoroethoxy or difluoromethoxy or, together with R3 - if this is in the 5- or 6-position - denotes difluoromethylenedioxy, 1,1,2-trifluoroethylenedioxy or 1-chloro-1,2,2-trifluoroethylenedioxy and

R5 denotes hydrogen and

R6 denotes hydrogen and

R7 denotes hydrogen or methyl or ethyl and

R8 denotes hydrogen or methyl or ethyl or methoxy or ethoxy and

R9 denotes hydrogen or methyl or ethyl and

R10 denotes hydrogen

and hydrogen atoms are present in the 4- and 7-positions of the benzimidazole.

**8.** Compounds of the formula I according to Claim 1, wherein

R1 denotes hydrogen, methyl or ethyl,

R2 denotes hydrogen, chlorine, fluorine, methyl, ethyl, methoxy or ethoxy,

R3 denotes hydrogen, methyl, ethyl, methoxy, ethoxy, 1,1,2,2-tetrafluoroethoxy, trifluoromethoxy, 2,2,2-trifluoroethoxy, difluoromethoxy or 2-chloro-1,1,2-trifluoroethoxy or, together with R4, denotes difluoromethylenedioxy, 1,1,2-trifluoroethylenedioxy or 1-chloro-1,2,2-trifluoroethylenedioxy,

R4 denotes 1,1,2,2-tetrafluoroethoxy, trifluoromethoxy, 2,2,2-trifluoroethoxy, difluoromethoxy or 2-chloro-1,1,2-trifluoroethoxy or, together with R3, denotes difluoromethylenedioxy, 1,1,2-trifluoroethylenedioxy or 1-chloro-1,2,2-trifluoroethylenedioxy,

R5 denotes hydrogen,

R6 denotes hydrogen, methyl or ethyl,

R8 denotes hydrogen, methyl, ethyl, methoxy, ethoxy or allyloxy,

one of the substituents R7 and R9 denotes methoxy or ethoxy and the other denotes hydrogen, methyl, ethyl, methoxy or ethoxy,

R10 denotes hydrogen, methyl or ethyl and

n represents the numbers 0 or 1,

and the salts of these compounds, with the proviso that

a substituent R1, R2 or R3 in the 5- or 6-position in the benzimidazole cannot have the meanings of hydrogen, chlorine, fluorine, methyl, ethyl, methoxy, ethoxy, 1,1,2,2-tetrafluoroethoxy, trifluoromethoxy, 2,2,2-trifluoroethoxy or difluoromethoxy or, together with R4 - if this is in the 6- or 5-position - of difluoromethylenedioxy, 1,1,2-trifluoroethylenedioxy or 1-chloro-1,2,2-trifluoroethylenedioxy, if

R4 is in the 6- or 5-position and denotes 1,1,2,2-tetrafluoroethoxy, trifluoromethoxy, 2,2,2-trifluoroethoxy or difluoromethoxy or, together with R3 - if this is in the 5- or 6-position - denotes difluoromethylenedioxy, 1,1,2-trifluoroethylenedioxy or 1-chloro-1,2,2-trifluoroethylenedioxy and

R5 denotes hydrogen and

R6 denotes hydrogen and

R8 denotes hydrogen or methyl or ethyl or methoxy or ethoxy and

one of the substituents R7 and R9 denotes methoxy or ethoxy and the other denotes hydrogen, methyl, ethyl, methoxy or ethoxy and

R10 denotes hydrogen,

and hydrogen atoms are present in the 4- and 7-positions of the benzimidazole.

**9.** Compounds of the formula I according to Claim 6 or 7 or 8, in which R6 denotes hydrogen and R10 denotes hydrogen.

**10.** Compounds of the formula I according to Claim 6 or 7 or 8, in which R6 denotes methyl or ethyl and R10 denotes hydrogen.

**11.** Compounds of the formula I according to Claim 1, wherein

R1 denotes hydrogen,

R2 denotes hydrogen, methyl or methoxy,

R3 denotes hydrogen, methyl, methoxy, 1,1,2,2-tetrafluoroethoxy, difluoromethoxy or 2-chloro-1,1,2-trifluoroethoxy or, together with R4, denotes difluoromethylenedioxy,

R4 denotes 1,1,2,2-tetrafluoroethoxy, difluoromethoxy or 2-chloro-1,1,2-trifluoroethoxy or, together with R3, denotes difluoromethylenedioxy,

R5 denotes hydrogen,

R6 denotes hydrogen or methyl,

R7 denotes hydrogen, methyl or methoxy,

R8 denotes methoxy,

R9 denotes hydrogen,

R10 denotes hydrogen and

n represents the numbers 0 or 1,

and the salts of these compounds, with the proviso that

a substituent R1, R2 or R3 in the 5- or 6-position in the benzimidazole cannot have the meanings of hydrogen, methyl, methoxy, 1,1,2,2-tetrafluoroethoxy or difluoromethoxy or, together with R4 - if this is in the 6- or 5-position - of difluoromethylenedioxy, if

R4 is in the 6- or 5-position and denotes 1,1,2,2-tetrafluoroethoxy or difluoromethoxy or, together with R3 - if this is in the 5- or 6-position - denotes difluoromethylenedioxy and

R5 denotes hydrogen and

R6 denotes hydrogen and

R7 denotes hydrogen, methyl or methoxy and

R8 denotes methoxy and

R9 denotes hydrogen and

R10 denotes hydrogen,

and hydrogen atoms are present in the 4- and 7-positions of the benzimidazole.

**12.** A compound according to Claim 1 selected from the group comprising 5-difluoromethoxy-2-[(4-methoxy-3-methyl-2-pyridyl)methylsulphinyl]-4,6-dimethyl-1H-benzimidazole,
5-difluoromethoxy-6-methoxy-2-{[1-(4-methoxy-2-pyridyl)ethyl]sulphinyl}-1H-benzimidazole,
5-(1,1,2,2-tetrafluoroethoxy)-2-{[1-(4-methoxy-2-pyridyl)ethyl]sulphinyl}-1H-benzimidazole,
5-difluoromethoxy-2-[(3,4-dimethoxy-2-pyridyl)methylsulphinyl]-4,6-dimethyl-1H-benzimidazole,
2,2-difluoro-6-{[1-(4-methoxy-2-pyridyl)ethyl]sulphinyl}-5H-[1,3]-dioxolo[4,5-f]benzimidazole,
5-(2-chloro-1,1,2-trifluoroethoxy)-2-[(4-methoxy-2-pyridyl)methylsulphinyl]-1H-benzimidazole,
5-(2-chloro-1,1,2-trifluoroethoxy)-2-{[1-(4-methoxy-2-pyridyl)ethyl]sulphinyl}-1H-benzimidazole,
5-difluoromethoxy-2-[(4-methoxy-3-methyl-2-pyridyl)methylthio]-4,6-dimethyl-1H-benzimidazole,
5-difluoromethoxy-6-methoxy-2-{[1-(4-methoxy-2-pyridyl)ethyl]thio}-1H-benzimidazole,
5-(2-Chloro-1,1,2-trifluoroethoxy)-2-[(4-methoxy-3-methyl-2-pyridyl)methylsulphinyl]-1H-benzimidazole
and the pharmacologically acceptable salts of these compounds.

**13.** Process for the preparation of the compounds of the formula I according to Claim 1 and their salts, characterised in that a) mercaptobenzimidazoles of the formula II are reacted with picoline derivatives III,

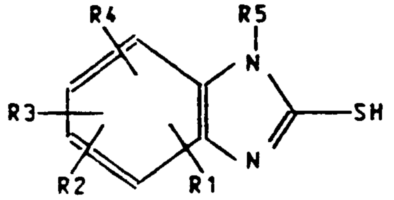

(II),

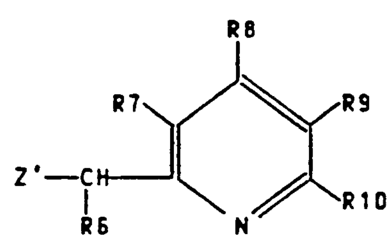

(III),

or

b) benzimidazoles of the formula IV are reacted with mercaptopicolines V,

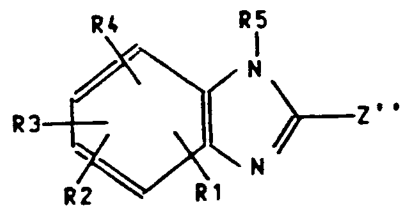

(IV),

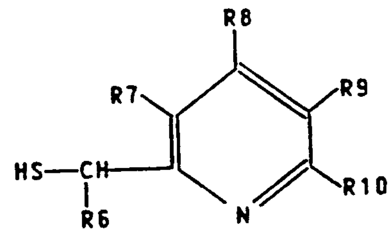

(V),

or

c) o-phenylenediamines of the formula VI are reacted with formic acid derivatives VII

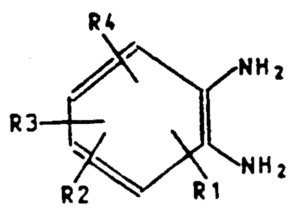

(VI),

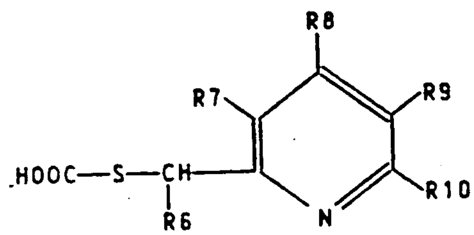

(VII),

and (if compounds of the formula I with n = 1 are the desired end products) the 2-benzimidazolyl 2-pyridylmethyl sulphides of the formula VIII (= compounds of the formula I with n = 0)

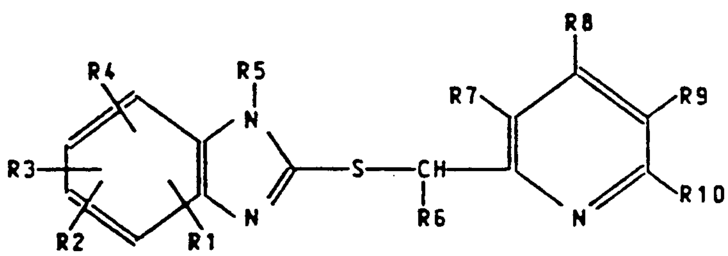

(VIII),

obtained according to a), b) or c) are then oxidised and/or, if desired, converted into the salts, or that

d) benzimidazoles of the formula IX are reacted with pyridine derivatives X

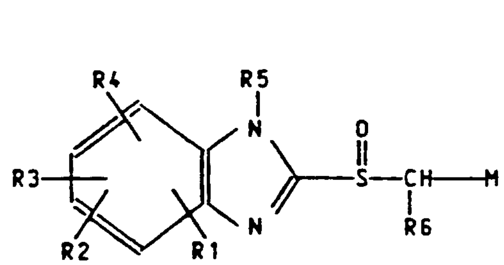

(IX),

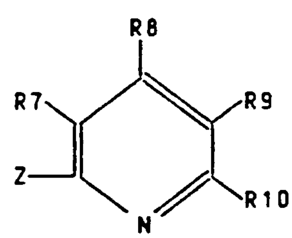

(X),

or

e) sulphinyl derivatives of the formula XI are reacted with 2-picoline derivatives XII

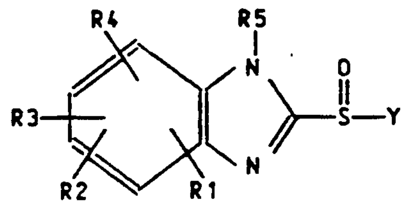

(XI),

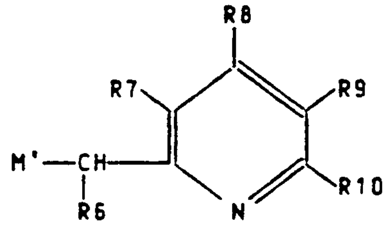

(XII),

and, if desired, are then converted into the salts, or

f) - if compounds of the formula I, wherein R5 represents a group which can readily be eliminated under physiological conditions, are the process products to be prepared - that compounds of the formula I wherein R5 denotes hydrogen are reacted with compounds of the formula R5'-Y' (XIII), wherein R5' is the desired group which can be readily eliminated under physiological conditions or, together with Y' is its precursor, and, if desired, the products are then converted into the salts, or

g) - if compounds of the formula I, wherein R5 denotes hydrogen, are the process products to be prepared - compounds of the formula I, wherein R5 represents a group which can be readily eliminated under physiological conditions, are solvolysed, and the products obtained are, if desired, converted into the salts, or

h) - if compounds of the formula I, wherein R6 denotes $C_1$-$C_6$-alkyl, are the process products to be prepared - compounds of the formula I, wherein R6 denotes hydrogen, are alkylated with compounds of the formula R6-Y'' (XIV), wherein R6 denotes $C_1$-$C_6$-alkyl, and, if desired, are then converted into the salts,

Y, Y'', Z, Z' and Z'' representing suitable leaving groups, Y' representing a leaving group or reactive group, M representing an alkali metal atom (Li, Na or K), M' representing the equivalent of a metal atom and R1, R2, R3, R4 R5, R6, R7, R8, R9, R10 and n (unless otherwise defined) having the meanings given in Claim 1.

**14.** Medicaments containing one or more compounds according to one or more of Claims 1 to 12 and/or their pharmacologically acceptable salts.

**15.** Compounds according to one or more of Claims 1 to 12 and their pharmacologically acceptable salts for use in the treatment and/or prophylaxis of diseases of the stomach and/or intestines and of those diseases which are based on increased secretion of gastric acid.

**16.** Use of compounds according to one or more of Claims 1 to 12 and of their pharmacologically acceptable salts for the preparation of medicaments for the treatment and/or prophylaxis of diseases of the stomach and/or intestines and of those diseases which are based on increased secretion of gastric acid.

**17.** Mercaptobenzimidazoles of the formula II

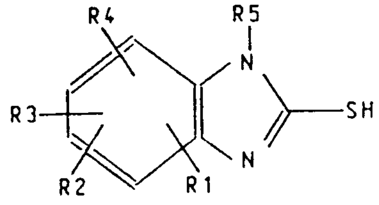

(II)

wherein

R1, R2, R3 and R4 can be in any desired positions in the benzo part of the benzimidazole and wherein

R1 denotes hydrogen or $C_1$-$C_6$-alkyl,

R2 denotes hydrogen, halogen, $C_1$-$C_6$-alkyl, $C_1$-$C_6$-alkoxy, $C_1$-$C_4$-alkoxy-$C_1$-$C_4$-alkyl, $C_1$-$C_4$-alkoxy-$C_1$-$C_4$-alkoxy, phenyl, phenoxy, phenoxy-$C_1$-$C_4$-alkyl, phenoxy-$C_1$-$C_4$-alkoxy, phen-$C_1$-$C_4$-alkyl or phen-$C_1$-$C_4$-alkoxy,

R3 denotes hydrogen, $C_1$-$C_6$-alkyl, $C_1$-$C_6$-alkoxy, $C_1$-$C_4$-alkoxy wholly or predominantly substituted by fluorine, chlorodifluoromethoxy or 2-chloro-1,1,2-trifluoroethoxy or, together with R4, denotes $C_1$-$C_2$-alkylenedioxy wholly or partially substituted by fluorine, or chlorotrifluoroethylenedioxy,

R4 denotes $C_1$-$C_4$-alkoxy wholly or predominantly substituted by fluorine, chlorodifluoromethoxy or 2-chloro-1,1,2-trifluoroethoxy or, together with R3, denotes $C_1$-$C_2$-alkylenedioxy wholly or partially substituted by fluorine, or chlorotrifluoroethylenedioxy and

R5 denotes hydrogen or a group which can readily be eliminated under physiological conditions,

and the salts of these compounds, with the proviso that

a substituent R1, R2 or R3 in the 5- or 6-position in the benzimidazole cannot have the meanings of hydrogen, halogen, $C_1$-$C_6$-alkyl, $C_1$-$C_6$-alkoxy, $C_1$-$C_4$-alkoxy wholly or predominantly substituted by fluorine, chlorodifluoromethoxy or, together with R4 - if this is in the 6- or 5-position - $C_1$-$C_2$-alkylenedioxy wholly or partially substituted by fluorine, or chlorotrifluoroethylenedioxy, if

R4 is in the 6- or 5-position and denotes $C_1$-$C_4$-alkoxy wholly or predominantly substituted by fluorine or chlorodifluoromethoxy or, together with R3 - if this is in the 5- or 6-position - $C_1$-$C_2$-alkylenedioxy wholly or partially substituted by fluorine, or chlorotrifluoroethylenedioxy and

R5 denotes hydrogen and

hydrogen atoms are present in the 4- and 7-positions of the benzimidazole.

**18.** Compounds of formula II according to claim 17, wherein

R1 denotes hydrogen,

R2 denotes hydrogen, methyl or methoxy,

R3 denotes hydrogen, methyl, methoxy, 1,1,2,2-tetrafluoroethoxy, difluoromethoxy or 2-chloro-1,1,2-trifluoroethoxy or, together with R4, denotes difluoromethylenedioxy,

R4 denotes 1,1,2,2-tetrafluoroethoxy, difluoromethoxy or 2-chloro-1,1,2-trifluoroethoxy or, together with R3, denotes difluoromethylenedioxy and

R5 denotes hydrogen,

with the proviso that a substituent R1, R2 or R3 in the 5- or 6-position in the benzimidazole cannot have the meanings of

hydrogen, methyl, methoxy, 1,1,2,2-tetrafluoroethoxy or difluoromethoxy or, together with R4 - if this is in the 6- or 5- position - of difluoromethylenedioxy, if

R4 is in the 6- or 5-position and denotes 1,1,2,2-tetrafluoroethoxy or difluoromethoxy or, together with R3 - if this is in the 5- or 6-position - denotes difluoromethylenedioxy

and hydrogen atoms are present in the 4- and 7-positions of the benzimidazole.

## Revendications

**1.** Nouveaux dérivés de la picoline, de formule I

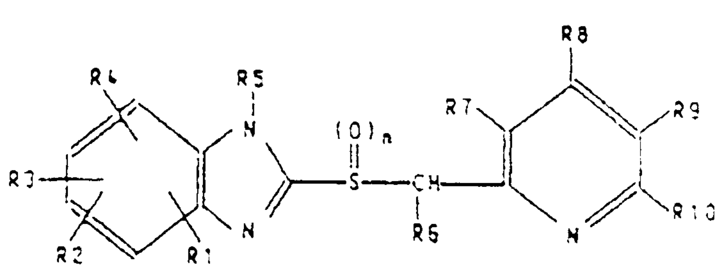

(I),

dans laquelle

R1, R2, R3 et R4 peuvent être situés en des positions quelconques de la partie benzénique du benzimidazole, et

R1 représente un atome d'hydrogène ou un groupe alkyle en $C_1$-$C_6$,
R2 représente un atome d'hydrogène ou d'halogène, un groupe alkyle en $C_1$-$C_6$, alcoxy en $C_1$-$C_6$, alcoxy (en $C_1$-$C_4$)-alkyle (en $C_1$-$C_4$), alcoxy (en $C_1$-$C_4$)-alcoxy ($C_1$-$C_4$), phényle, phénoxy, phénoxy-alkyle (dont le groupe alkyle est en $C_1$-$C_4$), phénoxy-alcoxy (dont le groupe alcoxy est en $C_1$-$C_4$), phène-alkyle (dont le groupe alkyle est en $C_1$-$C_4$) ou phène-alcoxy (dont le groupe alcoxy est en $C_1$-$C_4$),
R3 représente un atome d'hydrogène, un groupe alkyle en $C_1$-$C_6$, alcoxy en $C_1$-$C_6$, alcoxy en $C_1$-$C_4$ entièrement substitué par du fluor ou substitué de façon prédominante par du fluor, un groupe chlorodifluorométhoxy, 2-chloro-1,1,2-trifluoréthoxy ou bien, il forme avec R4 un groupe alkylènedioxy en $C_1$ ou $C_2$, entièrement ou partiellement substitué par du fluor, ou un groupe chlorotrifluoréthylènedioxy,
R4 représente un groupe alcoxy en $C_1$-$C_4$, entièrement substitué par du fluor ou substitué de façon prédominante par du fluor, un groupe chlorodifluorométhoxy, 2-chloro-1,1,2-trifluoréthoxy ou, avec R3, il forme un groupe alkylènedioxy en $C_1$ ou $C_2$, entièrement ou partiellement substitué par du fluor, ou un groupe chlorotrifluoréthylènedioxy,
R5 représente un atome d'hydrogène ou un groupe facilement scindable dans des conditions physiologiques,
R6 représente un atome d'hydrogène ou un groupe alkyle en $C_1$-$C_4$,
R7 représente un atome d'hydrogène, un groupe alkyle en $C_1$-$C_6$ ou alcoxy en $C_1$-$C_6$,
R8 représente un atome d'hydrogène, un groupe alkyle en $C_1$-$C_6$, alcoxy en $C_1$-$C_6$, alcoxy (en $C_1$-$C_4$)-alcoxy(en $C_1$-$C_4$), alcényloxy en $C_2$-$C_5$ ou alcynyloxy en $C_2$-$C_5$,
R9 représente un atome d'hydrogène, un groupe alkyle en $C_1$-$C_6$ ou alcoxy en $C_1$-$C_6$,
R10 représente un atome d'hydrogène ou un groupe alkyle en $C_1$-$C_6$, et
n est un nombre valant 0 ou 1,

et les sels de ces composés,
l'un des substituants R1, R2 ou R3 fixé en position 5 ou 6 du système benzimidazole ne pouvant pas représenter
un atome d'hydrogène ou d'halogène, un groupe alkyle en $C_1$-$C_6$, alcoxy en $C_1$-$C_6$, alcoxy en $C_1$-$C_4$, entièrement substitué par du fluor ou substitué de façon prédominante par du fluor, un groupe chlorodifluorométhoxy ou, avec R4 (lorsque celui-ci se trouve en position 6 ou 5, un groupe alkylènedioxy en $C_1$ ou $C_2$ entièrement ou partiellement substitué par du fluor ou un groupe chlorotrifluoréthylènedioxy, lorsque
R4 se trouve en position 6 ou 5 et représente un groupe alcoxy en $C_1$-$C_4$, entièrement substitué par du fluor ou substitué de façon prédominante par du fluor, ou un groupe chlorodifluorométhoxy ou, avec R3 - lorsque celui-ci se trouve en position 5 ou 6 - un groupe alkylènedioxy en $C_1$ ou $C_2$, entièrement ou partiellement substitué par du fluor, ou un groupe chlorotrifluoréthylènedioxy, et
R5 représente un atome d'hydrogène, et
R6 représente un atome d'hydrogène, et
R7 représente un atome d'hydrogène ou un groupe alkyle en $C_1$-$C_6$ ou alcoxy en $C_1$-$C_6$, et
R8 représente un atome d'hydrogène ou un groupe alkyle en $C_1$-$C_6$ ou alcoxy en $C_1$-$C_6$, et
R9 représente un atome d'hydrogène ou un groupe alkyle en $C_1$-$C_6$ ou alcoxy en $C_1$-$C_6$, et
R10 représente un atome d'hydrogène, et que des atomes d'hydrogène se trouvent en position 4 et 7 du noyau benzimidazole.

**2.** Composés de formule I selon la revendication 1, dans lesquels R1, R2, R3 et R4 peuvent être situés sur des positions quelconques de la partie benzénique du benzimidazole, et
R1 représente un atome d'hydrogène ou un groupe alkyle en $C_1$-$C_6$,
R2 représente un atome d'hydrogène ou d'halogène, un groupe alkyle en $C_1$-$C_6$, alcoxy en $C_1$-$C_6$, alcoxy(en $C_1$-$C_4$)-alkyle (en $C_1$-$C_4$), alcoxy(en $C_1$-$C_4$)-alcoxy(en $C_1$-$C_4$), phényle, phénoxy, phénoxy-alkyle (en $C_1$-$C_4$), phénoxy-alcoxy (en $C_1$-$C_4$), phènealkyle(en $C_1$-$C_4$) ou phènealcoxy(en $C_1$-$C_4$),
R3 représente un atome d'hydrogène, un groupe alkyle en $C_1$-$C_6$, alcoxy en $C_1$-$C_6$, alcoxy(en $C_1$-$C_4$) entièrement substitué par du fluor ou substitué de façon prédominante par du fluor, un groupe chlorodifluorométhoxy, 2-chloro-1,1,2-trifluoréthoxy ou, avec R4, un groupe alkylènedioxy en $C_1$ ou $C_2$, entièrement ou partiellement substitué par du fluor, ou un groupe chlorotrifluoréthylènedioxy,
R4 représente un groupe alcoxy en $C_1$-$C_4$, entièrement substitué par du fluor ou substitué de

façon prédominante par du fluor, un groupe chlorodifluorométhoxy, 2-chloro-1,1,2-trifluoréthoxy ou, avec R3, un groupe alkylènedioxy en $C_1$ ou $C_2$, entièrement ou partiellement substitué par du fluor, ou un groupe chlorotrifluoréthylènedioxy,

R5 représente un atome d'hydrogène ou un groupe facilement scindable dans des conditions physiologiques,

R6 représente un atome d'hydrogène ou un groupe alkyle en $C_1$-$C_4$,

R7 représente un atome d'hydrogène ou un groupe alkyle en $C_1$-$C_6$,

R8 représente un atome d'hydrogène, un groupe alkyle en $C_1$-$C_6$, alcoxy en $C_1$-$C_6$, alcoxy(en $C_1$-$C_4$)-alcoxy(en $C_1$-$C_4$), alcényloxy en $C_2$-$C_5$ ou alcynyloxy en $C_2$-$C_5$,

R9 représente un atome d'hydrogène ou un groupe alkyle en $C_1$-$C_6$,

R10 représente un atome d'hydrogène ou un groupe alkyle en $C_1$-$C_6$, et

n est un nombre valant 0 ou 1,

et les sels de ces composés,

l'un des substituants R1, R2, ou R3, situé en position 5 ou 6 du noyau benzimidazole, ne pouvant pas représenter un atome d'hydrogène ou d'halogène, un groupe alkyle en $C_1$-$C_6$, alcoxy en $C_1$-$C_6$, alcoxy en $C_1$-$C_4$ entièrement substitué par du fluor ou substitué de façon prédominante par du fluor, un groupe chlorodifluorométhoxy ou, avec R4 - lorsque celui-ci se trouve en position 6 ou 5 - un groupe alkylènedioxy en $C_1$ ou $C_2$, entièrement ou partiellement substitué par du fluor, ou un groupe chlorotrifluoréthylènedioxy, lorsque

R4 se trouve en position 6 ou 5 et représente un groupe alcoxy en $C_1$-$C_4$, entièrement substitué par du fluor ou substitué de façon prédominante par du fluor, ou un groupe chlorodifluorométhoxy ou, avec R3 - quand celui-ci se trouve en position 5 ou 6 - un groupe alkylènedioxy en $C_1$ ou $C_2$, entièrement ou partiellement substitué par du fluor, ou un groupe chlorotrifluoréthylènedioxy, et

R5 représente un atome d'hydrogène, et

R6 représente un atome d'hydrogène, et

R7 représente un atome d'hydrogène ou un groupe alkyle en $C_1$-$C_6$, et

R8 représente un atome d'hydrogène ou un groupe alkyle en $C_1$-$C_6$ ou alcoxy en $C_1$-$C_6$, et

R9 représente un atome d'hydrogène ou un groupe alkyle en $C_1$-$C_6$, et

R10 représente un atome d'hydrogène, et

les atomes d'hydrogène se trouvent sur les positions 4 et 7 du benzimidazole.

3. Composés de formule I selon la revendication 1, dans laquelle R1, R2, R3 et R4 peuvent être en des positions quelconques de la partie benzénique du benzimidazole, et

R1 représente un atome d'hydrogène ou un groupe alkyle en $C_1$-$C_6$,

R2 représente un atome d'hydrogène ou d'halogène, un groupe alkyle en $C_1$-$C_6$, alcoxy en $C_1$-$C_6$, alcoxy(en $C_1$-$C_4$)-alkyle (en $C_1$-$C_4$), alcoxy(en $C_1$-$C_4$)-alcoxy(en $C_1$-$C_4$), phényle, phénoxy, phénoxy-alkyle (en $C_1$-$C_4$), phénoxy-alcoxy (en $C_1$-$C_4$), phènealkyle(en $C_1$-$C_4$) ou phènealcoxy(en $C_1$-$C_4$),

R3 représente un atome d'hydrogène, un groupe alkyle en $C_1$-$C_6$, alcoxy en $C_1$-$C_6$, alcoxy en $C_1$-$C_4$, entièrement substitué par du fluor ou substitué de façon prédominante par du fluor, un groupe chlorodifluorométhoxy, 2-chloro-1,1,2-trifluoréthoxy ou, avec R4, un groupe alkylènedioxy en $C_1$ ou $C_2$, entièrement ou partiellement substitué par du fluor, ou un groupe chlorotrifluoréthylènedioxy,

R4 représente un groupe alcoxy en $C_1$-$C_4$, entièrement substitué par du fluor ou substitué de façon prédominante par du fluor, un groupe chlorodifluorométhoxy, 2-chloro-1,1,2-trifluoréthoxy ou, avec R3, un groupe alkylènedioxy en $C_1$ ou $C_2$, entièrement ou partiellement substitué par du fluor, ou un groupe chlorotrifluoréthylènedioxy,

R5 représente un atome d'hydrogène ou un groupe facilement scindable dans des conditions physiologiques,

R6 représente un atome d'hydrogène ou un groupe alkyle en $C_1$-$C_4$,

R8 représente un atome d'hydrogène, un groupe alkyle en $C_1$-$C_6$, alcoxy en $C_1$-$C_6$, alcoxy(en $C_1$-$C_4$)-alcoxy(en $C_1$-$C_4$), alcényloxy en $C_2$-$C_5$ ou alcynyloxy en $C_2$-$C_5$,

l'un des substituants R7 et R9 représente un groupe alcoxy en $C_1$-$C_6$ et l'autre représente un atome d'hydrogène, un groupe alkyle en $C_1$-$C_6$, ou alcoxy en $C_1$-$C_6$,

R10 représente un atome d'hydrogène ou un groupe alkyle en $C_1$-$C_6$, et

n représente l'un des nombres 0 ou 1,

et les sels de ces composés, et
l'un des substituants R1, R2 ou R3 situé en position 5 ou 6 du noyau benzimidazole ne peut pas représenter un atome d'hydrogène ou d'halogène, un groupe alkyle en $C_1$-$C_6$, alcoxy en $C_1$-$C_6$, alcoxy en $C_1$-$C_4$, entièrement substitué par du fluor ou substitué de façon prédominante par du fluor, un groupe chlorodifluorométhoxy ou, avec R4 - lorsque celui-ci se trouve en position 6 ou 5 - un groupe alkylènedioxy en $C_1$ ou $C_2$, entièrement ou partiellement substitué par du fluor, ou un groupe chlorotrifluoréthylènedioxy, lorsque

- R4 se trouve en position 6 ou 5 et représente un groupe alcoxy en $C_1$-$C_4$, entièrement substitué par du fluor ou substitué de façon prédominante par du fluor, ou un groupe chlorodifluorométhoxy ou, avec R3 - quand celui-ci se trouve en position 5 ou 6 - un groupe alkylènedioxy en $C_1$ ou $C_2$, entièrement ou partiellement substitué par du fluor, un groupe chlorotrifluoréthylènedioxy, et
- R5 représente un atome d'hydrogène, et
- R6 représente un atome d'hydrogène, et
- R8 représente un atome d'hydrogène ou un groupe alkyle en $C_1$-$C_6$ ou alcoxy en $C_1$-$C_6$, et l'un des substituants R7 et R9 représente un groupe alcoxy en $C_1$-$C_6$ et l'autre représente un atome d'hydrogène ou un groupe alkyle en $C_1$-$C_6$ ou alcoxy en $C_1$-$C_6$, et
- R10 représente un atome d'hydrogène, et les atomes d'hydrogène se trouvent en position 4 et 7 du noyau imidazole.

**4.** Composés de formule I selon la revendication 1 ou 2 ou 3, dans laquelle R6 représente un atome d'hydrogène et R10 représente un atome d'hydrogène, et les symboles R1, R2, R3, R4, R5, R7, R8, R9 et n ont les sens indiqués dans les revendications 1 ou 2 ou 3, et leurs sels.

**5.** Composés de formule I selon la revendication 1 ou 2 ou 3, dans laquelle R6 représente un groupe alkyle en $C_1$-$C_4$ et R10 représente un atome d'hydrogène, et R1, R2, R3, R4, R5, R7, R8, R9 et n ont les sens indiqués dans les revendications 1 ou 2 ou 3, et leurs sels.

**6.** Composés de formule I selon la revendication 1, dans laquelle

- R1 représente un atome d'hydrogène ou un groupe méthyle ou éthyle,
- R2 représente un atome d'hydrogène, de chlore ou de fluor, un groupe méthyle, éthyle, méthoxy ou éthoxy,
- R3 représente un atome d'hydrogène, un groupe méthyle, éthyle, méthoxy, éthoxy, 1,1,2,2-tétrafluoréthoxy, trifluorométhoxy, 2,2,2-trifluoréthoxy, difluorométhoxy, 2-chloro-1,2-trifluoréthoxy ou, avec R4, un groupe difluorométhylènedioxy, 1,1,2-trifluorèthylènedioxy ou 1-chloro-1,2,2-trifluoréthylènedioxy,
- R4 représente un groupe 1,1,2,2-tétrafluoréthoxy, trifluorométhoxy, 2,2,2-trifluoréthoxy, difluorométhoxy, 2-chloro-1,1,2-trifluoréthoxy ou, avec R3, un groupe difluorométhylènedioxy, 1,1,2-trifluoréthylènedioxy ou 1-chloro-1,2,2-trifluoréthylènedioxy,
- R5 représente un atome d'hydrogène,
- R6 représente un atome d'hydrogène ou un groupe méthyle ou éthyle,
- R7 représente un atome d'hydrogène ou un groupe méthyle, éthyle, méthoxy ou éthoxy,
- R8 représente un atome d'hydrogène ou un groupe méthyle, éthyle, méthoxy, éthoxy ou allyloxy,
- R9 représente un atome d'hydrogène ou un groupe méthyle, éthyle, méthoxy ou éthoxy,
- R10 représente un atome d'hydrogène ou un groupe méthyle ou éthyle, et
- n est un nombre valant 0 ou 1, et les sels de ces composés,

l'un des substituants R1, R2 ou R3, situé en position 5 ou 6 du benzimidazole, ne pouvant pas représenter un atome d'hydrogène, de chlore ou de fluor, un groupe méthyle, éthyle, méthoxy, éthoxy, 1,1,2,2-tétrafluoréthoxy, trifluorométhoxy, 2,2,2-trifluoréthoxy, difluorométhoxy ou, avec R4 - lorsque celui-ci se trouve en position 6 ou 5 - un groupe difluorométhylènedioxy, 1,1,2-trifluoréthylènedioxy ou 1-chloro-1,2,2-trifluoréthylènedioxy,
lorsque

- R4 se trouve en position 6 ou 5 et représente un groupe 1,1,2,2-tétrafluoréthoxy, trifluorométhoxy, 2,2,2-trifluoréthoxy, difluorométhoxy ou, avec R3 - lorsque celui-ci se trouve en position 5 ou 6 - un groupe difluorométhylènedioxy, 1,1,2-trifluoréthylènedioxy ou 1-chloro-1,2,2-trifluoréthylènedioxy, et que
- R5 représente un atome d'hydrogène, et

R6 représente un atome d'hydrogène, et
R7 représente un atome d'hydrogène ou un groupe méthyle ou éthyle ou méthoxy ou éthoxy, et
R8 représente un atome d'hydrogène ou un groupe méthyle ou éthyle ou méthoxy ou éthoxy, et
R9 représente un atome d'hydrogène ou un groupe méthyle ou éthyle ou méthoxy ou éthoxy, et
R10 représente un atome d'hydrogène, et les atomes d'hydrogène se trouvent sur les positions 4 et 7 du benzimidazole.

**7.** Composés de formule I selon la revendication 1, dans laquelle
R1 représente un atome d'hydrogène ou un groupe méthyle ou éthyle,
R2 représente un atome d'hydrogène, de chlore ou de fluor ou un groupe méthyle, éthyle, méthoxy ou éthoxy,
R3 représente un atome d'hydrogène, un groupe méthyle, éthyle, méthoxy, éthoxy, 1,1,2,2-tétrafluoréthoxy, trifluorométhoxy, 2,2,2-trifluoréthoxy, difluorométhoxy, 2-chloro-1,1,2-trifluoréthoxy ou, avec R4, un groupe difluorométhylènedioxy, 1,1,2-trifluoréthylènedioxy ou 1-chloro-1,2,2-trifluoréthylènedioxy,
R4 représente un groupe 1,1,2,2-tétrafluoréthoxy, trifluornméthoxy, 2,2,2-trifluoréthoxy, difluorométhoxy, 2-chloro-1,1,2-trifluoréthoxy ou, avec R3 un groupe difluorométhylènedioxy, 1,1,2-trifluoréthylènedioxy ou 1-chloro-1,2,2-trifluoréthylènedioxy,
R5 représente un atome d'hydrogène,
R6 représente un atome d'hydrogène ou un groupe méthyle ou éthyle,
R7 représente un atome d'hydrogène ou un groupe méthyle ou éthyle,
R8 représente un atome d'hydrogène ou un groupe méthyle, éthyle, méthoxy, éthoxy ou allyloxy,
R9 représente un atome d'hydrogène ou un groupe méthyle ou éthyle,
R10 représente un atome d'hydrogène ou un groupe méthyle ou éthyle, et
n représente les nombres 0 ou 1, et les sels de ces composés,

un substituant R1, R2 ou R3, fixé en position 5 ou 6 du benzimidazole, ne pouvant représenter un atome d'hydrogène, de chlore ou de fluor ou un groupe méthyle, éthyle, méthoxy, éthoxy, 1,1,2,2-tétrafluoréthoxy, trifluorométhoxy, 2,2,2-trifluoréthoxy, difluorométhoxy ou, avec R4 - quand celui-ci se trouve en position 6 ou 5 - un groupe difluorométhylènedioxy, 1,1,2-trifluoréthylènedioxy ou 1-chloro-1,2,2-trifluoréthylènedioxy,

lorsque
R4 se trouve en position 6 ou 5 et représente un groupe 1,1,2,2-tétrafluoréthoxy, trifluorométhoxy, 2,2,2-trifluoréthoxy, difluorométhoxy ou, avec R3 - quand celui-ci se trouve en position 5 ou 6 - un groupe difluorométhylènedioxy, 1,1,2-trifluoréthylènedioxy ou 1-chloro-1,2,2-trifluoréthylènedioxy, et que
R5 représente un atome d'hydrogène, et
R6 représente un atome d'hydrogène, et
R7 représente un atome d'hydrogène ou un groupe méthyle ou éthyle, et
R8 représente un atome d'hydrogène ou un groupe méthyle ou éthyle ou méthoxy ou éthoxy, et
R9 représente un atome d'hydrogène ou un groupe méthyle ou éthyle, et
R10 représente un atome d'hydrogène,

et les atomes d'hydrogène se trouvent sur les positions 4 et 7 du benzimidazole.

**8.** Composés de formule I selon la revendication 1, dans laquelle
R1 représente un atome d'hydrogène ou un groupe méthyle ou éthyle,
R2 représente un atome d'hydrogène, de chlore ou de fluor ou un groupe méthyle, éthyle, méthoxy ou éthoxy,
R3 représente un atome d'hydrogène, un groupe méthyle, éthyle, méthoxy, éthoxy, 1,1,2,2-tétrafluoréthoxy, trifluorométhoxy, 2,2,2-trifluoréthoxy, difluorométhoxy, 2-chloro-1,1,2-trifluoréthoxy ou, avec R4, un groupe difluorométhylènedioxy, 1,1,2-trifluoréthylènedioxy ou 1-chloro-1,2,2-trifluoréthylènedioxy,
R4 représente un groupe 1,1,2,2-tétrafluoréthoxy, trifluorométhoxy, 2,2,2-trifluoréthoxy, difluorométhoxy, 2-chloro-1,1,2-trifluoréthoxy ou, avec R3 un groupe difluorométhylènedioxy, 1,1,2-trifluoréthylènedioxy ou 1-chloro-1,2,2-trifluoréthylènedioxy,
R5 représente un atome d'hydrogène,
R6 représente un atome d'hydrogène ou un groupe méthyle ou éthyle,
R8 représente un atome d'hydrogène ou un groupe mèthyle, éthyle, méthoxy, éthoxy ou allyloxy,

l'un des substituants R7 et R9 représente un groupe méthoxy, ou éthoxy et l'autre représente un atome d'hydrogène ou un groupe méthyle, éthyle, méthoxy ou éthoxy,
R10 représente un atome d'hydrogène ou un groupe méthyle ou éthyle, et
n est un nombre valant 0 ou 1, et les sels de ces composes,

l'un des substituants R1, R2 ou R3 fixé en position 5 ou 6 du benzimidazole, ne pouvant représenter un atome d'hydrogène, de chlore ou de fluor ou un groupe méthyle, éthyle, méthoxy, éthoxy, 1,1,2,2-tétrafluoréthoxy, trifluorométhoxy, 2,2,2-trifluoréthoxy, difluorométhoxy ou, avec R4 - quand celui-ci se trouve en position 6 ou 5 - un groupe difluorométhylènedioxy, 1,1,2-trifluoréthylènedioxy ou 1-chloro-1,2,2-trifluoréthylènedioxy,
lorsque
R4 se trouve en position 6 ou 5 et représente un groupe 1,1,2,2-tétrafluoréthoxy, trifluorométhoxy, 2,2,2-trifluoréthoxy, difluorométhoxy ou, avec R3 - quand celui-ci se trouve en position 5 ou 6 - un groupe difluorométhylènedioxy, 1,1,2-trifluoréthylènedioxy ou 1-chloro-1,2,2-trifluoréthylènedioxy, et que
R5 représente un atome d'hydrogène et
R6 représente un atome d'hydrogène, et
R8 représente un atome d'hydrogène ou un groupe méthyle ou éthyle ou méthoxy ou éthoxy, et l'un des substituants R7 et R9 représente un groupe méthoxy ou éthoxy et l'autre substituant représente un atome d'hydrogène, un groupe méthyle, éthyle, méthoxy ou éthoxy, et
R10 représente un atome d'hydrogène, et que les atomes d'hydrogène se trouvent sur les positions 4 et 7 du benzimidazole.

**9.** Composés de formule I selon la revendication 6 ou 7 ou 8, dans laquelle R6 représente un atome d'hydrogène et R10 représente un atome d'hydrogène.

**10.** Composés de formule I selon la revendication 6 ou 7 ou 8, dans laquelle R6 représente un groupe méthyle ou éthyle, et R10 représente un atome d'hydrogène.

**11.** Composés de formule I selon la revendication 1, dans laquelle
R1 représente un atome d'hydrogène,
R2 représente un atome d'hydrogène ou un groupe méthyle ou méthoxy,
R3 représente un atome d'hydrogène ou un groupe méthyle ou méthoxy, 1,1,2,2-tétrafluoréthoxy, difluorométhoxy, 2-chloro-1,1,2-trifluoréthoxy ou, avec R4, un groupe difluorométhylènedioxy,
R4 représente un groupe 1,1,2,2-tétrafluoréthoxy, difluorométhoxy, 2-chloro-1,1,2-trifluoroéthoxy ou, avec R3, un groupe difluorométhylènedioxy,
R5 représente un atome d'hydrogène,
R6 représente un atome d'hydrogène ou un groupe méthyle,
R7 représente un atome d'hydrogène ou un groupe méthyle ou mèthoxy,
R8 représente un groupe méthoxy,
R9 représente un atome d'hydrogène,
R10 représente un atome d'hydrogène, et
n représente l'un des nombres 0 ou 1, et les sels de ces composés,

l'un des substituants R1, R2 ou R3, fixé en position 5 ou 6 du benzimidazole, ne pouvant représenter un atome d'hydrogène ou un groupe méthyle, méthoxy, 1,1,2,2-tétrafluoréthoxy, difluorométhoxy ou, avec R4 - quand celui-ci se trouve en position 6 ou 5 - un groupe difluorométhylènedioxy,
lorsque
R4 se trouve en position 6 ou 5 et représente un groupe 1,1,2,2-tétrafluoréthoxy, difluorométhoxy ou, avec R3 - quand celui-ci se trouve en position 5 ou 6 - un groupe difluorométhylènedioxy, et
R5 représente un atome d'hydrogène, et
R6 représente un atome d'hydrogène, et

R7 représente un atome d'hydrogène ou un groupe méthyle ou méthoxy, et
R8 représente un groupe méthoxy, et
R9 représente un atome d'hydrogène, et
R10 représente un atome d'hydrogène, et que
les atomes d'hydrogène se trouvent sur les positions 4 et 7 du benzimidazole.

**12.** Composé selon la revendication 1, choisi dans l'ensemble consistant en :
le 5-difluorométhoxy-2-[(4-méthoxy-3-méthyl-2-pyridyl)méthylsulfinyl]-4,6-diméthyl-1H-benzimidazole,
le 5-difluorométhoxy-6-méthoxy-2-{[1-(4-méthoxy-2-pyridyl)éthyl]sulfinyl}-1H-benzimidazole,
le 5-(1,1,2,2-tétrafluoréthoxy)-2-{[1-(4-méthoxy-2-pyridyl)éthyl]-sulfinyl}-1H-benzimidazole,
le 5-difluorométhoxy-2-[(3,4-diméthoxy-2-pyridyl)méthylsulfinyl]-4,6-diméthyl-1H-benzimidazole,
le 2,2-difluoro-6-{[1-(4-méthoxy-2-pyridyl)éthyl]sulfinyl}-5H-[1,3]-dioxolo[4,5-f]benzimidazole,
le 5-(2-chloro-1,1,2-trifluoréthoxy)-2-[(4-méthoxy-2-pyridyl)méthylsulfinyl]-1H-benzimidazole,
le 5-(2-chloro-1,1,2-trifluoréthoxy)-2-{[1-(4-méthoxy-2-pyridyl)éthyl]sulfinyl}-1H-benzimidazole,
le 5-difluorométhoxy-2-[(4-méthoxy-3-méthyl-2-pyridyl)méthylthio]-4,6-diméthyl-1H-benzimidazole,
le 5-difluorométhoxy-6-méthoxy-2-{[1-(4-méthoxy-2-pyridyl)éthyl]thio}-1H-benzimidazole,
le 5-(2-chloro-1,1,2-trifluoréthoxy)-2-[(4-méthoxy-3-méthyl-2-pyridyl)méthylsulfinyl]-1H-benzimidazole,
et les sels pharmacologiquement tolérables de ces composés.

**13.** Procédé pour préparer les composés de formule I selon la revendication 1, et leurs sels, caractérisé en ce que l'on fait réagir :

a) des mercaptobenzimidazoles de formule II avec des dérivés de picoline III,

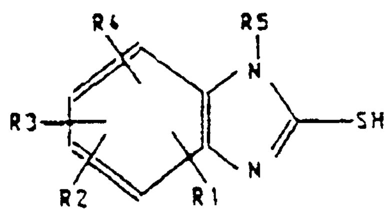

(II),

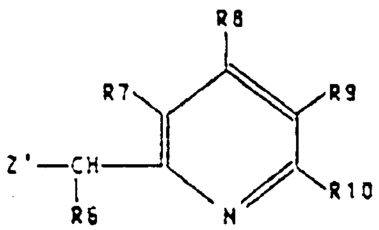

(III),

ou

b) on fait réagir des benzimidazoles de formule IV avec des mercaptopicolines V :

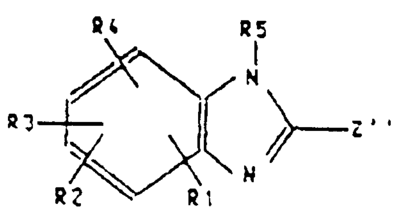

(IV),

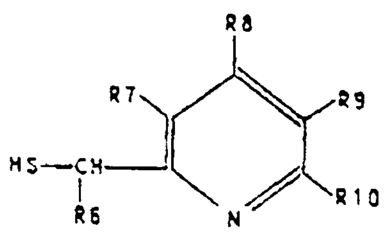

(V),

ou

c) on fait réagir des o-phénylènediamines de formule VI avec des dérivés de l'acide formique VII

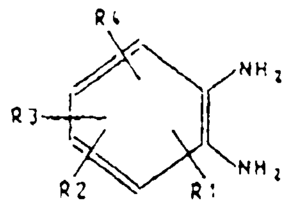

(VI),

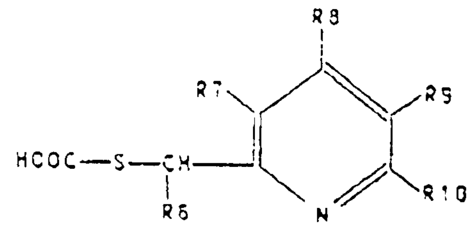

(VII),

et (si les composés de formule I dans laquelle n vaut 1 constituent les produits finals voulus), on oxyde ensuite les sulfures de 2-benzimidazolyl-2-pyridylméthyle de formule VIII, obtenus selon a), b) ou c) (= composés de formule I dans laquelle n = 0):

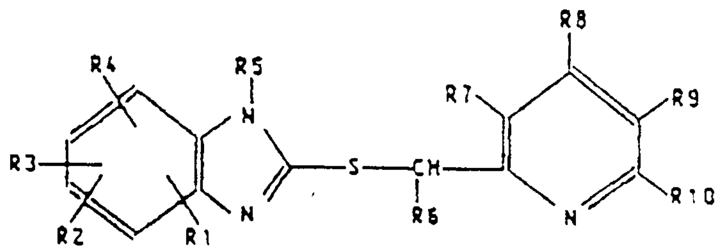

(VIII),

et/ou on les transforme, si on le souhaite, en les sels,

ou en ce que :

d) on fait réagir des benzimidazoles de formule IX avec des dérivés de pyridine X

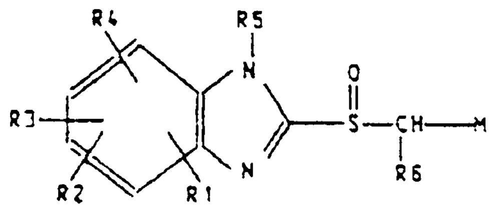

(IX),

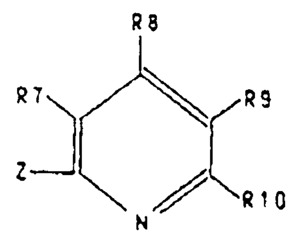

(X),

ou

e) on fait réagir des dérivés de sulfinyle de formule XI avec des dérivés de 2-picoline de formule XII

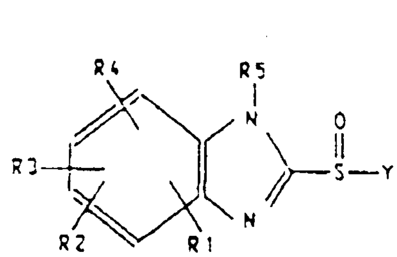

(XI),

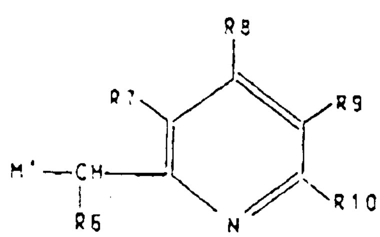

(XII),

et éventuellement ensuite, on les transforme en les sels, ou

f) si les composés de formule I, dans laquelle R5 représente un groupe facilement scindable dans les conditions physiologiques, constituent les produits à fabriquer selon le procédé - en ce qu'on fait réagir les composés de formule I, dans laquelle R5 représente un atome d'hydrogène, avec des composés de formule R5'-Y' (XIII), dans laquelle R5' représente le groupe facilement scindable dans des conditions physiologiques ou en constituant avec Y' un précurseur, et éventuellement on transforme ensuite en les sels, ou bien,

g) si les composés de formule I, dans laquelle R5 représente un atome d'hydrogène, constituent les produits à fabriquer par le procédé, en ce qu'on soumet des composés de formule I, dans laquelle R5 représente un groupe facilement scindable dans des conditions physiologiques, à une solvolyse, et l'on transforme éventuellement les produits ainsi obtenus en les sels, ou bien

h) si les composés de formule I, dans laquelle R6 représente un groupe alkyle en $C_1$-$C_6$ constituent les produits à fabriquer par le procédé, en ce qu'on soumet des composés de formule I, dans laquelle R6 représente un atome d'hydrogène, à une alkylation effectuée avec des composés de formule R6-Y'' (XIV), dans laquelle R6 représente un groupe alkyle en $C_1$-$C_6$, et éventuellement, on les transforme ensuite en les sels,

les symboles Y, Y'', Z, Z' et Z'' représentant des groupes partants convenables, Y' représentant un groupe partant ou un groupe réactif, M représentant un atome de métal alcalin (Li, Na ou K), M' représentant l'équivalent d'un atome de métal et R1, R2, R3, R4, R5, R6, R7, R8, R9, R10 et n (dans la mesure où ils n'ont pas une autre définition) ayant les sens indiqués à la revendication 1.

**14.** Médicaments contenant un ou plusieur composés selon une ou plusieurs des revendications 1 à 12 et/ou leurs sels pharmacologiquement tolérables.

**15.** Composés selon une ou plusieurs des revendications 1 à 12 et leurs sels pharmacologiquement tolérables, destinés à être appliqués au traitement curatif et/ou prophylactique de maladies de l'estomac et/ou de l'intestin et de maladies fondées sur une augmentation de la sécrétion des acides ou sucs gastriques.

**16.** Utilisation de composés selon une ou plusieurs des revendications 1 à 12 et de leurs sels pharmacologiquement tolérables pour fabriquer des médica-ments destinés au traitement curatif et/ou prophylactique de maladies de l'estomac et/ou de l'intestin et de maladies qui reposent sur une augmentation de la sécrétion des sucs ou acides gastriques.

**17.** Mercaptobenzimidazoles de formule II

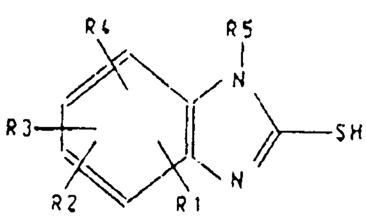

(II)

dans laquelle

R1, R2, R3 et R4 peuvent être fixés en des positions quelconques de la partie benzénique du benzimidazole, et

- R1 représente un atome d'hydrogène ou un groupe alkyle en $C_1$-$C_6$,
- R2 représente un atome d'hydrogène ou d'halogène, un groupe alkyle en $C_1$-$C_6$, alcoxy en $C_1$-$C_6$, alcoxy (en $C_1$-$C_4$)-alkyle (en $C_1$-$C_4$), alcoxy (en $C_1$-$C_4$)-alcoxy (en $C_1$-$C_4$), phényle, phénoxy, phénoxy-alkyle (en $C_1$-$C_4$), phénoxy-alcoxy (en $C_1$-$C_4$), phène-alkyle (en $C_1$-$C_4$) ou phène-alcoxy (en $C_1$-$C_4$),
- R3 représente un atome d'hydrogène, un groupe alkyle en $C_1$-$C_6$, alcoxy en $C_1$-$C_6$, alcoxy en $C_1$-$C_4$ entièrement substitué par du fluor ou substitué de façon prédominante par du fluor, un groupe chlorodifluorométhoxy, 2-chloro-1,1,2-trifluoréthoxy ou, avec R4, un groupe alkylènedioxy en $C_1$ ou $C_2$, entièrement ou partiellement substitué par du fluor ou un groupe chlorotrifluoréthylènedioxy,

R4 représente un groupe alcoxy en $C_1$-$C_4$, entièrement substitué par du fluor ou substitué de façon prédominante par du fluor, un groupe chlorodifluorométhoxy, 2-chloro-1,1,2-trifluoréthoxy ou, avec R3, un groupe alkylènedioxy en $C_1$ ou $C_2$, entièrement ou partiellement substitué par du fluor, ou un groupe chlorotrifluoréthylènedioxy, et

R5 représente un atome d'hydrogène ou un groupe scindable facilement dans des conditions physiologiques,

et les sels de ces composés,

un substituant R1, R2 ou R3, fixé en position 5 ou 6 du benzimidazole, ne pouvant représenter un atome d'hydrogène ou d'halogène, un groupe alkyle en $C_1$-$C_6$, alcoxy en $C_1$-$C_6$, un groupe alcoxy en $C_1$-$C_4$ entièrement substitué par du fluor ou substitué de façon prédominante par du fluor, un groupe chlorodifluorométhoxy ou, avec R4 - quand celui-ci se trouve en position 6 ou 5 - un groupe alkylènedioxy en $C_1$ ou $C_2$, entièrement ou partiellement substitué par du fluor ou un groupe chlorotrifluoréthylènedioxy,

lorsque

R4 se trouve en position 6 ou 5 et représente un groupe alcoxy en $C_1$-$C_4$, entièrement substitué par du fluor ou substitué de façon prédominante par du fluor, ou un groupe chlorodifluorométhoxy ou, avec R3 - quand celui-ci se trouve en position 5 ou 6 - un groupe alkylènedioxy en $C_1$ ou $C_2$, entièrement ou partiellement substitué par du fluor, ou un groupe chlorotrifluoréthylènedioxy, et que

R5 représente un atome d'hydrogène, et que les atomes d'hydrogène se trouvent sur les positions 4 et 7 du benzimidazole.

**18.** Composés de formule II selon la revendication 17, dans laquelle

R1 représente un atome d'hydrogène,

R2 représente un atome d'hydrogène ou un groupe méthyle ou méthoxy,

R3 représente un atome d'hydrogène, un groupe méthyle, méthoxy, 1,1,2,2-tétrafluoréthoxy, difluorométhoxy, 2-chloro-1,1,2-trifluoréthoxy ou, avec R4, un groupe difluorométhylènedioxy,

R4 représente un groupe 1,1,2,2-tétrafluoréthoxy, difluorométhoxy, 2-chloro-1,1,2-trifluoréthoxy ou, avec R3, un groupe difluorométhylènedioxy, et

R5 représente un atome d'hydrogène,

l'un des substituants R1, R2 ou R3, fixé en position 5 ou 6 du benzimidazole, ne pouvant représenter un atome d'hydrogène ou un groupe méthyle, méthoxy, 1,1,2,2-tétrafluoréthoxy, difluorométhoxy ou, avec R4 - quand celui-ci se trouve en position 6 ou 5 - un groupe difluorométhylènedioxy,

lorsque

R4 se trouve en position 6 ou 5 et représente un groupe 1,1,2,2-tétrafluoréthoxy, difluorométhoxy ou, avec R3 - quand celui-ci se trouve en position 5 ou 6 - un groupe difluorométhylènedioxy,

et que les atomes d'hydrogène se trouvent sur les positions 4 et 7 du benzimidazole.